# EUROPEAN PATENT SPECIFICATION

(11) **EP 2 960 337 B1**
(45) Date of publication and mention of the grant of the patent: **25.09.2019**
(21) Application number: 14754590.9
(22) Date of filing: 19.02.2014
(51) Int. Cl.: C12P 7/00, C12P 7/20, C12P 9/00

(54) **ENZYMATIC PRODUCTION METHOD FOR GLYCEROL PHOSPHATE**
ENZYMATISCHES HERSTELLUNGSVERFAHREN FÜR GLYCERINPHOSPHAT
PROCÉDÉ DE PRODUCTION ENZYMATIQUE DE GLYCÉROL PHOSPHATE

(30) Priority: 19.02.2013 JP 2013029701
(43) Date of publication of application: 30.12.2015
(73) Proprietor: Thermostable Enzyme Laboratory Co., Ltd., Hyogo 650-0047 (JP)
(72) Inventor: IWATA, Hideyuki, Kobe-shi Hyogo 650-0047 (JP); TANAKA, Hisae, Kobe-shi Hyogo 650-0047 (JP)
(74) Representative: Mewburn Ellis LLP
(86) International application number: PCT/JP2014/053888
(87) International publication number: WO 2014/129492

(56) References cited:
- EP-A2- 0 462 833
- VAN HERK: "Bacterial class A acid phosphatases as versatile tools in organic synthesis", Van't Hoff Institute for Molecular Science (HIMS) PhD thesis / Chapter 1, 2008, pages 1-27 (as renumbered), XP002760238, Amsterdam Retrieved from the Internet: URL:http://dare.uva.nl/document/2/53744 [retrieved on 2016-07-25]
- VAN HERK ET AL: "Simple enzymatic in situ generation of dihydroxyacetone phosphate and its use in a cascade reaction for the production of carbohydrates: increased efficiency by phosphate cycling", JOURNAL OF ORGANIC CHEMISTRY, vol. 71, 2006, pages 6244-6247, XP002760269,
- XIAOCHI CHEN ET AL: "Isolation, cloning, and expression of an acid phosphatase containing phosphotyrosyl phosphatase activity from Prevotella intermedia", JOURNAL OF BACTERIOLOGY, vol. 181, 1999, pages 7107-7114, XP002760239,
- PRADINES ET AL: "Large-scale enzymatic synthesis of glycerol 1-phosphate", ENZYME AND MICROBIAL TECHNOLOGY, vol. 13, 1991, pages 19-23, XP023854198,
- BABICH ET AL: "Phosphorylation by alkaline phosphatase: immobilization and synthetic potential", INTERNATIONAL JOURNAL OF CHEMISTRY, vol. 5, 24 July 2013 (2013-07-24), pages 87-98, XP002760240,
- HERK TV ET AL.: 'Regioselective Phosphorylation of Carbohydrates and Various Alcohols by Bacterial Acid Phosphatases; Probing the Substrate Specificity of the Enzyme from Shigella flexneri' ADV SYNTH CATAL. vol. 347, no. 7, 01 January 2005, pages 1155 - 1162, XP002416329 DOI: 10.1002/ADSC.200505072
- BABICH L ET AL.: 'Continuous-flow reactor-based enzymatic synthesis of phosphorylated compounds on a large scale' CHEM EUR J. vol. 18, no. 21, 21 May 2012, pages 6604 - 6609, XP055283547 DOI: 10.1002/CHEM.201200101
- DATABASE UNIPROT [Online] 13 June 2012 IYER PR ET AL., XP055283618 Retrieved from IBIS Database accession no. D5QGJ0
- DATABASE UNIPROT [Online] 06 February 2013 SALZBERG SL ET AL.: 'SUBNAME: FULL=PHOSPHATASE', XP055293032 Retrieved from IBIS Database accession no. B2SUV7
- DATABASE UNIPROT [Online] 28 November 2012 BROWN PJB ET AL.: 'Definition: SubName: Full=Major phosphate- irrepressible acid phosphatase', XP055283561 Retrieved from IBIS Database accession no. F4R2A1
- POND JL ET AL.: 'Cloning, sequencing, and characterization of the principal acid phosphatase, the phoC+ product, from Zymomonas mobilis' J BACTERIOL. vol. 171, no. 2, 01 February 1989, pages 767 - 774, XP055283551
- DATABASE UNIPROT [Online] 06 February 2013 NAKAZAWA H ET AL.: 'Definition: SubName: Full=Acid phosphatase', XP055283568 Retrieved from IBIS Database accession no. C4XPK0
- DATABASE UNIPROT [Online] 01 March 2013 PRUST C ET AL.: 'Definition: SubName: Full=Nonspecific acid phosphatase', XP055283573 Retrieved from IBIS Database accession no. Q5FQI1
- DATABASE UNIPROT [Online] 06 February 2013 LUCAS S ET AL.: 'SUBNAME: FULL=ACID PHOSPHATASE', XP055293037 Retrieved from IBIS Database accession no. G0B7H8
- DATABASE UNIPROT [Online] 28 November 2012 LUCAS S ET AL.: 'Definition: SubName: Full=Acid phosphatase', XP055293043 Retrieved from IBIS Database accession no. E8XYI1
- UCHIYA K ET AL.: 'Identification and characterization of phoN-Sf, a gene on the large plasmid of Shigella flexneri 2a encoding a nonspecific phosphatase' J BACTERIOL. vol. 178, no. 8, 01 August 1996, pages 4548 - 4554, XP055283622

## Description

### TECHNICAL FIELD

The present invention relates to a method for the production of a phosphorylated glycerol (= glycerol phosphate) with high efficiency.

### BACKGROUND ART

Since phosphorylated glycerol is highly useful, for example, as raw materials for cosmetics and as intermediates for producing pharmaceuticals, methods have been investigated in which a phosphorylated glycerol can be obtained with high efficiency. A method for achieving efficient phosphorylation is, for example, one involving the use of a phosphorylating enzyme that catalyzes the phosphorylation of glycerol. Many types of phosphorylating enzymes have been known until now, and there have been found those derived from many different species (Non-Patent Document 1). However, even though any of these many phosphorylating enzymes which exist is employed, the improvement in the efficiency of the phosphorylation reaction is not always accomplished. In addition, it is necessary that in reactions for obtaining a phosphorylated glycerol, a phosphorylating enzyme and glycerol are reacted in the presence of a phosphate group donor. Phosphorylating enzymes that have been used in the past, however, have a problem that expensive ATP (adenosine triphosphate) is required as a phosphate group donor in the reaction, thereby increasing production costs. Polyphosphoric acids, on the other hand, are mentioned as an example of an inexpensive phosphate group donor, but there have not been known any enzymes that use a polyphosphoric acid to catalyze the glycerol phosphorylating reaction (see, for example, Non-Patent Document 2). Because of these backgrounds, these is a great need for a method by which a phosphorylated glycerol of great industrial usefulness is produced with high efficiency and at low cost.

A class A acidic phosphatase derived from *Shigella* flexneri has been used to produce glycerophosphoric acid from glycerol using pyrophosphate as the phosphate donor (Non-Patent Document 3). The active centre of this phosphatase can be represented by sequence GSYPSGHT (Non-Patent Documents 4-6).

### PRIOR ART DOCUMENTS

### NON-PATENT DOCUMENTS

Non-Patent Document 1: Cell Mol Life Sci., 1998 Aug; 54(8): 833-50.
Non-Patent Document 2: Protein Eng., 1998 Dec; 11(12): 1219-27.
Non-Patent Document 3: Adv Synth Catal., 2005 Jun; 347: 1155-1162.
Non-Patent Document 4: J Bacteriol., 1996 Aug; 178(8): 4548-4554.
Non-Patent Document 5: T. Van Herk, PhD Thesis, Van't Hoff Institute for Molecular Sciences, 2008, Chapter 1.
Non-Patent Document 6: J Bacteriol., 1999 Nov; 181(22): 7107-7114.

### SUMMARY OF THE INVENTION

### PROBLEMS TO BE SOLVED BY THE INVENTION

The present invention has a main purpose of providing a method for the phosphorylation of glycerol in which a high efficiency of phosphorylation can be achieved.

### MEANS FOR SOLVING THE PROBLEM

The inventors have made intensive studies to solve the above problem, with the result that it has been found that the efficiency of the phosphorylation of glycerol is significantly improved by using a phosphorylating enzyme derived from a particular microorganism. In addition, it has been found that by using this enzyme, a phosphorylated glycerol is efficiently obtained even when the phosphorylation reaction is performed using an inexpensive phosphate group donor like a polyphosphoric acid. Further, the inventors have identified the amino acid sequence responsible for the activity of the phosphorylating enzyme obtained from the particular microorganism. The present invention has been completed as a result of further improvements based on these findings.

Thus, the present invention provides methods for phosphorylation according to the embodiments described below:
Embodiment 1: a method for production of a phosphorylated glycerol, comprising a step of allowing a phosphorylating enzyme to act on glycerol in the presence of a phosphate group donor,
   wherein the phosphorylating enzyme is a class A acid phosphatase and includes:
   - a polypeptide as defined in (i) or (ii) below:
      (i) a polypeptide that has the amino acid sequence set forth in SEQ ID NO: 2 and has an activity to catalyze the glycerol phosphorylating reaction, or
      (ii) a polypeptide that has an amino acid sequence having 1 to 3 amino acids substituted, deleted, inserted, or added in the amino acid sequence set forth in SEQ ID NO: 2 and has an activity to catalyze the glycerol phosphorylating reaction which is comparable or superior to that of the polypeptide as defined in (i); and
   - a polypeptide having, on the C-terminal side of the amino acid sequence set forth in SEQ ID NO: 2, an amino acid sequence represented by the sequence (1):

      -X¹-X²-X³-Pro-Ser-Gly-His-X⁴- (1)

      wherein,
      X¹ denotes glycine, alanine, or phenylalanine;
      X² denotes any amino acid;
      X³ denotes tyrosine or tryptophan; and
      X⁴ denotes threonine, serine, or alanine;
      with the proviso that when X¹ is phenylalanine, X⁴ is threonine or serine.
Embodiment 2: the method for production according to embodiment 1, wherein the amino acid residue at the first position in the amino acid sequence set forth in SEQ ID NO: 2 is located between positions 5 and 40 from the N-terminus of the phosphorylating enzyme.
Embodiment 3: the method for production according to embodiment 1 or 2, wherein X¹ and X⁴ in the sequence (1) are any of (A1) to (A8) below:
   (A1) X¹ is glycine and X⁴ is threonine;
   (A2) X¹ is glycine and X⁴ is serine;
   (A3) X¹ is alanine and X⁴ is serine;
   (A4) X¹ is alanine and X⁴ is threonine;
   (A5) X¹ is alanine and X⁴ is alanine;
   (A6) X¹ is glycine and X⁴ is alanine;
   (A7) X¹ is phenylalanine and X⁴ is threonine; and
   (A8) X¹ is phenylalanine and X⁴ is serine.
Embodiment 4: the method for production according to embodiments 1 to 3, wherein in the sequence (1), X² is serine, alanine, or aspartic acid.
Embodiment 5: the method for production according to any of embodiments 1 to 4, wherein X¹, X², X³, and X⁴ in the sequence (1) are any of (B1) to (B11) below:
   (B1) X¹ is glycine, X² is serine, X³ is tyrosine, and X⁴ is threonine;
   (B2) X¹ is glycine, X² is alanine, X³ is tyrosine, and X⁴ is threonine;
   (B3) X¹ is glycine, X² is serine, X³ is tyrosine, and X⁴ is serine;
   (B4) X¹ is glycine, X² is aspartic acid, X³ is tyrosine, and X⁴ is threonine;
   (B5) X¹ is alanine, X² is serine, X³ is tyrosine, and X⁴ is serine;
   (B6) X¹ is alanine, X² is serine, X³ is tyrosine, and X⁴ is threonine;
   (B7) X¹ is alanine, X² is serine, X³ is tyrosine, and X⁴ is alanine;
   (B8) X¹ is glycine, X² is serine, X³ is tyrosine, and X⁴ is alanine;
   (B9) X¹ is phenylalanine, X² is serine, X³ is tyrosine, and X⁴ is threonine;
   (B10) X¹ is phenylalanine, X² is serine, X³ is tyrosine, and X⁴ is serine; and
   (B11) X¹ is glycine, X² is serine, X³ is tryptophan, and X⁴ is serine.
Embodiment 6: the method for production according to any of embodiments 1 to 5, wherein the histidine (His) residue at the 7-th position in the amino acid sequence represented by the sequence (1) is located between positions 120 and 160 from the N-terminus of the phosphorylating enzyme.
Embodiment 7: the method for production according to any of embodiments 1 to 6, wherein the phosphorylating enzyme further comprises at least one of the amino acid sequences as defined in (iii) to (vi) below:
   (iii) a polypeptide that has, on the N-terminal side of the amino acid sequence set forth in SEQ ID NO: 2, the amino acid sequence set forth in SEQ ID NO: 40 and has an activity to catalyze the glycerol phosphorylating reaction;
   (iv) a polypeptide that has, on the N-terminal side of the amino acid sequence set forth in SEQ ID NO: 2, an amino acid sequence having 1 to 3 amino acids substituted, deleted, inserted, or added in the amino acid sequence set forth in SEQ ID NO: 40 and has an activity to catalyze the glycerol phosphorylating reaction which is comparable or superior to that of the polypeptide as defined in (iii);
   (v) a polypeptide that has, on the C-terminal side of the amino acid sequence set forth in SEQ ID NO: 2, the amino acid sequence set forth in SEQ ID NO: 41 and has an activity to catalyze the glycerol phosphorylating reaction; and
   (vi) a polypeptide that has, on the C-terminal side of the amino acid sequence set forth in SEQ ID NO: 2, an amino acid sequence having 1 to 10 amino acids substituted, deleted, inserted, or added in the amino acid sequence set forth in SEQ ID NO: 41 and has an activity to catalyze the glycerol phosphorylating reaction which is comparable or superior to that of the polypeptide as defined in (v).
Embodiment 8: the method for production according to any of embodiments 1 to 7, wherein the phosphorylating enzyme includes a polypeptide as defined in (vii) or (viii) below:
   (vii) a polypeptide that has the amino acid sequence set forth in any of SEQ ID NOs: 9, 15 to 17, 19, 22, and 25; or
   (viii) a polypeptide that includes an amino acid sequence having, in the amino acid sequence set forth in any of SEQ ID NOs: 9, 15 to 17, 19, 22, and 25, 1 to 10 amino acids substituted, deleted, inserted, or added in a region outside the amino acid sequence set forth in SEQ ID NO: 2 and has an activity to catalyze the glycerol phosphorylating reaction which is comparable or superior to that of the polypeptide as defined in (vii).
Embodiment 9: the method for production according to any of embodiments 1 to 6, wherein the phosphorylating enzyme further includes any of the polypeptides as defined in (III) to (VI) below:
   (III) a polypeptide that has the amino acid sequence set forth in SEQ ID NO: 32, on the N-terminal side of the amino acid sequence represented by the sequence (1);
   (IV) a polypeptide that has, on the N-terminal side of the amino acid sequence represented by the sequence (1), an amino acid sequence having 1 to 10 amino acids substituted, deleted, inserted, or added in the amino acid sequence set forth in SEQ ID NO: 32 and has an activity to catalyze the glycerol phosphorylating reaction which is
      comparable or superior to that of its corresponding polypeptide as defined in (III);
   (V)) a polypeptide that has the amino acid sequence set forth in any of SEQ ID NOs: 33 to 39 and 111 to 115, on the C-terminal side of the amino acid sequence represented by the sequence (1); and
   (VI) a polypeptide that has, on the C-terminal side of the amino acid sequence represented by the sequence (1), an amino acid sequence having 1 to 10 amino acids substituted, deleted, inserted, or added in the amino acid sequence set forth in any of SEQ ID NOs: 33 to 39 and 111 to 115 and has an activity to catalyze the glycerol phosphorylating reaction which is comparable or superior to that of its corresponding polypeptide as defined in (V).
Embodiment 10: the method for production according to any of embodiments 1 to 6 and 9, wherein the phosphorylating enzyme includes a polypeptide as defined in (VII) or (VIII) below:
   (VII) a polypeptide that has the amino acid sequence set forth in SEQ ID NO: 9, or
   (VIII) a polypeptide that includes an amino acid sequence having, in the amino acid sequence set forth in SEQ ID NO: 9, 1 to 10 amino acids substituted, deleted, inserted, or added in a region outside the active center including the amino acid sequence represented by the sequence (1) and has an activity to catalyze the glycerol phosphorylating reaction which is comparable or superior to that of its corresponding polypeptide as defined in (VII).
Embodiment 11: the method for production according to any of embodiments 1 to 10, wherein the phosphate group donor is a polyphosphoric acid.
Embodiment 12: the method for production according to any of embodiments 1 to 11, wherein the phosphorylated glycerol is α-glycerophosphate.
Embodiment 13: the method for production according to any of embodiments 1 to 12, wherein the pH of the reaction solution is from 4 to 5 in the step of allowing the phosphorylating enzyme to act on the glycerol in the presence of the phosphate group donor.
Embodiment 14: the method for production according to any of embodiments 1 to 13, wherein the glycerol is added in an amount of 1000 to 50000 parts by weight per part by weight of the phosphorylating enzyme, in the step of allowing the phosphorylating enzyme to act on the glycerol in the presence of the phosphate group donor.
Embodiment 15: the method for production according to any of embodiments 1 to 14, wherein the concentration of the phosphate group donor in the reaction solution is from 2% to 10% by weight in the step of allowing the phosphorylating enzyme to act on the glycerol in the presence of the phosphate group donor.

### ADVANTAGES OF THE INVENTION

According to the methods for phosphorylation according to the present invention, it is possible to significantly enhance the efficiency of the phosphorylation of glycerol. In addition, it is possible to use an inexpensive polyphosphoric acid as a phosphate group donor, thereby to allow reducing production costs.

### BRIEF DESCRIPTION OF THE DRAWINGS

Fig. 1 represents graphs showing the results of the phosphorylation of glycerol in which phosphorylating enzymes derived from various microorganisms were used. In Fig. 1, graph (i) shows the results when the reaction was carried out using 4 M of glycerol and at a pH of 5.0, graph (ii) shows the results when using 4 M of glycerol and at a pH of 4.0, and graph (iii) shows the results when using 0.5 M of glycerol and at a pH of 4.5.
Fig. 2 represents graphs showing the results of glycerol phosphorylating reactions in which the type of phosphate group donor was changed.
Fig. 3 represents a graph showing the amount of the resulting glycerol-3-phosphate in which the glycerol phosphorylating reaction was performed using Mutant a.
Fig. 4 represents a graph showing the amount of the resulting glycerol-3-phosphate in which the glycerol phosphorylating reaction was performed using Mutants b to g.
Figs. 5(i) and (ii) show the structures of phosphorylating enzyme mutants A to L in Fig. 3. In Figs. 3 (i) and (ii), the mutated portion in the amino acid sequences of these mutants is underlined.
Fig. 6 represents a graph showing the amount of the resulting glycerol-3-phosphate in which the glycerol phosphorylating reaction was performed using Mutants A to L.

### EMBODIMENTS OF THE INVENTION

### 1.Phosphorylating enzymes

The present invention provides a method for the production of a phosphorylated glycerol. The method is characterized by allowing a given phosphorylating enzyme to act on glycerol in the presence of a phosphate group donor. Hereinafter, a method for the production of a phosphorylated glycerol according to the present invention is sometimes referred to simply as "a method of the present invention."

The glycerol to be phosphorylated in the present invention is represented by C₃H₈O₃ and is also referred to as 1,2,3-trihydroxypropane or glycerin.

The method of the present invention uses as the phosphorylating enzyme a class A acid phosphatase, which catalyzes a glycerol phosphorylating reaction, thereby to obtain a phosphorylated glycerol. Here, an acid phosphatase is a phosphomonoesterase having a function of attaching free phosphate groups from other molecules. In addition, class A is a classification of acid phosphatases and indicates that enzymes of class A belong to a group of enzymes having the property that the enzymatic activity is not inhibited by EDTA and by inorganic phosphates. Further, a class A acid phosphatase is defined as a generic term for enzymes having an amino acid sequence of KX₆RP-(X₁₂₋₅₄)-PSGH-(X₃₁₋₅₄)-SRX₅HX₃D wherein each X independently denotes any amino acid (The EMBO Journal, Vol 19, No.11, pp 2412-2423).

The phosphorylating enzyme which is used in the method of the present invention is:
- a polypeptide as defined in (i) or (ii) below:
   (i) a polypeptide that has the amino acid sequence set forth in SEQ ID NO: 2 and has an activity to catalyse the glycerol phosphorylating reaction, or
   (ii) a polypeptide that has an amino acid sequence having 1 to 3 amino acids substituted, deleted, inserted, or added in the amino acid sequence set forth in SEQ ID NO: 2 and has an activity to catalyze the glycerol phosphorylating reaction which is comparable or superior to that of the polypeptide as defined in (i); and
- a polypeptide having, on the C-terminal side of the amino acid sequence set forth in SEQ ID NO: 2, an amino acid sequence represented by the sequence (1):

   -X¹-X²-X³-Pro-Ser-Gly-His-X⁴- (1)

   wherein,
   X¹ denotes glycine, alanine, or phenylalanine;
   X² denotes any amino acid;
   X³ denotes tyrosine or tryptophan; and
   X⁴ denotes threonine, serine, or alanine;
   with the proviso that when X¹ is phenylalanine, X⁴ is threonine or serine.

The amino acid sequence set forth in SEQ ID NO: 2, and amino acid sequences having 1 to 3 amino acids substituted, deleted, inserted, or added in the amino acid sequence set forth in SEQ ID NO: 2 are a partial amino acid sequence that is located on the N-terminal side of the active center in the amino acid sequence of the phosphorylating enzyme. In the amino acid sequence of the phosphorylating enzyme according to the present invention, it is suitable that the amino acid sequence set forth in
SEQ ID NO: 2 is located on the N-terminal side of the active center, including, in particular, embodiments in which the first amino acid residue in the amino acid sequence set forth in SEQ ID NO: 2 is located between positions 5 and 40, preferably between positions 8 and 35, further preferably between positions 10 and 30, from the N-terminus of the amino acid sequence of the phosphorylating enzyme.

In polypeptides as defined in (i) and (ii) above, the amino acid sequence of the active center is any amino acid sequence that is not limited in particular, with the proviso that it can serve as the active center of a class A acid phosphatase, and preferably is the amino acid sequence represented by the sequence (1) as described above.

In polypeptides as defined in (i) and (ii) above, wherein the polypeptide comprises as the active center the amino acid sequence represented by the sequence (1), the amino acid sequence set forth in SEQ ID NO: 2 and a stretch of the amino acid sequence coding for the active center represented by the sequence (1) may be joined directly. Alternatively, they may be joined, for example, via 70 to 130 amino acids, preferably via 80 to 110 amino acids, further preferably via 90 to 110 amino acids, as long as the activity of the polypeptide to catalyze the glycerol phosphorylating reaction is not disturbed. In addition, polypeptides as defined in (i) and (ii) above may have any amino acid sequence, for example, of 5 to 50 amino acids, preferably of 8 to 40 amino acids, further preferably of 10 to 30 amino acids, on the N-terminal side of the amino acid sequence set forth in SEQ ID NO: 2, as long as the activity of the polypeptide to catalyze the glycerol phosphorylating reaction is not disturbed.

Further, polypeptides as defined in (i) and (ii) above may have any amino acid sequence, for example, of 60 to 120 amino acids, preferably of 70 to 110 amino acids, further preferably of 80 to 100 amino acids, on the C-terminal side of a stretch of the amino acid sequence coding for the active center, as long as the activity of the polypeptide to catalyze the glycerol phosphorylating reaction is not disturbed. In polypeptides as defined in (i) and (ii) above, the amino acid sequence on the C-terminal side of the active center may be similar to that on the C-terminal side of the active center of a class A acid phosphatase.

A specific embodiment of the phosphorylating enzyme which can be used in the method of the present invention is an enzyme in which X¹, X², and X⁴ in the sequence (1) are any of (A1) to (A8) below:
(A1) X¹ being glycine and X⁴ being threonine; preferably X¹ being glycine, X² being serine, alanine, or aspartic acid, and X⁴ being threonine; further preferably X¹ being glycine, X² being alanine, and X⁴ being threonine;
(A2) X¹ being glycine and X⁴ being serine; preferably X¹ being glycine, X² being serine, and X⁴ being serine;
(A3) X¹ being alanine and X⁴ being serine; preferably X¹ being alanine, X² being serine, and X⁴ being serine;
(A4) X¹ being alanine and X⁴ being threonine; preferably X¹ being alanine, X² being serine, and X⁴ being threonine;
(A5) X¹ being alanine and X⁴ being alanine; preferably X¹ being alanine, X² being serine, and X⁴ being alanine;
(A6) X¹ being glycine and X⁴ being alanine; preferably X¹ being glycine, X² being serine, and X⁴ being alanine;
(A7) X¹ being phenylalanine and X⁴ being threonine; preferably X¹ being phenylalanine, X² being serine, and X⁴ being threonine; and
(A8) X¹ being phenylalanine and X⁴ being serine; preferably X¹ being phenylalanine, X² being serine, and X⁴ being serine.

Among these embodiments (A1) to (A8), the embodiment (A1) is preferable from the viewpoint that the phosphorylation of glycerol is performed with higher efficiency.

A more specific embodiment of the phosphorylating enzyme which can be used in the method of the present invention is an enzyme in which X¹, X², and X⁴ in the sequence (1) are any of (B1) to (B11) below:
(B1) X¹ being glycine, X² being serine, X³ being tyrosine, and X⁴ being threonine;
(B2) X¹ being glycine, X² being alanine, X³ being tyrosine, and X⁴ being threonine;
(B3) X¹ being glycine, X² being serine, X³ being tyrosine, and X⁴ being serine;
(B4) X¹ being glycine, X² being aspartic acid, X³ being tyrosine, and X⁴ being threonine;
(B5) X¹ being alanine, X² being serine, X³ being tyrosine, and X⁴ being serine;
(B6) X¹ being alanine, X² being serine, X³ being tyrosine, and X⁴ being threonine;
(B7) X¹ being alanine, X² being serine, X³ being tyrosine, and X⁴ being alanine;
(B8) X¹ being glycine, X² being serine, X³ being tyrosine, and X⁴ being alanine;
(B9) X¹ being phenylalanine, X² being serine, X³ being tyrosine, and X⁴ being threonine;
(B10) X¹ being phenylalanine, X² being serine, X³ being tyrosine, and X⁴ being serine; and
(B11) X¹ being glycine, X² being serine, X³ being tryptophan, and X⁴ being serine.

Among these embodiments (B1) to (B11), the embodiment (B1) is preferable from the viewpoint that the phosphorylation of glycerol is performed with higher efficiency.

In the amino acid sequence of a class A acid phosphatase, the position where the active center is positioned is known; thus those skilled in the art would be able to determine where to place the active center represented by the sequence (1), as appropriate in the amino acid sequence of a phosphorylating enzyme to be used in the present invention.

Additionally, in the amino acid sequence of a class A acid phosphatase, it is known that two active centers are present; the active center represented by the sequence (1) is located on the N-terminal side of these two active centers. The other of these two active centers, which is the active center located on the C-terminal side, usually has an amino acid sequence of RX₅HX₂S wherein each X independently denotes any amino acid, and is joined to the active center represented by sequence (1), usually via 20 to 50 amino acids, preferably via 30 to 40 amino acids, from the C-terminal end of the active center represented by the sequence (1).

Also in the amino acid sequence of a class A acid phosphatase, the amino acid sequences on both the N-terminal and C-terminal sides of the active center represented by the sequence (1) are known; thus those skilled in the art would be able to determine the amino acid sequence located outside the active center represented by the sequence (1), as appropriate in the amino acid sequence of a phosphorylating enzyme to be used in the present invention.

The position of the active center represented by the sequence (1) in the entire amino acid sequence of a phosphorylating enzyme according to the present invention is not limited in particular, as long as the enzyme has an activity to catalyze the phosphorylation of glycerol. For example, the active center represented by the sequence (1) is placed at such a position that the histidine (His) residue at the 7-th position in the amino acid sequence represented by the sequence (1) is located between positions 120 and 160, preferably between positions 125 and 155, further preferably between positions 130 and 150, from the N-terminus of the polypeptide employed as the phosphorylating enzyme (class A acid phosphatase). In addition, the phosphorylating enzyme which is used in the method for producing a phosphorylated glycerol according to the present invention may have, besides the active center represented by the sequence (1), a histidine residue as a second active center at such a position that the histidine residue is located between positions 160 and 200, preferably between positions 165 and 195, further preferably between positions 170 and 190, from the N-terminus of the polypeptide employed as the phosphorylating enzyme.

The amino acid sequence set forth in SEQ ID NO: 2 specifically is: PDERLVLAPPPAPGSAAQ (SEQ ID NO: 2) [0025]

The amino acid sequence set forth in SEQ ID NO: 2 may be joined directly to a stretch of the amino acid sequence coding for the active center represented by the sequence (1). Alternatively, the amino acid sequence set forth in SEQ ID NO: 2 may be joined to a stretch of the amino acid sequence coding for the active center represented by the sequence (1), for example, via 70 to 130 amino acids, preferably via 80 to 120 amino acids, further preferably via 90 to 110 amino acids, as long as the activity of the enzyme to catalyze the glycerol phosphorylating reaction is not disturbed. In addition, the phosphorylating enzyme according to the present invention may comprise any amino acid sequence, for example, of 5 to 50 amino acids, preferably of 8 to 40 amino acids, further preferably of 10 to 30 amino acids, on the N-terminal side of the amino acid sequence set forth in SEQ ID NO: 2, as long as the activity of the enzyme to catalyze the glycerol phosphorylating reaction is not disturbed. Further, the phosphorylating enzyme according to the present invention may comprise any amino acid sequence, for example, of 60 to 120 amino acids, preferably of 70 to 110 amino acids, further preferably of 80 to 100 amino acids, on the C-terminal side of a stretch of the amino acid sequence coding for the active center represented by the sequence (1), as long as the activity of the enzyme to catalyze the glycerol phosphorylating reaction is not disturbed.

In the amino acid sequence in which a phosphorylating enzyme according to the present invention is composed, the position of the amino acid sequence set forth in SEQ ID NO: 2 is not limited in particular, as long as the enzyme has an activity to catalyze the phosphorylation of glycerol. For example, the amino acid sequence set forth in SEQ ID NO: 2 is placed at such a position that the first amino acid residue in the amino acid sequence set forth in SEQ ID NO: 2 is located between positions 5 and 40, preferably between positions 8 and 35, further preferably between positions 10 and 30, from the N-terminus of the polypeptide employed as the phosphorylating enzyme according to the present invention.

In addition, in a polypeptide as defined in (ii) above, the number of amino acids that are substituted, deleted, inserted, or added in the amino acid sequence set forth in SEQ ID NO: 2 is limited, with the proviso that the polypeptide has an activity to catalyze the glycerol phosphorylating reaction which is comparable or superior to that of the corresponding parent polypeptide. The amino acid sequence set forth in SEQ ID NO: 2 has 1 to 3 amino acids, or preferably 1 or 2 amino acids substituted, deleted, inserted, or added therein. Methods for obtaining mutants that have an amino acid(s) substituted, deleted, inserted, or added will be described in detail below in the section entitled "2. Preparation of phosphorylating enzymes."

In cases of adding an amino acid to a given amino acid sequence, the number of amino acids to be added is from 1 to 10, more preferably from 1 to 8, further preferably from 1 to 5, particularly preferably from 1 to 3, or 1 or 2. Furthermore, in addition to the added amino acid(s), a sequence for protein purification, such as His tag, may optionally added at the C-terminus of the modified amino acid sequences.

In cases of deleting an amino acid from a given amino acid sequence, the number of amino acids to be deleted is from 1 to 10, more preferably from 1 to 8, further preferably from 1 to 5, particularly preferably from 1 to 3, or 1 or 2.

In cases of inserting an amino acid into a given amino acid sequence, the number of amino acids to be inserted is from 1 to 10, more preferably from 1 to 8, further preferably from 1 to 5, particularly preferably from 1 to 3, or 1 or 2.

In cases of substituting an amino acid in a given amino acid, a conservative substitution can be made based on properties of the side-chain functional groups of amino acids. In addition, a non-conservative substitution may be made in which the property of an amino acid residue before the substitution is different from that of an amino acid residue after the substitution, as long as the resulting polypeptide has an activity to catalyze the glycerol phosphorylating reaction which is comparable or superior to that of the corresponding parent polypeptide. Natural amino acids are classified, based on their side-chain functional groups, into categories of non-polar amino acids, of non-charged amino acids, of acidic amino acids, and of basic amino acids. A conservative substitution refers to a substitution having an amino acid residue that belongs to the same category into which the parent amino acid residue and the substituted amino acid residue are classified. Here, a "non-polar amino acid" specifically includes alanine, valine, leucine, isoleucine, proline, methionine, phenylalanine, and tryptophan; a "non-charged amino acid" specifically includes
glycine, serine, threonine, cysteine, tyrosine, asparagine, and glutamine; an "acidic amino acid" specifically includes aspartic acid and glutamic acid; and a "basic amino acid" specifically includes lysine, arginine, and histidine.

A phosphorylating enzyme according to the present invention may further comprise at least one of the amino acid sequences as defined in (iii) to (vi) below:
(iii) a polypeptide that has, on the N-terminal side of the amino acid sequence set forth in SEQ ID NO: 2, the amino acid sequence set forth in SEQ ID NO: 40 and has an activity to catalyze the glycerol phosphorylating reaction;
(iv) a polypeptide that has, on the N-terminal side of the amino acid sequence set forth in SEQ ID NO: 2, an amino acid sequence having 1 to 3 amino acids substituted, deleted, inserted, or added in the amino acid sequence set forth in SEQ ID NO: 40 and has an activity to catalyze the glycerol phosphorylating reaction which is comparable or superior to that of the polypeptide as defined in (iii);
(v) a polypeptide that has, on the C-terminal side of the amino acid sequence set forth in SEQ ID NO: 2, the amino acid sequence set forth in SEQ ID NO: 41 and has an activity to catalyze the glycerol phosphorylating reaction; and
(vi) a polypeptide that has, on the C-terminal side of the amino acid sequence set forth in SEQ ID NO: 2, an amino acid sequence having 1 to 10 amino acids substituted, deleted, inserted, or added in the amino acid sequence set forth in SEQ ID NO: 41 and has an activity to catalyze the glycerol phosphorylating reaction which is comparable or superior to that of the polypeptide as defined in (v).

SEQ ID NO: 40 corresponds to the amino acid sequence in SEQ ID NO: 9 that is located on the N-terminal side of the amino acid sequence corresponding to SEQ ID NO: 2. SEQ ID NO: 41 corresponds to the amino acid sequence in SEQ ID NO: 9 that is located on the C-terminal side of the amino acid sequence corresponding to SEQ ID NO: 2. The specific amino acid sequences of these are as indicated below.
SEQ ID NO: 40:
MDTSATAQGGILPDSAA

The same as in the above-described polypeptides as defined in (ii) applies to polypeptides as defined in (iv) or (vi) above, for the number of amino acids to be substituted, deleted, inserted, or added, the type of amino acid to be substituted, and the like.

A phosphorylating enzyme according to the present invention includes more specifically one including a polypeptide as defined in (vii) or (viii) below:
(vii) a polypeptide that has the amino acid sequence set forth in any of SEQ ID NOs: 9, 15 to 17, 19, 22, and 25; or
(viii) a polypeptide that includes an amino acid sequence having, in the amino acid sequence set forth in any of SEQ ID NOs: 9, 15 to 17, 19, 22, and 25, 1 to 10 amino acids substituted, deleted, inserted, or added in a region outside the amino acid sequence set forth in SEQ ID NO: 2 and has an activity to catalyze the glycerol phosphorylating reaction which is comparable or superior to that of the polypeptide as defined in (vii).

The same as in the above-described polypeptides as defined in (ii) applies to polypeptides as defined in (iv) or (vi) above, for the number of amino acids to be substituted, deleted, inserted, or added, the type of amino acid to be substituted, and the like.

An embodiment of the phosphorylating enzyme according to the present invention is a class A acid phosphatase that comprises the active center including an amino acid sequence represented by the sequence (1) (SEQ ID NO: 1) and a polypeptide as defined in (I) or (III) as indicated below and has an activity to catalyze the glycerol phosphorylating reaction:

-X¹-X²-X³-Pro-Ser-Gly-His-X⁴- (1)

wherein,
X¹ denotes glycine, alanine, or phenylalanine;
X² denotes any amino acid;
X³ denotes tyrosine or tryptophan; and
X⁴ denotes threonine, serine, or alanine;
with the proviso that when X¹ is phenylalanine, X⁴ is threonine or serine, and
   (I) a polypeptide that has the amino acid sequence set forth in SEQ ID NO: 2, on the N-terminal side of the active center represented by the sequence (1), or
   (II) a polypeptide that comprises, on the N-terminal side of the active center represented by the sequence (1), an amino acid sequence having 1 to 3 amino acids substituted, deleted, inserted, or added in the amino acid sequence set forth in SEQ ID NO: 2 and has an activity to catalyze the glycerol phosphorylating reaction which is comparable or superior to that of the polypeptide as defined in (I).

X² in the sequence (1) may be any amino acid and preferably is an uncharged amino acid, a non-polar amino acid, or an acidic amino acid, further preferably serine, alanine, or aspartic acid, with serine being particularly preferable.

Further, another embodiment of the phosphorylating enzyme which can be used in the present invention and which comprises SEQ ID NO: 2 as defined in claim 1 includes any of the polypeptides as defined in (III) to (VI) below:
(III) a polypeptide that has the amino acid sequence set forth in SEQ ID NO: 32, on the N-terminal side of the amino acid sequence represented by the sequence (1);
(IV) a polypeptide that has, on the N-terminal side of the amino acid sequence represented by the sequence (1), an amino acid sequence having 1 to 10 amino acids substituted, deleted, inserted, or added in the amino acid sequence set forth in SEQ ID NO: 32 and has an activity to catalyze the glycerol phosphorylating reaction which is comparable or superior to that of its corresponding polypeptide as defined in (III);
(V) a polypeptide that has the amino acid sequence set forth in any of SEQ ID NOs: 33 to 39 and 111 to 115, on the C-terminal side of the amino acid sequence represented by the sequence (1); and
(VI) a polypeptide that has, on the C-terminal side of the amino acid sequence represented by the sequence (1), an amino acid sequence having 1 to 10 amino acids substituted, deleted, inserted, or added in the amino acid sequence set forth in any of SEQ ID NOs: 33 to 39 and 111 to 115 and has an activity to catalyze the glycerol phosphorylating reaction which is comparable or superior to that of its corresponding polypeptide as defined in (V).

SEQ ID NOs: 26 to 32, 106, and 107 correspond to the amino acid sequences on the N-terminal side of the active center represented by sequence (1), in SEQ ID NOs: 3 to 9, 104, and 105, respectively. The specific amino acid sequences set forth in SEQ ID NOs: 26 to 32, 106, and 107 are as indicated below.

SEQ ID NOs: 108 to 110 correspond to the amino acid sequences on the N-terminal side of the active center, in SEQ ID NOs: 10 to 12 representing the amino acid sequences of class A acid phosphatases. The specific amino acid sequences set forth in SEQ ID NOs: 108 to 110 are as indicated below.

SEQ ID NOs: 33 to 39, 111, and 112 correspond to the amino acid sequences on the C-terminal side of the active center represented by sequence (1), in SEQ ID NOs: 3 to 9, 104, and 105, respectively. The specific amino acid sequences set forth in SEQ ID NOs: 33 to 39, 111, and 112 are as indicated below.

SEQ ID NOs: 113 to 115 correspond to the amino acid sequences on the C-terminal side of the active center, in SEQ ID NOs: 10 to 12 representing the amino acid sequences of class A acid phosphatases. The specific amino acid sequences set forth in SEQ ID NOs: 113 to 115 are as indicated below.

In the present invention, a phosphorylating enzyme may comprise a combination of the sequence (1), the N-terminal sequence (SEQ ID NO: 32), and one selected from the C-terminal sequences (SEQ ID NOs: 33 to 39 and 111 to 115), as long as the activity of the enzyme to catalyze the glycerol phosphorylating reaction is not disturbed.

The same as in the above-described polypeptides as defined in (ii) applies to polypeptides as defined in (IV) or (VI), for the number of amino acids to be substituted, deleted, inserted, or added, the type of amino acid to be substituted, and the like.

A phosphorylating enzyme according to the present invention includes more specifically one including a polypeptide as defined in (VII) or (VIII) below:
(VII) a polypeptide that has the amino acid sequence set forth in SEQ ID NO: 9, or
(VIII) a polypeptide that includes an amino acid sequence having, in the amino acid sequence set forth in SEQ ID NO: 9, 1 to 10 amino acids substituted, deleted, inserted, or added in a region outside the active center including the amino acid sequence represented by the sequence (1) and has an activity to catalyze the glycerol phosphorylating reaction which is comparable or superior to that of its corresponding polypeptide as defined in (VII).

The same as in the above-described polypeptides as defined in (ii) applies to polypeptides as defined in (VIII), for the number of amino acids to be substituted, deleted, inserted, or added, the type of amino acid to be substituted, and the like. In addition, when an amino acid sequence in which one of these polypeptides is composed comprises a sequence corresponding to the amino acid sequence set forth in SEQ ID NO: 2, it is preferable that a mutation is introduced into a region outside of such a sequence.

In a method for producing a phosphorylated glycerol according to the present invention, the phosphorylating enzyme may be used alone by selecting one phosphorylating enzyme from among phosphorylating enzymes of one type, or alternatively in combination of two or more phosphorylating enzymes.

### 2.Preparation of phosphorylating enzymes

An enzyme as described above having a catalytic activity in a glycerol phosphorylating reaction can be obtained, for example, from a culture of a microorganism that possesses such an enzyme. Alternatively, an enzyme as described above having a catalytic activity in a glycerol phosphorylating reaction can be prepared as a recombinant protein in accordance with conventionally known methods, by obtaining a base sequence encoding such a phosphorylating enzyme of interest from a microorganism possessing the enzyme.

A phosphorylating enzyme including one of the amino acid sequences set forth in SEQ ID NOs: 3 to 9, 104, and 105 can be obtained from particular microorganisms as described below, respectively.

Accordingly, a phosphorylating enzyme including the amino acid sequence set forth in SEQ ID NO: 3 is available from *Gluconacetobacter hansenii.* A phosphorylating enzyme including the amino acid sequence set forth in SEQ ID NO: 4 is available from *Xanthomonas oryzae*; a phosphorylating enzyme including the amino acid sequence set forth in SEQ ID NO: 5 is available from *Brevundimonas diminuta*; a phosphorylating enzyme including the amino acid sequence set forth in SEQ ID NO: 6 is available from *Sphingomonas trueperi*; a phosphorylating enzyme including the amino acid sequence set forth in SEQ ID NO: 7 is available from *Zymomonas mobilis*; a phosphorylating enzyme including the amino acid sequence set forth in SEQ ID NO: 8 is available from *Desulfovibrio magneticus*; a phosphorylating enzyme including the amino acid sequence set forth in SEQ ID NO: 9 is available from *Gluconobacter oxydans.* A phosphorylating enzyme including the amino acid sequence set forth in SEQ ID NO: 104 is available from *Serratia plymutica.* A phosphorylating enzyme including the amino acid sequence set forth in SEQ ID NO: 105 is available from *Rahnella aquatilis.*

A phosphorylating enzyme including one of the amino acid sequences set forth in SEQ ID NOs: 3 to 9, 104, and 105 can be obtained by culturing the corresponding microorganism and treating cultured cells. To this end, more specifically, the methods described below are illustrated.

In cases when a phosphorylating enzyme including the amino acid sequence set forth in SEQ ID NO: 3 is obtained from cultured cells of *Gluconacetobacter hansenii,* culture conditions can be set as appropriate from culture methods known for this microorganism, for example, including aerobic conditions at a culture temperature of 30°C in a medium containing 1% polypeptone, 0.2% dry yeast extract, and 0.1% magnesium sulfate.

In cases when a phosphorylating enzyme including the amino acid sequence set forth in SEQ ID NO: 4 is obtained from cultured cells of *Xanthomonas oryzae,* culture conditions can be set as appropriate from culture methods known for this microorganism, for example, including aerobic conditions at a culture temperature of 30°C in a medium containing 1% polypeptone, 0.2% dry yeast extract, and 0.1% magnesium sulfate.

In cases when a phosphorylating enzyme including the amino acid sequence set forth in SEQ ID NO: 5 is obtained from cultured cells of *Brevundimonas diminuta,* culture conditions can be set as appropriate from culture methods known for this microorganism, for example, including aerobic conditions at a culture temperature of 30°C in a medium containing 1% polypeptone, 0.2% dry yeast extract, and 0.1% magnesium sulfate.

In cases when a phosphorylating enzyme including the amino acid sequence set forth in SEQ ID NO: 6 is obtained from cultured cells of *Sphingomonas trueperi,* culture conditions can be set as appropriate from culture methods known for this microorganism, for example, including aerobic conditions at a culture temperature of 26°C in a medium containing 5% peptone and 3% beef extract.

In cases when a phosphorylating enzyme including the amino acid sequence set forth in SEQ ID NO: 7 is obtained from cultured cells of *Zymomonas mobilis,* culture conditions can be set as appropriate from culture methods known for this microorganism, for example, including aerobic conditions at a culture temperature of 30°C in a medium containing 0.5% dry yeast extract and 2% glucose.

In cases when a phosphorylating enzyme including the amino acid sequence set forth in SEQ ID NO: 8 is obtained from cultured cells of *Desulfovibrio magneticus,* culture conditions can be set as appropriate from culture methods known for this microorganism, for example, including anaerobic conditions at a culture temperature of 30°C in a medium containing 0.02% potassium dihydrogenphosphate, 60 ppm ammonium chloride, 50 ppm cysteine, 0.058% sodium fumarate, 0.044% sodium pyruvate, 0.2% iron quinate solution, 0.4% Wolfe's vitamin solution, and 0.2% Wolfe's mineral solution.

In cases when a phosphorylating enzyme including the amino acid sequence set forth in SEQ ID NO: 9 is obtained from cultured cells of *Gluconobacter oxydans,* culture conditions can be set as appropriate from culture methods known for this microorganism, for example, including aerobic conditions at a culture temperature of 30°C in a medium containing 0.5% polypeptone, 0.5% dry yeast extract, 0.5% glucose, and 0.1% magnesium sulfate.

In cases when a phosphorylating enzyme including the amino acid sequence set forth in SEQ ID NO: 104 is obtained from cultured cells of *Serratia plymutica,* culture conditions can be set as appropriate from culture methods known for this microorganism, for example, including aerobic conditions at a culture temperature of 37°C in a medium containing 1% polypeptone, 0.2% dry yeast extract, and 0.1% magnesium sulfate.

In cases when a phosphorylating enzyme including the amino acid sequence set forth in SEQ ID NO: 105 is obtained from cultured cells of *Rahnella aquatilis,* culture conditions can be set as appropriate from culture methods known for this microorganism, for example, including aerobic conditions at a culture temperature of 30°C in a medium containing 1% polypeptone, 0.2% dry yeast extract, and 0.1% magnesium sulfate.

After the culturing is completed, usual methods for enzyme isolation can be employed to collect the phosphorylating enzyme from the culture. Methods for enzyme isolation include, for example, those in which cultured cells are disrupted by sonication to prepare a cell extract solution, followed by known methods for enzyme purification, such as ammonium sulfate fractionation, ion exchange, chromatofocusing, gel filtration, and the like. In addition, a cultured broth containing an enzyme secreted outside the cells may be used as an enzyme solution.

Also, a phosphorylating enzyme including one of the amino acid sequences set forth in SEQ ID NOs: 3 to 9, 104, and 105 can be prepared as a recombinant protein, which then can be used in the method of the present invention as long as it falls under claim 1. In cases when the phosphorylating enzyme is obtained as a recombinant protein, the preparation of the recombinant protein can be performed based on known genetic engineering procedures. In usual cases, a phosphorylating enzyme can be produced as a recombinant protein by inserting a polynucleotide (DNA fragment) encoding the amino acid sequence of a given phosphorylating enzyme into a vector, introducing the vector into a host cell to obtain a transformant, and culturing the transformant. The polynucleotide sequences that encodes phosphorylating enzymes including the amino acid sequences set forth in SEQ ID NOs: 3 to 9, 104, and 105 are represented by SEQ ID NOs: 94 to 100, 122, and 123, respectively. In such cases, a sequence derived from the vector, which is described below, may be incorporated into the polynucleotide, with the proviso that the sequence does not disturb the activity of the phosphorylating enzyme. In addition, the polynucleotide encoding the phosphorylating enzyme may be optimized so that the codon usage is adapted to that of the host cell to be used.

As a vector, use can be made of any vector that allows the expression of the gene encoded by the DNA fragment in an appropriate host cell. Such a vector includes, for example, a plasmid vector, a phage vector, a cosmid vector, a shuttle vector, and the like.

In the present invention, a vector may comprise a regulatory element(s) that is/are operably linked to a polynucleotide (DNA fragment) encoding an enzyme mutant as described above, for example, a promoter, for example, a functional promoter such as a T7 promoter, a *lacUV5* promoter, a *trp* promoter, a *trc* promoter, a *tac* promoter, an *lpp* promoter, a *tufB* promoter, a *recA* promoter, and a pL promoter; a transcriptional element, for example, an enhancer, a CCAAT box, a TATA box, and an SPI site; a signal sequence, particularly a nuclear localization signal, an endoplasmic reticulum localization signal, PTS1 (Peroxisomal targeting signal 1), and PTS2; and others. Here, by operably linked, it is meant that various regulatory
elements such as a promoter and an enhancer that regulate the expression of a gene, and a given gene are linked in a state where they are allowed to operate within the host cell.

A vector to be used in the present invention can be selected as appropriate from among vectors commonly used in the art and can be used. Examples of such a vector includes, more specifically, pET22b(+), pET39b(+), and others.

Host cells are not limited in particular, as long as they can be transformed with an expression vector comprising a polynucleotide (DNA fragment) encoding an above-mentioned phosphorylating enzyme and allow the expression of the phosphorylating enzyme encoded by the polynucleotide.

Examples of such host cells include, more specifically, microorganisms, for example, bacteria of the genera *Escherichia, Bacillus, Pseudomonas, Serratia, Brevibacterium, Corynebacterium, Streptococcus, Lactobacillus* and the like; actinomycetes of the genera *Rhodococcus, Streptomyces,* and the like; yeasts of the genera *Saccharomyces, Kluyveromyces, Schizosaccharomyces, Zygosaccharomyces, Yarrowia, Trichosporon, Rhodosporidium, Pichia, Candida,* and the like; fungi of the genera *Neurospora, Aspergillus, Cephalosporium, Trichoderma,* and the like; and the others. Among these, bacteria is preferred from the viewpoint of the efficiency of introduction of genes into host cells and of gene expression. Cells of *Escherichia coli,* a bacterium of the genus *Escherichia,* are preferable.

Methods for transformation are known and can be selected as appropriate, depending on the type of host cell to be transformed and others. Examples of methods for transformation specifically include methods using competent cells, electroporation, heat shock, or the like.

Transformants can be cultured, based on conventionally known culture conditions, depending on the type of cell used as host cell. In cases when *Escherichia coli* cells are used as host cell, for example, culture conditions can be a culture temperature of 20 to 40°C, a culture period of 6 to 24 hours, and a pH of the culture medium of 5 to 8 under aerobic conditions, for example, by shaking culture or aeration culture.

A culture medium that is to be used in the present invention is not limited in particular, as long as it allows the host cell to grow and to produce an enzyme mutant of the present invention. Use can be made of media usually employed in the art which contain required amounts of a carbon source, a nitrogen source, inorganic substances, amino acids, nucleic acids, vitamins, and others. In addition, an antibiotic such as ampicillin or tetracycline may be added to the medium as necessary, during the culturing period.

Methods for collecting the phosphorylating enzyme of interest after the culturing is completed may follow the procedures for collecting the phosphorylating enzyme from cultured cells as previously described.

Introduction of a mutation (substitution, deletion, insertion, and/or addition) into a given amino acid sequence can be carried out according to conventionally known methods.

Methods for introducing an amino acid mutation include, for example, site-directed mutagenesis, which can be performed by using Inverse PCR-based procedures or commercially available kits, such as QuikChange II Kit (manufactured by Stratagene). These approaches make it possible to obtain a polynucleotide encoding a phosphorylating enzyme having a desired mutation, based on the base sequences set forth in SEQ ID NOs: 94 to 100, 122, and 123. A phosphorylating enzyme mutant can be prepared as a recombinant protein by using the resulting polynucleotide and following known genetic engineering procedures similar to the above.

A phosphorylating enzyme that includes a polypeptide having a mutation as described above includes one in which the amino acid sequence identity of such a polypeptide to the corresponding polypeptide before the mutation is introduced is 20% or higher, preferably 30% or higher, further preferably 40% or higher and which is capable of catalyzing the glycerol phosphorylating reaction at a level comparable or superior to that of the phosphorylating enzyme including the corresponding polypeptide before the mutation is introduced.

The sequence identity between two polypeptides is expressed as a numerical value obtained by optimally aligning the two polypeptide to be compared, dividing the number of positions at which amino acids are identical in both sequences by the total number of the amino acids compared, and multiplying the quotient by 100.
Specifically, the sequence identity between two polypeptide can be determined by using a Maximum matching program, "GENETYX Ver. 10" (GENETYX CORPORATION).

### 3. Phosphorylation of glycerol

In a method for phosphorylating glycerol according to the present invention, the phosphorylation reaction can be carried out by adding glycerol, a phosphate group donor, and an above-described phosphorylating enzyme to a solvent to prepare a reaction composition. Alternatively, the phosphorylation of glycerol may be performed by allowing a culture of an above-described microorganism or transformant that is capable of producing an above-described phosphorylating enzyme to act on glycerol in the presence of a phosphate group donor.

In the method of the present invention, a culture means a cultured broth containing cells, a cultured cell mass, or a treated material thereof. Here, a treated material thereof means, for example, a cell-free extract, a freeze-dried cell preparation, an acetone-dried cell preparation, or a disrupted material of these cell preparations. Such cultures can also be used as an immobilized enzyme or cell preparation. Immobilization can be performed using methods well known to those skilled in the art, for example, covalent bonding, physical adsorption, entrapment, and the like.

A phosphate group donor is not limited in particular, as long as it functions as a source of the phosphate for an enzyme mutant of the present invention, whereby a phosphorylated glycerol is yielded, and includes, for example, a polyphosphoric acid represented by formula (1): Hₙ₊₂PₙO₃ₙ₊₁, wherein n ≥ 2. The degree of polymerization of the polyphosphoric acid is not limited in particular, and preferably 2 to 10000 structural units described above are polymerized, further preferably 2 to 1000 structural units described above are polymerized. Examples of a polyphosphoric acid specifically include diphosphoric acid (n = 2; pyrophosphoric acid), triphosphoric acid (n = 3), tetraphosphoric acid (n = 4), and the like. It is also possible that in the formula (1), polyphosphoric acids in which 5 or more phosphoric acid groups are polymerized (n = 5 or more) are suitably used as a phosphate group donor. A mixture of two or more of these polyphosphoric acids with different degrees of polymerization may be used as a phosphate group donor.

Further, as a phosphate group donor which can be used in the present invention, use can also be made of esters, salts with alkali metals such as sodium and potassium, salts with alkaline earth metals such as calcium, and ammonium salts of the polyphosphoric acid described above. Examples of esters of polyphosphoric acids specifically include adenosine diphosphate (ADP), adenosine triphosphate (ATP), and others. Examples of salts of polyphosphoric acids specifically include sodium pyrophosphate (Na₄P₂O₇), sodium tripolyphosphate (Na₅P₃O₁₀), potassium tripolyphosphate (K₅P₃O₁₀), sodium tetrapolyphosphate (Na₆P₄O₁₃), sodium hexametaphosphate, ammonium pyrophosphate salt, ammonium tripolyphosphate salt, and others. Moreover, a phosphate group donor includes para-nitrophenyl phosphate, acetyl phosphate, and the like, in addition to the polyphosphoric acid described above.

In the method of the invention, the phosphate group donor preferably includes polyphosphoric acids, such as diphosphoric acid, triphosphoric acid, and tetraphosphoric acid; esters of polyphosphoric acids, such as adenosine diphosphate (ADP) and adenosine triphosphate (ATP); alkali metal salts of polyphosphoric acids, such as sodium pyrophosphate, sodium tripolyphosphate, potassium tripolyphosphate, sodium tetrapolyphosphate, and sodium hexametaphosphate; para-nitrophenyl phosphate and acetyl phosphate. More preferably, the phosphate group donor includes polyphosphoric acids, and esters and alkali metal salts thereof. Further preferably, the phosphate group donor includes polyphosphoric acids and alkali metal salts thereof, with polyphosphoric acids being preferable from the viewpoint that they are available at low cost. These phosphate group donors may be used alone or in combination of two or more.

Examples of the concentration at which a phosphorylating enzyme is used in the reaction include 0.0001 % to 5% by weight, preferably 0.001% to 1% by weight, further preferably 0.001% to 0.5% by weight, in the reaction composition. Examples of the concentration of a phosphate group donor include 0.5% to 20% by weight, preferably 1% to 15% by weight, further preferably 2% to 10% by weight. Examples of the concentration of glycerol include 0.1% to 80% by weight, preferably 1% to 60% by weight, further preferably 5% to 50% by weight.

Examples of the addition ratio of a phosphate group donor per part by weight of a phosphorylating enzyme (polypeptide) include 100 to 30000 parts by weight, preferably 250 to 20000 parts by weight, further preferably 500 to 15000 parts by weight. Examples of the addition ratio of glycerol per part by weight of a phosphorylating enzyme (polypeptide) include 100 to 100000 parts by weight, preferably 500 to 70000 parts by weight, further preferably 1000 to 50000 parts by weight.

Examples of the reaction temperature at which the phosphorylation reaction is carried out include 10 to 60°C, preferably 20 to 50°C, further preferably 30 to 40°C.

Examples of the optimal pH of the reaction solution when the phosphorylation reaction is carried out include pH 2 to 7, preferably pH 4 to 6, more preferably pH 3 to 5, further preferably pH 4 to 5. The pH of the reaction solution can be adjusted using hydrochloric acid, citric acid, gluconic acid, succinic acid, acetic acid, tartaric acid, sorbic acid, lactic acid, maleic acid, sulfuric acid, phosphoric acid, malic acid, arginine, aqueous ammonia, diisopropanolamine, diethanolamine, triisopropanolamine, triethanolamine, monoethanolamine, potassium hydroxide, calcium hydroxide, sodium hydroxide, and buffering agents such as salts thereof, and conventionally known buffering agents.

The reaction period when the phosphorylation reaction is carried out is not limited in particular, as long as a phosphorylated glycerol can be produced, and for example, is 1 to 50 hours, preferably 5 to 40 hours, further preferably 10 to 30 hours.

As a reaction solvent, an aqueous solvent, such as ion-exchanged water, distilled water, and ultrapure water can be used in usual cases. In addition, an aqueous solvent may contain an organic solvent such as methanol, trimethylamine, triethylamine, and dimethyl sulfoxide (DMSO), or a non-ionic surfactant such as polyoxyethylene lauryl ether, polyoxyethylene dodecyl ether, polyethylene glycol p-octylphenyl ether (whose trade name is Triton X-100), and polyoxyethylene sorbitan monolaurate (whose trade name is Tween 20), in a range in which advantageous effects of the present invention are not inhibited.

Examples of amounts of an organic solvent added are usually 0.1% to 50% by weight, preferably 1% to 30% by weight, further preferably 5% to 20% by weight. Examples of amounts of a non-ionic surfactant added are usually 0.0001% to 0.1% by weight, preferably 0.0005% to 0.05% by weight, further preferably 0.001% to 0.01% by weight.

Methods for collecting a phosphorylated glycerol yielded in the above-described reaction can be, for example, methods using various types of column chromatography, such as ion-exchange chromatography, affinity chromatography, adsorption column chromatography, gel filtration chromatography, and reverse phase chromatography, and methods by which the phosphorylated glycerol is precipitated by the addition of an organic solvent such as ethanol or acetonitrile.

In cases when the glycerol phosphorylating reaction is performed under conditions in amounts of 5000 to 50000 parts by weight of glycerol per part by weight of a phosphorylating enzyme (polypeptide) and at a pH of 4 to 6, the phosphorylating enzyme (polypeptide) used is, for example, one including the amino acid sequence set forth in SEQ ID NO: 9.

In a method for producing a phosphorylated glycerol according to the present invention, it is possible to achieve a further higher efficiency of the phosphorylation by adjusting conditions for the phosphorylation reaction and selecting an appropriate phosphorylating enzyme. For example, when the glycerol phosphorylating reaction is performed under conditions in amounts of 5000 to 50000 parts by weight of glycerol per part by weight of a phosphorylating enzyme (polypeptide) and at a pH of 3 to 5, the phosphorylating enzyme (polypeptide) used includes, for example, a phosphorylating enzyme including the amino acid sequence set forth in SEQ ID NO: 9.

Further, when the glycerol phosphorylating reaction is performed under conditions in amounts of 2000 to 10000 parts by weight of glycerol per part by weight of a phosphorylating enzyme (polypeptide) and at a pH of 4 to 5, the phosphorylating enzyme (polypeptide) used includes, for example, a phosphorylating enzyme including the amino acid sequence set forth in SEQ ID NO: 9.

In the method of the present invention, the hydroxyl group position at which the phosphate group is attached is not limited in particular, as long as glycerol is phosphorylated. A specific example of the phosphorylated glycerol yielded in the method of the present invention is glycerol-3-phosphate (also referred to as glycerophosphate). Glycerophosphate occurs in the α and β forms; the glycerophosphate obtained by method of the present invention can include either of these forms. Preferably, the method of the present invention yields glycerophosphate in the α form. In examples described below, it has been found that glycerol-3-phosphate in the α form (also referred to as α-glycerophosphate) is predominantly produced.

### EXAMPLES

### [Preparation of phosphorylating enzymes]

### Obtaining of genes for phosphorylating enzymes

Genomic DNA was prepared from each strain of the microorganism indicated below in Table 1 and a gene for a phosphorylating enzyme was amplified by PCR method using primers corresponding to the respective strains of the microorganisms.

**[Table 1]**

| SEQ ID NO: of the amino acid sequence of the phosphoryl ating enzyme | SEQ ID NO: of the base sequence of a gene encoding the phosphorylating enzyme | Derived from | Primer SEQ ID NO: | Primer sequence (5' to 3') | |
|---|---|---|---|---|---|
| 3 | 94 | Gluconacetobacter hansenii | 42 | FW | TGCACCCATGGACGCAGGGTATCTAACACCTGCGAC |
| | | | 43 | RV | CAATAAGCTTGAGCGGAGAATGGGCCGCAGCATCATG |
| 4 | 95 | Xanthomonas oryzae | 44 | FW | GATCGCCATGGACGCGCATGGATACCTGGAAAAG |
| | | | 45 | RV | TTCCAAGCTTCATCACACCAGGCAACACCGTCTTC |
| 5 | 96 | Brevundimonas diminuta | 46 | FW | AGCTTCCATGGATCATCCGCACGGCTATCTGACCGCC |
| | | | 47 | RV | ATCAAAGCTTCTGACGCGCCAGCGAGGCGGGGCAGG |
| 6 | 97 | Sphingomonas trueperi | 48 | FW | TCAGGCCATGGACACGGCGCCGTACCTCGCCG |
| | | | 49 | RV | AAGAAAGCTTGGGGCGGACGCGAAGGGCCTCCGC |
| 7 | 98 | Zymomonas mobilis | 50 | FW | TCTGGCCATGGATCTTTCTCAAAGCGTTTCAGCT |
| | | | 51 | RV | TATGAAGCTTGCGAGCGCTTTGTTCAATCTTGC |
| 8 | 99 | Desulfovibrio magneticus | 52 | FW | TATGGATCCGCAGCTGGCCTGGTCCGAAACGCAGTTC |
| | | | 53 | RV | CCTCAAGCTTCTGGGCCAGACCCAGGGCGCTGCGGACTT |
| 9 | 100 | Gluconobacter oxydans | 54 | FW | TAACGCCCATGGATACGTCCGCTACCGCCCAAGGCGGC |
| | | | 55 | RV | AACAAAGCTTTTCGCTGAGCAGGGAATGGATCGCGGC |
| 10 | 101 | Flavobacterium johnsoniae | 56 | FW | CTTAGACCATGGATGCATTGCTGGATGGATATTTG |
| | | | 57 | RV | CCATAAGCTTAGCTCCTGCCATTTCTTTTTTAACCT |
| 11 | 102 | Methylobacterium ectorquens | 58 | FW | TACGTTCCATGGATTCCGCCCCCTCGCTCGAGAAGGA |
| | | | 59 | RV | ATCCAAGCTTGAGCAGGGGCTGGCGCGCGGCCGCAT |
| 12 | 103 | Acetobacter pasteurianus | 60 | FW | CTTAGACCATGGATAGCAGCCTTTTTGGATATACC |
| | | | 61 | RV | AAGTAAGCTTAAAAGCGTCTTGCGCAGCCTGCTGTTC |
| 104 | 122 | Serratia plymutica | 124 | FW | GCTGCCACCATGGATACCGGGCCGACGGTAACGGAC |
| | | | 125 | RV | GATCAAGCTTGTGCATACTCAAAGAGAGTGCAAC |
| 105 | 123 | Rahnella aquatilis | 126 | FW | GTTCAGGCCATGGAAGAAGCCAAACCCTTTATCACC |
| | | | 127 | RV | ACGAAAGCTTTTTCAGGCCAGATTGGGCGCGAAG |

### Preparation of transformants

For SEQ ID NOs: 3 to 7, 9 to 12, 104, and 105, the resultant PCR product was digested with restriction enzymes (*Nco*I and *Hind*III), and ligated into a pET22b(+) vector (Novagen) treated with *Nco*I/*Hind*III restriction enzymes to obtain a vector for expression of the encoded phosphorylating enzyme. For SEQ ID NO: 8, the resultant PCR product was digested with restriction enzymes (*Bam*HI and *Hind*III), and ligated into a pET22b(+) vector (Novagen) treated with *Bam*HI/*Hind*III restriction enzymes to obtain a vector for expression of the encoded phosphorylating enzyme. These vectors further carry, besides a region encoding the phosphorylating enzyme, a T7 promoter, a *pelB* signal for transport of the expressed protein into the periplasm, a His-tag gene for protein purification, and an ampicillin resistance gene for selection with a drug. The vector obtained by the above-described procedures was transformed into cells of an *Escherichia coli* (*E. coli*) BL21(DE3) strain (Novagen) by heat shock method.

### Preparation of phosphorylating enzymes

Culturing of transformant cells of an *E. coli* BL21(DE3) strain carrying the vector for expression of the encoded phosphorylating enzyme was performed by incubating them at a culture temperature of 27°C in an LB medium (manufactured by Nacalai Tesque, Inc. and having a composition of 1% by weight of tryptone, 0.5% by weight of dry yeast extract, 0.5% by weight of sodium chloride) containing 0.2% by weight of glucose, 0.5% by weight of casamino acid, and 0.1 mg/mL of ampicillin. When the optical density of the culture at 600 nm reached 0.5, isopropyl-β-thiogalactopyranoside was added to a concentration of 0.1 mM and additional culturing was performed at a culture temperature of 27°C for a period of 16 to 24 hours.

After the culture was completed, 15 g of wet cells was suspended in 20 mM Tris-HCl buffer (pH 8.0) containing 150 mM sodium chloride, and then subjected to an ultrasonic homogenizer (TOMY UD201) to obtain a cell extract solution. The cell extract solution was subjected to centrifugation at 20,000 × g for 20 minutes to collect the supernatant fraction. To the supernatant, ammonium sulfate was added to a saturation of 60%, followed by centrifugation at 20,000 × g for 20 minutes to collect a precipitate. The precipitate after the ammonium sulfate fractionation was suspended in 20 mM Tris-HCl buffer (pH 7.5), and then dialyzed against 20 mM Tris-HCl buffer (pH 7.5) containing 50 mM sodium chloride. After the dialysis was completed, affinity purification was carried out using a His-Trap HP column. Fractions containing the phosphorylating enzyme were collected, and then subjected to dialysis against 20 mM Tris-HCl buffer (pH 7.5) to obtain the phosphorylating enzyme. Conditions for the affinity purification were as follows.

### Purification conditions for phosphorylating enzymes

Column: His-Trap HP (manufactured by GE healthcare)
Equilibration buffer: 20 mM Tris-HCl (pH 7.5), 50 mM sodium chloride
Elution buffer: 20 mM Tris-HCl (pH 7.5), 50 mM sodium chloride, 1 M imidazole
(elution in a gradient of the elution buffer from 0 to 50%)
Flow rate: 5.0 mL/min
Purification equipment: AKTA prime plus (GE healthcare)

The amino acid sequence and position of the active center of the phosphorylating enzymes obtained are indicated in Table 2.

**[Table 2]**

| Amino acid sequence of the phosphorylating enzyme | Amino acid sequence of the active center | Position of the active center |
|---|---|---|
| SEQ ID NO: 3 | Gly Ala Tyr Pro Ser Gly His Thr | Positions 130 to 137 in SEQ ID NO: 3 |
| SEQ ID NO: 4 | Gly Ser Tyr Pro Ser Gly His Thr | Positions 131 to 138 in SEQ ID NO: 4 |
| SEQ ID NO: 5 | Gly Ser Tyr Pro Ser Gly Ilis Ser | Positions 131 to 138 in SEQ ID NO: 5 |
| SEQ ID NO: 6 | Gly Asp Tyr Pro Ser Gly His Thr | Positions 125 to 132 in SEQ ID NO: 6 |
| SEQ ID NO: 7 | Gly Ser Tyr Pro Ser Gly His Thr | Positions 142 to 149 in SEQ ID NO: 7 |
| SEQ ID NO: 8 | Ala Ser Tyr Pro Ser Gly His Ser | Positions 134 to 141 in SEQ ID NO: 8 |
| SEQ ID NO: 9 | Ala Ser Tyr Pro Ser Gly His Thr | Positions 134 to 141 in SEQ ID NO: 9 |
| SEQ ID NO: 10 | Gly Ser Phe Pro Ser Gly His Ala | Positions 133 to 140 in SEQ ID NO: 10 |
| SEQ ID NO: 11 | Phe Ser Tyr Pro Ser Gly His Ala | Positions 144 to 151 in SEQ ID NO: 11 |
| SEQ ID NO: 12 | Tyr Ala Tyr Pro Ser Gly His Thr | Positions 135 to 142 in SEQ ID NO: 12 |
| SEQ ID NO: 104 | Gly Ser Trp Pro Ser Gl y His Ser | Positions 143 to 150 in SEQ ID NO: 104 |
| SEQ ID NO: 105 | Gly Ser Trp Pro Ser Gly His Ser | Positions 123 to 130 in SEQ ID NO: 105 |

### [Experimental Example 1]

The microorganism-derived phosphorylating enzymes obtained as described above (which are represented by SEQ ID NOs: 3 to 12, 104, and 105) were used to phosphorylate glycerol. Conditions for and results of the glycerol phosphorylating reaction are indicated below.

### Phosphorylation of glycerol

To ultrapure water were added glycerol (4 M or 0.5 M), a polyphosphoric acid as a phosphate group donor (4% by weight; manufactured by Nacalai Tesque, Inc. under a trade name of polyphosphoric acid), and Triton X-100 (0.1% by weight), to prepare a reaction mixture solution. When glycerol was used at a concentration of 4 M, the pH of the solution was adjusted to pH 4.0 or 5.0 with KOH; and when glycerol was used at a concentration of 0.5 M, the pH of the solution was adjusted to pH 4.5 with KOH. Further, the phosphorylating enzyme including one of the amino acid sequences set forth in SEQ ID NOs: 3 to 12, 104, and 105 was added to the reaction mixture solution to make a final concentration of 20 µg/mL. The reaction solution was subjected to reaction in a temperature-controlled air incubator at 37°C overnight. After that, a 0.1-mL sample of the reaction solution was taken and diluted 50 to 500 times in 50 mM PIPES buffer (pH 7.0). The concentration of glycerol-3-phosphate in the sample was detected by an enzymatic method using glycerol-3-phosphate dehydrogenase under the conditions described below.

### Detection method for glycerol-3-phosphate

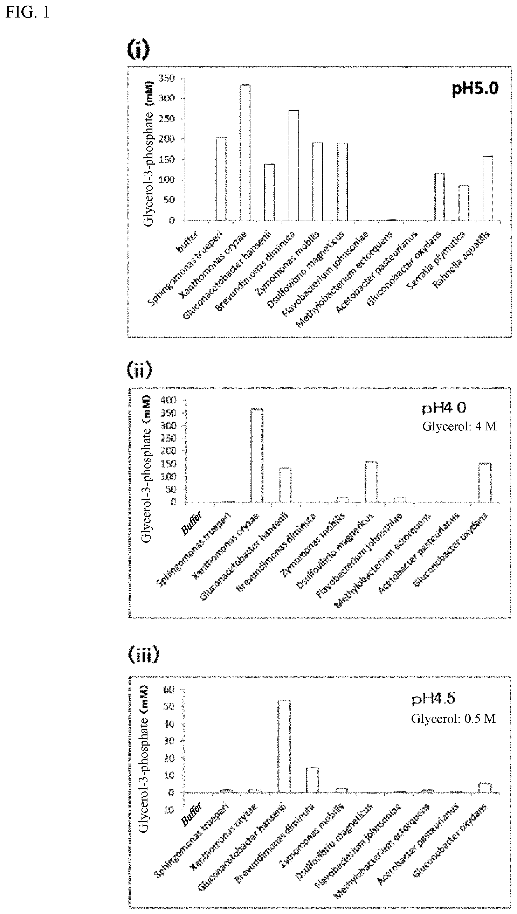

Glycerol-3-phosphate dehydrogenase and potassium ferricyanide were mixed with 50 mM PIPES buffer (pH 7.0) to make final concentrations of 10 U/mL and 2 mM, respectively. To 0.9 mL of this measurement solution was added a 0.1-mL sample of the reaction solution after the phosphorylation reaction, to prepare a reaction mixture, which then was subjected to measurement of the rate of decrease in the absorbance at 420 nm, for 3 minutes at 37°C. A standard curve was prepared from the rates of decrease in the absorbance obtained by using known concentrations of glycerol-3-phosphate, and used to calculate the concentration of glycerol-3-phosphate produced. The results are shown in Fig. 1. In Fig. 1, graph (i) shows the results when the reaction was carried out using 4 M glycerol and at a pH of 5.0, graph (ii) shows the results when using 4 M glycerol and at a pH of 4.0, and graph (iii) shows the results when using 0.5 M glycerol and at a pH of 4.5. The absorbance of 50 mM PIPES buffer (pH 7.0) was measured as a control (indicated as "Buffer" in the figure).

### Results

From Fig. 1(i), it was found that when the reaction was carried out using 4 M glycerol and at a pH of 5.0, the phosphorylation of glycerol using the phosphorylating enzymes including the respective amino acid sequences set forth in SEQ ID NOs: 3 to 9, 104, and 105 resulted in a significantly high phosphorylation efficiency, relative to the other phosphorylating enzymes. It was also found that among these phosphorylating enzymes, the phosphorylating enzyme including the amino acid sequence set forth in SEQ ID NO: 4 produced a particularly high amount of glycerol-3-phosphate and has an enhanced phosphorylation action on glycerol under these conditions. From these results, it turned out that a class A acid phosphatase having as the active center an amino acid sequence represented by the sequence (1) as described above can be utilized as a glycerol-phosphorylating enzyme.

From Fig. 1(ii), it was found that when the reaction was carried out using 4 M glycerol and at a pH of 4.0, the phosphorylation of glycerol using the phosphorylating enzyme including the amino acid sequence set forth in SEQ ID NO: 3, 4, 8, or 9, resulted in a significantly high phosphorylation efficiency, relative to the other phosphorylating enzymes. It was also found that among these phosphorylating enzymes, the phosphorylating enzyme including the amino acid sequence set forth in SEQ ID NO: 4 produced a particularly high amount of glycerol-3-phosphate and has an enhanced phosphorylation action on glycerol under these conditions.

From Fig. 1(iii), it was found that when the reaction was carried out using 0.5 M glycerol and at a pH of 4.5, the phosphorylation of glycerol using the phosphorylating enzyme including the amino acid sequence set forth in SEQ ID NO: 3, 5, or 9, resulted in a high phosphorylation efficiency, relative to the other phosphorylating enzymes. It was found that in particular, the phosphorylation of glycerol using the phosphorylating enzyme including the amino acid sequence set forth in SEQ ID NO: 3 resulted in a significantly high phosphorylation efficiency.

### [Experimental Example 2]

### Examination of phosphate group donors

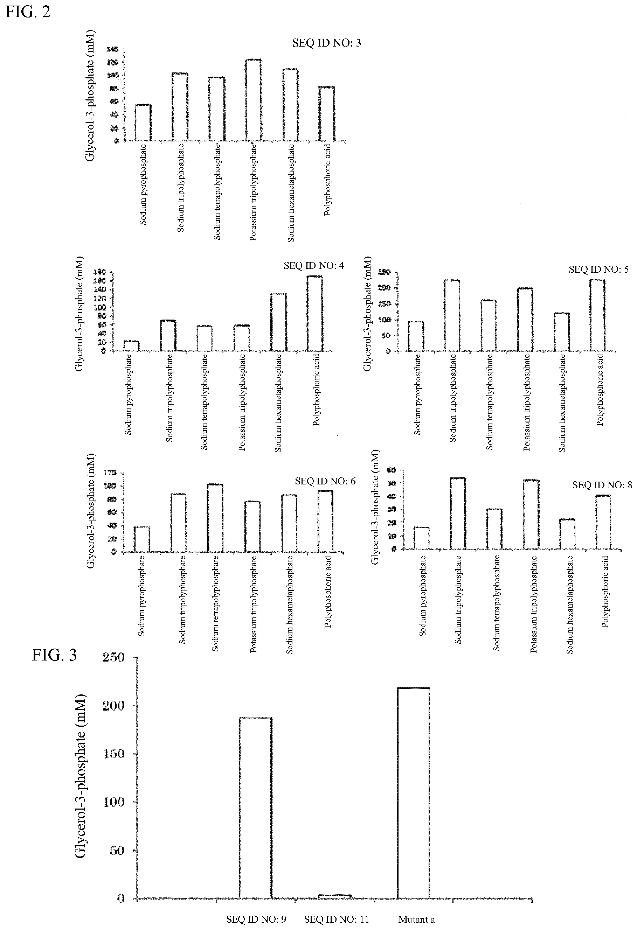

The efficiency of the phosphorylation of glycerol by phosphorylating enzymes (SEQ ID NOs: 3 to 6 and 8) was examined by changing the type of phosphate group donor. As a phosphate group donor, use was made of sodium pyrophosphate, sodium tripolyphosphate, sodium tetrapolyphosphate, potassium tripolyphosphate, sodium hexametaphosphate, or polyphosphoric acid. The polyphosphoric acid used was a polyphosphoric acid manufactured by Nacalai Tesque, Inc. The concentration of a phosphate group donor was set to be 0.2 M, except that the polyphosphoric acid was set to be at 7.5% by weight. A phosphorylating enzyme (phosphorylating enzyme set forth in SEQ ID NO: 3 to 6 or 8; 0.02 mg/mL each), a substrate (glycerol; at a concentration of 1 M), and a phosphate group donor were added to ultrapure water to prepare a reaction solution. The pH of the reaction solution was adjusted to a pH of 5.0 with potassium hydroxide or acetic acid. The reaction was carried out at 37°C for 24 hours. After the reaction was completed, the amount of glycerol-3-phosphate produced was measured by an enzymatic method using glycerol-3-phosphate dehydrogenase as in Experimental Example 1. The results are shown in Fig. 2.

As shown in Fig. 2, it was found that the phosphorylated glycerol was obtained when any of the phosphate group donors was used. Particularly, the phosphorylating enzyme set forth in SEQ ID NO: 3 allowed the production of the phosphorylated product with higher efficiency by using sodium tripolyphosphate, sodium tetrapolyphosphate, potassium tripolyphosphate, sodium hexametaphosphate, or polyphosphoric acid as the phosphate group donor.

The phosphorylating enzyme set forth in SEQ ID NO: 4 allowed the production of the phosphorylated product with higher efficiency by using sodium hexametaphosphate or polyphosphoric acid as the phosphate group donor.

The phosphorylating enzyme set forth in SEQ ID NO: 5 allowed the production of the phosphorylated product with higher efficiency by using sodium tripolyphosphate, potassium tripolyphosphate, or polyphosphoric acid as the phosphate group donor.

The phosphorylating enzyme set forth in SEQ ID NO: 6 allowed the production of the phosphorylated product with higher efficiency by using sodium tripolyphosphate, sodium tetrapolyphosphate, potassium tripolyphosphate, sodium hexametaphosphate, or polyphosphoric acid as the phosphate group donor.

The phosphorylating enzyme set forth in SEQ ID NO: 8 allowed the production of the phosphorylated product with higher efficiency by using sodium tripolyphosphate, potassium tripolyphosphate, or polyphosphoric acid as the phosphate group donor.

### [Experimental Example 3]

### Phosphorylation of glycerol by phosphorylating enzyme mutants

### (3-1) A mutant having mutations introduced in the vicinity of the active center - 1

### (Introduction of mutations and generation of transformants)

Mutations were introduced into a phosphorylating enzyme including the amino acid sequence set forth in SEQ ID NO: 11 that did not have any detectable activity to phosphorylate glycerol, to generate an enzyme mutant, Mutant a. The generation procedure is indicated below.

To obtain Mutant a, PCR reactions were performed using as a template a base sequence encoding the amino acid sequence set forth in SEQ ID NO: 11, and combinations of the primers indicated in Table 3. The resulting PCR product was digested with the restriction enzymes described corresponding to the column of Mutant in Table 3. Ligation was performed between a pET22b(+) vector (Novagen) treated with the same restriction enzymes as those with which the PCR product had been treated and the restriction enzyme-treated PCR product, to obtain a vector for expression of the encoded glycerol-phosphorylating enzyme. The vector further carry, besides the base sequence encoding Mutant a, a T7 promoter, a *pel*B signal for transport of the expressed protein into the periplasm, a His-tag gene for protein purification, and an ampicillin resistance gene for selection with a drug. The vector obtained by the above-described procedures was transformed into cells of an *Escherichia coli* (*E. coli*) BL21(DE3) strain (Novagen) by heat shock method.

The primer sets and restriction enzyme sites used for the generation of Mutant a, and the base sequences of the primers are indicated in Tables 3 and 4, respectively. In Table 3, "F144G/A151T" in Mutant a, in the column of Mutant, indicates that the phenylalanine residue at position 144 in SEQ ID NO: 11 was replaced with a glycine residue and the alanine residue at position 151 was replaced with a threonine residue.

**[Table 3]**

| SEQ ID NO: of (the whole amino acid sequence of) mutant | Mutant | Primer set | Restriction enzyme site |
|---|---|---|---|
| 13 | a (F144G/A151T) | Primers (1) and (4); | *Nco*I and *Bam*HI; |
| | | Primers (3) and (2) | *BamH*I and *Hind*III |

**[Table 4]**

| Primer SEQ ID NO: | Primer No. | Sequence (5' to 3') |
|---|---|---|
| 62 | (1) | TACGTTCCATGGATTCCGCCCCCTCGCTCGAGAAGGA |
| 63 | (2) | ATCCAAGCTTGAGCAGGGGCTGGCGCGCGGCCGCAT |
| 64 | (3) | AGCGGATCCTATCCCTCCGGTCATACTAGCCAGGGG |
| 65 | (4) | ATAGGATCCGCTGTCCGCGAGTTCGGCCGTGCG |

| | | |
|---|---|---|
| * In Table 4, the underlined sequences indicate a restriction enzyme cleavage site. | | |

### (Culturing of transformants)

Culturing of transformant cells of an *E. coli* BL21(DE3) strain carrying the vector for expression of the encoded glycerol-phosphorylating enzyme was performed by incubating them at a culture temperature of 27°C in an LB medium (manufactured by Nacalai Tesque, Inc. and having a composition of 1% by weight of tryptone, 0.5% by weight of dry yeast extract, 0.5% by weight of sodium chloride) containing 0.2% by weight of glucose, 0.5% by weight of casamino acid, and 0.1 mg/mL of ampicillin. When the optical density of the culture at 600 nm reached 0.5, isopropyl-β-thiogalactopyranoside was added to be 0.1 mM and additional culturing was performed at a culture temperature of 27°C for a period of 16 to 24 hours.

### (Obtaining of a glycerol-phosphorylating enzyme mutant)

After the culture was completed, 15 g of wet cells was suspended in 20 mM Tris-HCl buffer (pH 8.0) containing 150 mM sodium chloride, and then subjected to an ultrasonic homogenizer (TOMY UD201) to obtain a cell extract solution. The cell extract solution was subjected to centrifugation at 20,000 × g for 20 minutes to collect the supernatant fraction. To the supernatant, ammonium sulfate was added to a saturation of 60%, followed by centrifugation at 20,000 × g for 20 minutes to collect a precipitate. The precipitate after the ammonium sulfate fractionation was suspended in 20 mM Tris-HCl buffer (pH 7.5), and then dialyzed against 20 mM Tris-HCl buffer (pH 7.5) containing 50 mM sodium chloride. After the dialysis was completed, affinity purification was carried out using a His-Trap HP column. Fractions containing the glycerol-phosphorylating enzyme were collected, and then subjected to dialysis against 20 mM Tris-HCl buffer (pH 7.5) to obtain the glycerol-phosphorylating enzyme mutant. The amino acid sequences of the active center of the phosphorylating enzyme including the amino acid sequence set forth in SEQ ID NO: 11 and the enzyme mutant, Mutant a, are indicated in Table 5.

**[Table 5]**

| | Amino acid sequence of the active center |
|---|---|
| SEQ ID NO: 11 | Phe Ser Tyr Pro Ser Gly His Ala |
| Mutant a (F144G/A151T) | Gly Ser Tyr Pro Ser Gly His Thr |

### Purification conditions

Conditions for the affinity purification were as follows.
Column: His-Trap HP (manufactured by GE healthcare)
Equilibration buffer: 20 mM Tris-HCl (pH 7.5), 50 mM sodium chloride
Elution buffer: 20 mM Tris-HCl (pH 7.5), 50 mM sodium chloride, 1 M imidazole
(elution in a gradient of the elution buffer from 0 to 50%)
Flow rate: 5.0 mL/min
Purification equipment: AKTA prime plus (GE healthcare)

### (Phosphorylation of glycerol using a glycerol-phosphorylating enzyme mutant, Mutant a)

The glycerol phosphorylating reaction was performed using Mutant a, which is the glycerol-phosphorylating enzyme obtained as described above. To ultrapure water were added glycerol (4 M) and a polyphosphoric acid as a phosphate group donor (7.5% by weight; manufactured by Nacalai Tesque, Inc. under a trade name of polyphosphoric acid), to prepare a reaction mixture solution. The pH of the reaction solution was adjusted to a pH of 5.0 with KOH. The phosphorylating enzyme, Mutant a, was added to the reaction mixture solution to make a final concentration of 50 µg/mL. The reaction solution was subjected to reaction in a temperature-controlled air incubator at 37°C for 24 hours. The concentration of glycerol-3-phosphate in the sample was detected by an enzymatic method using glycerol-3-phosphate dehydrogenase. The phosphorylation of glycerol and detection of glycerol-3-phosphate were carried out as in Experimental Example 1. The results are shown in Fig. 3.

As shown in Fig. 3, it was observed that Mutant a having F144G/A151T mutations introduced in the phosphorylating enzyme set forth in SEQ ID NO: 11 that had an extremely low level of phosphorylating enzyme activity resulted in a significant improvement in the enzyme activity.

### (3-2) Mutants having mutations introduced in the vicinity of the active center - 2

Mutations were introduced into a phosphorylating enzyme including the amino acid sequence set forth in SEQ ID NO: 11 that did not have any detectable activity to phosphorylate glycerol, to generate enzyme mutants, Mutants b to g. Generation procedures for Mutants b to g were as described above in (3-1), except for using as a template a base sequence (set forth in SEQ ID NO: 128) in which a (wild-type) base sequence encoding the amino acid sequence set forth in SEQ ID NO: 11 was codon optimized for expression in *E. coli* and using the primers indicated in Tables 6 and 7.

**[Table 6]**

| SEQ ID NO: of (the whole amino acid sequence of) mutant | Mutant | Primer set | Restriction enzyme site |
|---|---|---|---|
| 116 | b (F144A) | Primers (5) and (7); | *Nco*I and *Nhe*I; |
| | | Primers (8) and (6) | *Nhe*I and *Hind*III |
| 117 | c (F144G) | Primers (5) and (9); | *Nco*I and *BamHI;* |
| | | Primers (10) and (6) | *BamHI* and *Hind*III |
| 118 | d (A151D) | Primers (5) and (11); | *Nco*I and *Nde*I*;* |
| | | Primers (12) and (6) | *Nde*I and *Hind*III |
| 119 | e (A151T) | Primers (5) and (13); | *Nco*I and *Nde*I*;* |
| | | Primers (11) and (6) | *Nde*I and *Hind*III |
| 120 | f (A151S) | Primers (5) and (14); | *Nco*I and *Nde*I*;* |
| | | Primers (15) and (6) | *Nde*I and *Hind*III |
| 121 | g (F144G/A151S) | Primers (5) and (9); | *Nco*I and *BamHI;* |
| | | Primers (15) and (6) | *BamHI* and *Hind*III |

**[Table 7]**

| Primer SEQ ID NO: | Primer No. | Sequence (5' to 3') |
|---|---|---|
| 129 | (5) | GGCGATGGCCATGGACAGTGCCCCGAGTCTGGAA |
| 130 | (6) | TAAAGAAGCTTCAGCAGCGGTTGACGGGCAGC |
| 131 | (7) | ACTCGGATAGCTAGCGCTGTCCGCCAGTTCAGC |
| 132 | (8) | GCGGACAGCGCTAGCTATCCGAGTGGTCATGCGTCCCAG |
| 133 | (9) | AATAGGATCCGCTGTCCGCCAGTTCAGCGGT |
| 134 | (10) | GACAGAGGATCCTATCCGAGTGGTCATGCGTCCCAGGGT |
| 135 | (11) | GATCAGACCATATGCCCAACCCTGGGAGTCATGACCACT |
| 136 | (12) | CAGGGTTGGGCATATGGTCTGATCATGGCAAAC |
| 137 | (13) | GATCAGACCATATGCCCAACCCTGGGACGTATGACCACT |
| 138 | (14) | GATCAGACCATATGCCCAACCCTGGGAGCTATGACCACT |
| 139 | (15) | GACAGAGGATCCTATCCGAGTGGTCATTCGTCCCAGGGT |

The amino acid sequences of the active center of the phosphorylating enzyme including the amino acid sequence set forth in SEQ ID NO: 11 and the enzyme mutants, Mutants b to g, are indicated in Table 8.

**[Table 8]**

| | Amino acid sequence of the active center |
|---|---|
| SEQ ID NO: 11 | Phe Ser Tyr Pro Ser Gly His Ala |
| Mutant b (F144A) | Ala Ser Tyr Pro Ser Gly His Ala |
| Mutant c (F144G) | Gly Ser Tyr Pro Ser Gly His Ala |
| Mutant d (A151D) | Phe Ser Tyr Pro Ser Gly His Asp |
| Mutant e (A151T) | Phe Ser Tyr Pro Ser Gly His Thr |
| Mutant f (A151S) | Phe Ser Tyr Pro Ser Gly His Ser |
| Mutant g (F144G/A151 S) | Gly Ser Tyr Pro Ser Gly His Ser |

Culturing of transformants, obtaining of glycerol-phosphorylating enzyme mutants, and phosphorylation of glycerol were performed in the same conditions as those described above in (3-1).

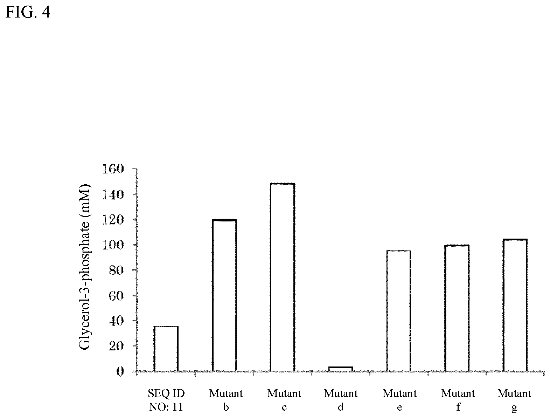

The results obtained are shown in Fig. 4. From these results, it was observed that Mutant d had little detectable activity to phosphorylate glycerol, while Mutants b, c, and e to g exhibited a superior activity to phosphorylate glycerol. Also from these results, it turned out that a class A acid phosphatase having as the active center an amino acid sequence represented by the sequence (1) as described above can be utilized as a glycerol-phosphorylating enzyme.

### (3-2) Mutants having mutations introduced in an N-terminal portion

Mutants A to L were generated in which an N-terminal portion of the amino acid sequence set forth in SEQ ID NO: 12 was replaced with a partial sequence comprised of the amino acid sequence set forth in SEQ ID NO: 9, by means of using as a template a DNA sequence encoding the amino acid sequence set forth in SEQ ID NO: 12. Generation procedures were as in Mutants a to d described above in (3-1), and PCR products were obtained using the primer sets and template indicated below in Table 9. The PCR product was treated with restriction enzymes corresponding to the restriction enzyme sites described for the respective mutants in Table 9, and inserted into a vector. The structures of Mutants A to L are shown in Fig. 5(i) and (ii).

Combinations of the primer sets, restriction enzyme sites, and templates used for the generation of Mutants A to L having mutations introduced in an N-terminal portion are shown in Table 9, and the base sequences of the primers are shown in Table 10. In Table 9, the description "A143-E244" in Mutant A, for example, in the column of Mutant, indicates that the amino acid sequence corresponding to positions 144 to 242 in SEQ ID NO: 12 was replaced with the corresponding amino acid sequence of from the alanine at position 143 to the glutamic acid at position 244 in SEQ ID NO: 9.

**[Table 9]**

| SEQ ID NO: of mutant | Mutant | Primer set | Restriction enzyme site | Template used |
|---|---|---|---|---|
| 14 | A (A143-E244) | Primers (1) and (6); | *Nco*I and *Pst*I; | a plasmid encoding SEQ ID NO: 12; |
| | | Primers (5) and (4) | *Pst*I and *Hind*III | a plasmid encoding SEQ ID NO: 9 |
| 15 | B (M1-A143) | Primers (3) and (8); | *Nco*I and *Pst*I; | a plasmid encoding SEQ ID NO: 9; |
| | | Primers (7) and (2) | *Pst*I and *Hind*III | a plasmid encoding SEQ ID NO: 12 |
| 16 | C (M1-P122) | Primers (3) and (10); | *Nco*I and *Nhe*I*;* | a plasmid encoding SEQ ID NO: 9; |
| | | Primers (9) and (2) | *Nhe*I and *Hind*III | a plasmid encoding SEQ ID NO: 12 |
| 17 | D (M1-R70) | Primers (3) and (12); | *Nco*I and *Sal*I*;* | a plasmid encoding SEQ ID NO: 9; |
| | | Primers (11) and (2) | *Xho*I and *Hind*III | a plasmid encoding SEQ ID NO: 12 |
| 18 | E (S126-A143) | Primers (1) and (14); | *Nco*I and *Spe*I; | a plasmid encoding SEQ ID NO: 9; |
| | | Primers (13) and (2) | *Spe*I and *Hind*III | a plasmid encoding SEQ ID NO: 12 |
| 19 | F (P18-R70) | Primers (1) and (16); | *Nco*I and *Spe*I; | a plasmid encoding SEQ ID NO: 9; |
| | | Primers (15) and (2) | *Spe*I and *Hind*III | a plasmid encoding Mutant D |
| 20 | G (A29-R70) | Primers (1) and (18); | *Nco*I and *Pst*I; | a plasmid encoding SEQ ID NO: 9; |
| | | Primers (17) and (2) | *Pst*I and *Hind*III | a plasmid encoding Mutant D |
| 21 | H (R53-R70) | Primers (1) and (20); | *Nco*I and *Nhe*I*;* | a plasmid encoding SEQ ID NO: 9; |
| | | Primers (19) and (2) | *Nhe*I and *Hind*III | a plasmid encoding Mutant D |
| 22 | I (M1-P52) | Primers (3) and (22); | *Nco*I and *Nhe*I*;* | a plasmid encoding SEQ ID NO: 9; |
| | | Primers (21) and (2) | *Nhe*I and *Hind*III | a plasmid encoding SEQ ID NO: 12 |
| 23 | J (M1-A25) | Primers (3) and (24); | *Nco*I and *Nhe*I*;* | a plasmid encoding SEQ ID NO: 9; |
| | | Primers (23) and (2) | *Nhe*I and *Hind*III | a plasmid encoding SEQ ID NO: 12 |
| 24 | K (M1-A17) | Primers (3) and (26); | *Nco*I and *Pst*I; | a plasmid encoding SEQ ID NO: 9; |
| | | Primers (25) and (2) | *Pst*I and *Hind*III | a plasmid encoding SEQ ID NO: 12 |
| 25 | L (M1-Q35) | Primers (3) and (28); | *Nco*I and *Pst*I; | a plasmid encoding SEQ ID NO: 9; |
| | | Primers (27) and (2) | *Pst*I and *Hind*III | a plasmid encoding SEQ ID NO: 12 |

**[Table 10]**

| Primer SEQ ID NO: | Primer No. | Sequence (5' to 3') |
|---|---|---|
| 66 | 1 | CTTAGACCATGGATAGCAGCCTTTTTGGATATACC |
| 67 | 2 | AAGTAAGCTTAAAAGCGTCTTGCGCAGCCTGCTGTTC |
| 68 | 3 | TAACGCCCATGGATACGTCCGCTACCGCCCAAGGCGGC |
| 67 | 4 | AACAAAGCTTTTCGCTGAGCAGGGAATGGATCGCGGC |
| 70 | 5 | GACTGCAGGCTACGGGATGGCGCTGCTCCTG |
| 71 | 6 | ATCCTGCAGTTGTATGGCCAGAGGGATA |
| 72 | 7 | CAACTGCAGGATGGCTCACGGCATCCATT |
| 73 | 8 | TCCTGCAGTCGTATGACCCGACGGATAGGACGC |
| 74 | 9 | AATAAACACATATGCACAGCACATTCAGATGGGCTT |
| 75 | 10 | GAGAGCTGCATATGGGCAGGTTCGTACCCACGAACG |
| 76 | 11 | TTTAATGTCGACCCAGAAAAACTTCCCGCAATG |
| 77 | 12 | GCCAGCTCGAGATTGAACCCGCCGCACAGGAGAA |
| 78 | 13 | ACACTAGTCCGGAAGGTCTTGGTCTCAACGCTTCCTATCCCTCT |
| 79 | 14 | GGACTAGTGCAGATGTCTTTATTTGTGCCGAC |
| 80 | 15 | CGACTAGTTCTGGCTCCGCCGCCGGCG |
| 81 | 16 | AAACTAGTCGTTCATCAGGTAGGGTAAACTG |
| 82 | 17 | GGAACTGCAGCGCAGAGTGATGACGACCGGGTG |
| 83 | 18 | AGTGCTGCAGATCCGGGCGCTGGTGGCGGGGGCAAAA |
| 84 | 19 | CGCTGGAAGCTAGCGCAGAGCGATGCGGATCTG |
| 85 | 20 | ATTCTGTAGCTAGCTTCCAGCGCGCCTTATCTTTTAA |
| 86 | 21 | CGCTGGAAGCTAGCCCAGAATGACGCCAACCTT |
| 87 | 22 | GCTCTGAGCTAGCTTCCAGCGCGGCGTGTCCTTCAG |
| 88 | 23 | GACTTCAGCTAGCACCGCCACCACAGGCAGAAGAACCC |
| 89 | 24 | CGGCGGAGCTAGCACGAGACGCTCGTCCGGAGCCGC |
| 90 | 25 | CAGTCTGCAGCACCTGATGGACGTGCATTTTTG |
| 91 | 26 | GGAGCTGCAGAGTCCGGCAGGATGCCGCCTTG |
| 92 | 27 | AACCTGCAGCACAACAGGCAGACTTGCGGGCT |
| 93 | 28 | CTGCTGCAGATCCGGGCGCCGGCGGCGGAGC |

### (Phosphorylation of glycerol using glycerol-phosphorylating enzyme mutants, Mutants A to L)

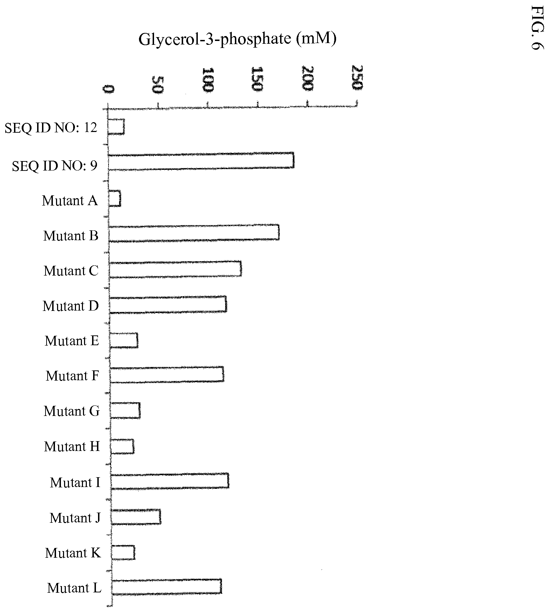

The glycerol phosphorylating reaction was performed using the resulting Mutants A to L. The phosphorylation of glycerol was carried out as described above for Mutants a to g, and the detection of glycerol-3-phosphate was carried out as in Experimental Example 1. The results are shown in Fig. 6.

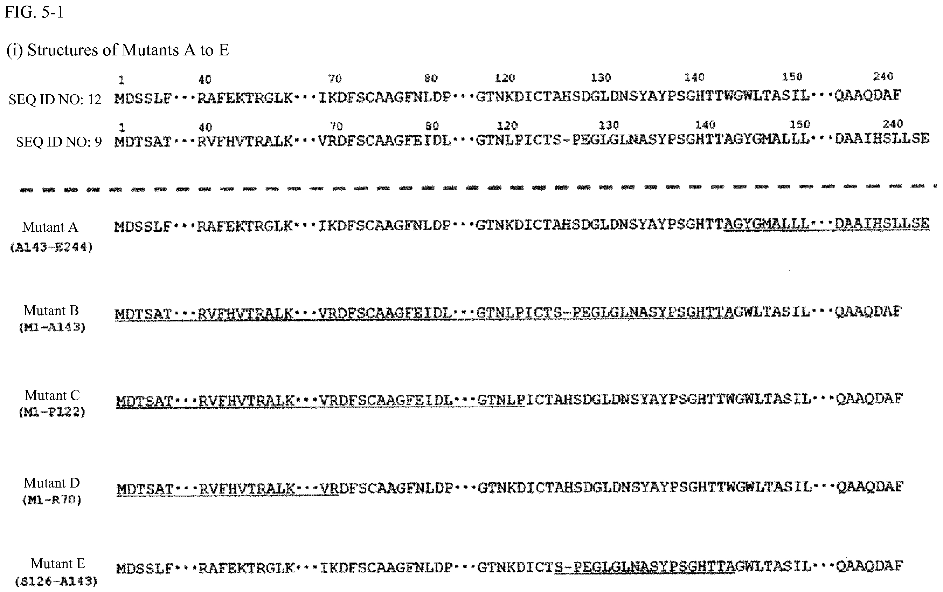

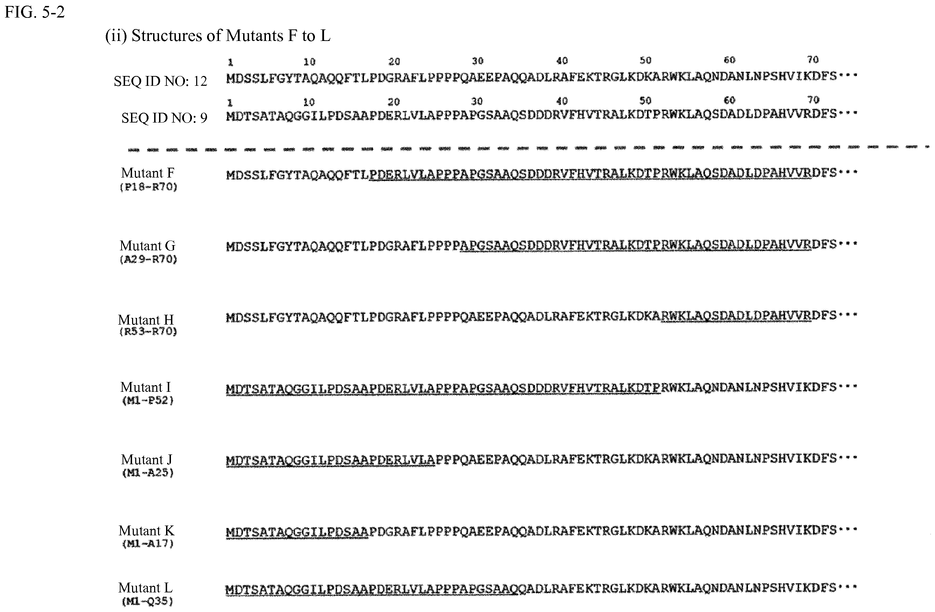

As shown in Fig. 5, the glycerol phosphorylating reactions in which Mutants A to L were used resulted in an increased amount of produced glycerol-3-phosphate for Mutants B, C, and D. Based on these results, it was revealed that the amino acid sequence of the amino acids at positions 1 to 70 from the N terminus of the amino acid sequence set forth in SEQ ID NO: 9 is responsible for the activity of the glycerol-phosphorylating enzyme. In addition, it was found that the glycerol phosphorylating reactions in which Mutants F to L were used resulted in an improvement in the activity of the phosphorylating enzyme for Mutants F, I, and L. The other Mutants G, H, J, and K, on the other hand, did not result in a great improvement in the activity of the phosphorylating enzyme, as compared to Mutants F and I. Based on these results, it was revealed that the amino acid sequence of from the proline residue at position 18 to the glutamine residue at position 35 from the N terminus of the amino acid sequence set forth in SEQ ID NO: 9 is responsible for the activity of the glycerol-phosphorylating enzyme.

Therefore, it was revealed that the amino acid sequence set forth in SEQ ID NO: 2, PDERLVLAPPPAPGSAAQ, is important for the expression of the enzyme activity of the above-described enzymes catalyzing the glycerol phosphorylating reaction.

### SEQUENCE LISTING FREE TEXT

SEQ ID NO: 1 represents the amino acid sequence represented by sequence (1).
SEQ ID NO: 2 represents the amino acid sequence of the N-terminal essential region of the phosphorylating enzyme.
SEQ ID NO: 13 represents the amino acid sequence of Mutant a (F144G/A151T).
SEQ ID NO: 14 represents the amino acid sequence of Mutant A (A143-E244).
SEQ ID NO: 15 represents the amino acid sequence of Mutant B (M1-A143).
SEQ ID NO: 16 represents the amino acid sequence of Mutant C (M1-P122).
SEQ ID NO: 17 represents the amino acid sequence of Mutant D (M1-R70).
SEQ ID NO: 18 represents the amino acid sequence of Mutant E (S126-A143).
SEQ ID NO: 19 represents the amino acid sequence of Mutant F (P18-R70).
SEQ ID NO: 20 represents the amino acid sequence of Mutant G (A29-R70).
SEQ ID NO: 21 represents the amino acid sequence of Mutant H (R53-R70).
SEQ ID NO: 22 represents the amino acid sequence of Mutant I (M1-P52).
SEQ ID NO: 23 represents the amino acid sequence of Mutant J (M1-A25).
SEQ ID NO: 24 represents the amino acid sequence of Mutant K (M1-A17).
SEQ ID NO: 25 represents the amino acid sequence of Mutant L (M1-Q35).
SEQ ID NO: 42 represents a forward primer for the phosphorylating enzyme set forth in amino acid sequence 3.
SEQ ID NO: 43 represents a reverse primer for the phosphorylating enzyme set forth in amino acid sequence 3.
SEQ ID NO: 44 represents a forward primer for the phosphorylating enzyme set forth in amino acid sequence 4.
SEQ ID NO: 45 represents a reverse primer for the phosphorylating enzyme set forth in amino acid sequence 4.
SEQ ID NO: 46 represents a forward primer for the phosphorylating enzyme set forth in amino acid sequence 5.
SEQ ID NO: 47 represents a reverse primer for the phosphorylating enzyme set forth in amino acid sequence 5.
SEQ ID NO: 48 represents a forward primer for the phosphorylating enzyme set forth in amino acid sequence 6.
SEQ ID NO: 49 represents a reverse primer for the phosphorylating enzyme set forth in amino acid sequence 6.
SEQ ID NO: 50 represents a forward primer for the phosphorylating enzyme set forth in amino acid sequence 7.
SEQ ID NO: 51 represents a reverse primer for the phosphorylating enzyme set forth in amino acid sequence 7.
SEQ ID NO: 52 represents a forward primer for the phosphorylating enzyme set forth in amino acid sequence 8.
SEQ ID NO: 53 represents a reverse primer for the phosphorylating enzyme set forth in amino acid sequence 8.
SEQ ID NO: 54 represents a forward primer for the phosphorylating enzyme set forth in amino acid sequence 9.
SEQ ID NO: 55 represents a reverse primer for the phosphorylating enzyme set forth in amino acid sequence 9.
SEQ ID NO: 56 represents a forward primer for the phosphorylating enzyme set forth in amino acid sequence 10.
SEQ ID NO: 57 represents a reverse primer for the phosphorylating enzyme set forth in amino acid sequence 10.
SEQ ID NO: 58 represents a forward primer for the phosphorylating enzyme set forth in amino acid sequence 11.
SEQ ID NO: 59 represents a reverse primer for the phosphorylating enzyme set forth in amino acid sequence 11.
SEQ ID NO: 60 represents a forward primer for the phosphorylating enzyme set forth in amino acid sequence 12.
SEQ ID NO: 61 represents a reverse primer for the phosphorylating enzyme set forth in amino acid sequence 12.
SEQ ID NO: 62 represents the base sequence of primer number (1).
SEQ ID NO: 63 represents the base sequence of primer number (2).
SEQ ID NO: 64 represents the base sequence of primer number (3).
SEQ ID NO: 65 represents the base sequence of primer number (4).
SEQ ID NO: 66 represents the base sequence of primer number 1.
SEQ ID NO: 67 represents the base sequence of primer number 2.
SEQ ID NO: 68 represents the base sequence of primer number 3.
SEQ ID NO: 69 represents the base sequence of primer number 4.
SEQ ID NO: 70 represents the base sequence of primer number 5.
SEQ ID NO: 71 represents the base sequence of primer number 6.
SEQ ID NO: 72 represents the base sequence of primer number 7.
SEQ ID NO: 73 represents the base sequence of primer number 8.
SEQ ID NO: 74 represents the base sequence of primer number 9.
SEQ ID NO: 75 represents the base sequence of primer number 10.
SEQ ID NO: 76 represents the base sequence of primer number 11.
SEQ ID NO: 77 represents the base sequence of primer number 12.
SEQ ID NO: 78 represents the base sequence of primer number 13.
SEQ ID NO: 79 represents the base sequence of primer number 14.
SEQ ID NO: 80 represents the base sequence of primer number 15.
SEQ ID NO: 81 represents the base sequence of primer number 16.
SEQ ID NO: 82 represents the base sequence of primer number 17.
SEQ ID NO: 83 represents the base sequence of primer number 18.
SEQ ID NO: 84 represents the base sequence of primer number 19.
SEQ ID NO: 85 represents the base sequence of primer number 20.
SEQ ID NO: 86 represents the base sequence of primer number 21.
SEQ ID NO: 87 represents the base sequence of primer number 22.
SEQ ID NO: 88 represents the base sequence of primer number 23.
SEQ ID NO: 89 represents the base sequence of primer number 24.
SEQ ID NO: 90 represents the base sequence of primer number 25.
SEQ ID NO: 91 represents the base sequence of primer number 26.
SEQ ID NO: 92 represents the base sequence of primer number 27.
SEQ ID NO: 93 represents the base sequence of primer number 28.
SEQ ID NO: 116 represents the amino acid sequence of Mutant b (F144A).
SEQ ID NO: 117 represents the amino acid sequence of Mutant c (F144G).
SEQ ID NO: 118 represents the amino acid sequence of Mutant d (A151D).
SEQ ID NO: 119 represents the amino acid sequence of Mutant e (A151T).
SEQ ID NO: 120 represents the amino acid sequence of Mutant f (A151S).
SEQ ID NO: 121 represents the amino acid sequence of Mutant g (F144G/A151S).
SEQ ID NO: 124 represents a forward primer for the phosphorylating enzyme set forth in amino acid sequence 104.
SEQ ID NO: 125 represents a reverse primer for the phosphorylating enzyme set forth in amino acid sequence 104.
SEQ ID NO: 126 represents a forward primer for the phosphorylating enzyme set forth in amino acid sequence 105.
SEQ ID NO: 127 represents a reverse primer for the phosphorylating enzyme set forth in amino acid sequence 105.
SEQ ID NO: 128 represents a base sequence in which a gene encoding the phosphorylating enzyme set forth in SEQ ID NO: 11 was codon optimized for expression in *Escherichia coli.*
SEQ ID NO: 129 represents the base sequence of primer number (5).
SEQ ID NO: 130 represents the base sequence of primer number (6).
SEQ ID NO: 131 represents the base sequence of primer number (7).
SEQ ID NO: 132 represents the base sequence of primer number (8).
SEQ ID NO: 133 represents the base sequence of primer number (9).
SEQ ID NO: 134 represents the base sequence of primer number (10).
SEQ ID NO: 135 represents the base sequence of primer number (11).
SEQ ID NO: 136 represents the base sequence of primer number (12).
SEQ ID NO: 137 represents the base sequence of primer number (13).
SEQ ID NO: 138 represents the base sequence of primer number (14).
SEQ ID NO: 139 represents the base sequence of primer number (15).

## Claims

1. A method for production of a phosphorylated glycerol (= glycerol phosphate), comprising a step of allowing a phosphorylating enzyme to act on glycerol in the presence of a phosphate group donor,
wherein the phosphorylating enzyme is a class A acid phosphatase and comprises:
- a polypeptide as defined in (i) or (ii) below:
(i) a polypeptide that has the amino acid sequence set forth in SEQ ID NO: 2 and has an activity to catalyze the glycerol phosphorylating reaction, or
(ii) a polypeptide that has an amino acid sequence having 1 to 3 amino acids substituted, deleted, inserted, or added in the amino acid sequence set forth in SEQ ID NO: 2 and has an activity to catalyze the glycerol phosphorylating reaction which is comparable or superior to that of the polypeptide as defined in (i); and, as an active center,
- a polypeptide having, on the C-terminal side of the amino acid sequence set forth in SEQ ID NO: 2, an amino acid sequence represented by the sequence (1):
-X¹-X²-X³-Pro-Ser-Gly-HiS-X⁴- (1)
wherein,
X¹ denotes glycine, alanine, or phenylalanine;
X² denotes any amino acid;
X³ denotes tyrosine or tryptophan; and
X⁴ denotes threonine, serine, or alanine;
with the proviso that when X¹ is phenylalanine, X⁴ is threonine or serine.

2. The method for production according to claim 1, wherein the amino acid residue at the first position in the amino acid sequence set forth in SEQ ID NO: 2 is located between positions 5 and 40 from the N-terminus of the phosphorylating enzyme.

3. The method for production according to claim 1 or 2, wherein X¹ and X⁴ in the sequence (1) are any of (A1) to (A8) below:
(A1) X¹ is glycine and X⁴ is threonine;
(A2) X¹ is glycine and X⁴ is serine;
(A3) X¹ is alanine and X⁴ is serine;
(A4) X¹ is alanine and X⁴ is threonine;
(A5) X¹ is alanine and X⁴ is alanine;
(A6) X¹ is glycine and X⁴ is alanine;
(A7) X¹ is phenylalanine and X⁴ is threonine; and
(A8) X¹ is phenylalanine and X⁴ is serine.

4. The method for production according to any of claims 1 to 3, wherein in the sequence (1), X² is serine, alanine, or aspartic acid.

5. The method for production according to any of claims 1 to 4, wherein X¹, X², X³, and X⁴ in the sequence (1) are any of (B1) to (B11) below:
(B1) X¹ is glycine, X² is serine, X³ is tyrosine, and X⁴ is threonine;
(B2) X¹ is glycine, X² is alanine, X³ is tyrosine, and X⁴ is threonine;
(B3) X¹ is glycine, X² is serine, X³ is tyrosine, and X⁴ is serine;
(B4) X¹ is glycine, X² is aspartic acid, X³ is tyrosine, and X⁴ is threonine;
(B5) X¹ is alanine, X² is serine, X³ is tyrosine, and X⁴ is serine;
(B6) X¹ is alanine, X² is serine, X³ is tyrosine, and X⁴ is threonine;
(B7) X¹ is alanine, X² is serine, X³ is tyrosine, and X⁴ is alanine;
(B8) X¹ is glycine, X² is serine, X³ is tyrosine, and X⁴ is alanine;
(B9) X¹ is phenylalanine, X² is serine, X³ is tyrosine, and X⁴ is threonine;
(B10) X¹ is phenylalanine, X² is serine, X³ is tyrosine, and X⁴ is serine; and
(B11) X¹ is glycine, X² is serine, X³ is tryptophan, and X⁴ is serine.

6. The method for production according to any of claims 1 to 5, wherein the histidine (His) residue at the 7-th position in the amino acid sequence represented by the sequence (1) is located between positions 120 and 160 from the N-terminus of the phosphorylating enzyme.

7. The method for production according to any of claims 1 to 6, wherein the phosphorylating enzyme further comprises at least one of the amino acid sequences as defined in (iii) to (vi) below:
(iii) a polypeptide that has, on the N-terminal side of the amino acid sequence set forth in SEQ ID NO: 2, the amino acid sequence set forth in SEQ ID NO: 40 and has an activity to catalyze the glycerol phosphorylating reaction;
(iv) a polypeptide that has, on the N-terminal side of the amino acid sequence set forth in SEQ ID NO: 2, an amino acid sequence having 1 to 3 amino acids substituted, deleted, inserted, or added in the amino acid sequence set forth in SEQ ID NO: 40 and has an activity to catalyze the glycerol phosphorylating reaction which is comparable or superior to that of the polypeptide as defined in (iii);
(v) a polypeptide that has, on the C-terminal side of the amino acid sequence set forth in SEQ ID NO: 2, the amino acid sequence set forth in SEQ ID NO: 41 and has an activity to catalyze the glycerol phosphorylating reaction; and
(vi) a polypeptide that has, on the C-terminal side of the amino acid sequence set forth in SEQ ID NO: 2, an amino acid sequence having 1 to 10 amino acids substituted, deleted, inserted, or added in the amino acid sequence set forth in SEQ ID NO: 41 and has an activity to catalyze the glycerol phosphorylating reaction which is comparable or superior to that of the polypeptide as defined in (v).

8. The method for production according to any of claims 1 to 7, wherein the phosphorylating enzyme comprises a polypeptide as defined in (vii) or (viii) below:
(vii) a polypeptide that has the amino acid sequence set forth in any of SEQ ID NOs: 9, 15 to 17, 19, 22, and 25; or
(viii) a polypeptide that comprises an amino acid sequence having, in the amino acid sequence set forth in any of SEQ ID NOs: 9, 15 to 17, 19, 22, and 25, 1 to 10 amino acids substituted, deleted, inserted, or added in a region outside the amino acid sequence set forth in SEQ ID NO: 2 and has an activity to catalyze the glycerol phosphorylating reaction which is comparable or superior to that of the polypeptide as defined in (vii).

9. The method for production according to any of claims 1 to 6, wherein the phosphorylating enzyme further comprises any of the polypeptides as defined in (III) to (VI) below:
(III) a polypeptide that has the amino acid sequence set forth in SEQ ID NO: 32, on the N-terminal side of the amino acid sequence represented by the sequence (1);
(IV) a polypeptide that has, on the N-terminal side of the amino acid sequence represented by the sequence (1), an amino acid sequence having 1 to 10 amino acids substituted, deleted, inserted, or added in the amino acid sequence set forth in SEQ ID NO: 32 and has an activity to catalyze the glycerol phosphorylating reaction which is comparable or superior to that of its corresponding polypeptide as defined in (III);
(V) a polypeptide that has the amino acid sequence set forth in any of SEQ ID NOs: 33 to 39 and 111 to 115, on the C-terminal side of the amino acid sequence represented by the sequence (1); and
(VI) a polypeptide that has, on the C-terminal side of the amino acid sequence represented by the sequence (1), an amino acid sequence having 1 to 10 amino acids substituted, deleted, inserted, or added in the amino acid sequence set forth in any of SEQ ID NOs: 33 to 39 and 111 to 115 and has an activity to catalyze the glycerol phosphorylating reaction which is comparable or superior to that of its corresponding polypeptide as defined in (V).

10. The method for production according to any of claims 1 to 6 and 9, wherein the phosphorylating enzyme comprises a polypeptide as defined in (VII) or (VIII) below:
(VII) a polypeptide that has the amino acid sequence set forth in SEQ ID NO: 9, or
(VIII) a polypeptide that comprises an amino acid sequence having, in the amino acid sequence set forth in SEQ ID NO: 9, 1 to 10 amino acids substituted, deleted, inserted, or added in a region outside the active center comprising the amino acid sequence represented by the sequence (1) and has an activity to catalyze the glycerol phosphorylating reaction which is comparable or superior to that of its corresponding polypeptide as defined in (VII).

11. The method for production according to any of claims 1 to 10, wherein the phosphate group donor is a polyphosphoric acid.

12. The method for production according to any of claims 1 to 11, wherein the phosphorylated glycerol is α-glycerophosphate.

13. The method for production according to any of claims 1 to 12, wherein the pH of the reaction solution is from 4 to 5 in the step of allowing the phosphorylating enzyme to act on the glycerol in the presence of the phosphate group donor.

14. The method for production according to any of claims 1 to 13, wherein the glycerol is added in an amount of 1000 to 50000 parts by weight per part by weight of the phosphorylating enzyme, in the step of allowing the phosphorylating enzyme to act on the glycerol in the presence of the phosphate group donor.

15. The method for production according to any of claims 1 to 14, wherein the concentration of the phosphate group donor in the reaction solution is from 2% to 10% by weight in the step of allowing the phosphorylating enzyme to act on the glycerol in the presence of the phosphate group donor.

## Patentansprüche

1. Verfahren zur Herstellung eines phosphorylierten Glycerins (= Glycerinphosphats), das einen Schritt umfasst, bei dem einem phosphorylierenden Enzym ermöglicht wird, bei Vorhandensein eines Phosphatgruppen-Donors mit Glycerin zu reagieren,
wobei das phosphorylierende Enzym eine saure Phosphatase der Klasse A ist und Folgendes umfasst:
- ein Polypeptid wie in den nachstehenden Punkten (i) oder (ii) definiert:
(i) ein Polypeptid mit der in SEQ ID No: 2 dargelegten Aminosäuresequenz und einer Katalysator-Aktivität für die Glycerin-Phosphorylierungsreaktion oder
(ii) ein Polypeptid mit einer Aminosäuresequenz, bei der 1 bis 3 Aminosäuren aus der in SEQ ID No: 2 dargelegten Aminosäuresequenz substituiert, deletiert, insertiert oder dazu hinzugefügt sind, das eine Katalysator-Aktivität für die Glycerin-Phosphorylierungsreaktion aufweist, die mit jener des in (i) definierten Polypeptids vergleichbar ist oder diese übertrifft; und, als aktives Zentrum,
- ein Polypeptid mit einer Aminosäuresequenz, die durch die Sequenz (1) dargestellt ist, auf der C-terminalen Seite der in SEQ ID No: 2 dargelegten Aminosäuresequenz:
-X¹-X²-X³-Pro-Ser-Gly-His-X⁴- (1)
worin
X¹ Glycin, Alanin oder Phenylalanin bezeichnet;
X² eine beliebige Aminosäure bezeichnet;
X³ Tyrosin oder Tryptophan bezeichnet; und
X⁴ Threonin, Serin oder Alanin bezeichnet;
mit der Maßgabe, dass X⁴ Threonin oder Serin ist, wenn X¹ Phenylalanin ist.

2. Verfahren zur Herstellung nach Anspruch 1, wobei sich der Aminosäurerest an der ersten Position der in SEQ ID No: 2 dargelegten Aminosäuresequenz zwischen den Positionen 5 und 40 vom N-Terminus des phosphorylierenden Enzyms befindet.

3. Verfahren zur Herstellung nach Anspruch 1 oder 2, wobei X¹ und X⁴ in der Sequenz (1) beliebige aus den nachstehenden Punkten (A1) bis (A8) sind:
(A1) X¹ ist Glycin und X⁴ ist Threonin;
(A2) X¹ ist Glycin und X⁴ ist Serin;
(A3) X¹ ist Alanin und X⁴ ist Serin;
(A4) X¹ ist Alanin und X⁴ ist Threonin;
(A5) X¹ ist Alanin und X⁴ ist Alanin;
(A6) X¹ ist Glycin und X⁴ ist Alanin;
(A7) X¹ ist Phenylalanin und X⁴ ist Threonin; und
(A8) X¹ ist Phenylalanin und X⁴ ist Serin.

4. Verfahren zur Herstellung nach einem der Ansprüche 1 bis 3, wobei in der Sequenz (1) X² Serin, Alanin oder Asparaginsäure ist.

5. Verfahren zur Herstellung nach einem der Ansprüche 1 bis 4, wobei X¹, X², X³ und X⁴ in der Sequenz (1) beliebige aus den nachstehenden Punkten (B1) bis (B11) sind:
(B1) X¹ ist Glycin, X² ist Serin, X³ ist Tyrosin und X⁴ ist Threonin;
(B2) X¹ ist Glycin, X² ist Alanin, X³ ist Tyrosin und X⁴ ist Threonin;
(B3) X¹ ist Glycin, X² ist Serin, X³ ist Tyrosin und X⁴ ist Serin;
(B4) X¹ ist Glycin, X² ist Asparaginsäure, X³ ist Tyrosin und X⁴ ist Threonin;
(B5) X¹ ist Alanin, X² ist Serin, X³ ist Tyrosin und X⁴ ist Serin;
(B6) X¹ ist Alanin, X² ist Serin, X³ ist Tyrosin und X⁴ ist Threonin;
(B7) X¹ ist Alanin, X² ist Serin, X³ ist Tyrosin und X⁴ ist Alanin;
(B8) X¹ ist Glycin, X² ist Serin, X³ ist Tyrosin und X⁴ ist Alanin;
(B9) X¹ ist Phenylalanin, X² ist Serin, X³ ist Tyrosin und X⁴ ist Threonin;
(B10) X¹ ist Phenylalanin, X² ist Serin, X³ ist Tyrosin und X⁴ ist Serin; und
(B11) X¹ ist Glycin, X² ist Serin, X³ ist Tryptophan und X⁴ ist Serin.

6. Verfahren zur Herstellung nach einem der Ansprüche 1 bis 5, wobei sich der Histidin-(His) Rest an der 7. Position in der Aminosäure, der durch die Sequenz (1) dargestellt ist, zwischen den Positionen 120 und 160 von dem N-Terminus des phosphorylierenden Enzyms befindet.

7. Verfahren zur Herstellung nach einem der Ansprüche 1 bis 6, wobei das phosphorylierende Enzym weiters zumindest eine der Aminosäuresequenzen, wie sie in den nachstehenden Punkten (iii) bis (vi) definiert sind, umfasst:
(iii) ein Polypeptid, das auf der N-terminalen Seite der in SEQ ID No: 2 dargelegten Aminosäuresequenz die in SEQ ID No: 40 dargelegte Aminosäuresequenz aufweist und eine Katalysator-Aktivität für die Glycerin-Phosphorylierungsreaktion aufweist;
(iv) ein Polypeptid, das auf der N-terminalen Seite der in SEQ ID No: 2 dargelegten Aminosäuresequenz eine Aminosäuresequenz aufweist, bei der 1 bis 3 Aminosäuren aus der in SEQ ID No: 40 dargelegten Aminosäuresequenz substituiert, deletiert, insertiert oder dazu hinzugefügt sind, und das eine Katalysator-Aktivität für die Glycerin-Phosphorylierungsreaktion aufweist, die mit jener des in (iii) definierten Polypeptids vergleichbar ist oder diese übertrifft;
(v) ein Polypeptid, das auf der C-terminalen Seite der in SEQ ID No: 2 dargelegten Aminosäuresequenz die in SEQ ID No: 41 dargelegte Aminosäuresequenz aufweist und eine Katalysator-Aktivität für die Glycerin-Phosphorylierungsreaktion aufweist; und
(vi) ein Polypeptid, das auf der C-terminalen Seite der in SEQ ID No: 2 dargelegten Aminosäuresequenz eine Aminosäuresequenz aufweist, bei der 1 bis 10 Aminosäuren aus der in SEQ ID No: 41 dargelegten Aminosäuresequenz substituiert, deletiert, insertiert oder dazu hinzugefügt sind, und das eine Katalysator-Aktivität für die Glycerin-Phosphorylierungsreaktion aufweist, die mit jener des in (v) definierten Polypeptids vergleichbar ist oder diese übertrifft.

8. Verfahren zur Herstellung nach einem der Ansprüche 1 bis 7, wobei das phosphorylierende Enzym ein Polypeptid umfasst, wie es in den nachstehenden Punkten (vii) oder (viii) definiert ist:
(vii) ein Polypeptid mit einer Aminosäuresequenz, die in einer beliebigen aus SEQ ID No: 9, 15 bis 17, 19, 22 und 25 dargelegt ist; oder
(viii) ein Polypeptid, das eine Aminosäuresequenz umfasst, bei der aus einer beliebigen der in SEQ ID No: 9, 15 bis 17, 19, 22 und 25 dargelegten Aminosäuresequenzen 1 bis 10 Aminosäuren in einer Region außerhalb der in SEQ ID No: 2 dargelegten Aminosäuresequenz substituiert, deletiert, insertiert oder dazu hinzugefügt sind, und das eine Katalysator-Aktivität für die Glycerin-Phosphorylierungsreaktion aufweist, die mit jener des in (vii) definierten Polypeptids vergleichbar ist oder diese übertrifft.

9. Verfahren zur Herstellung nach einem der Ansprüche 1 bis 6, wobei das phosphorylierende Enzym weiters zumindest eines der Polypeptide, wie sie in den nachstehenden Punkten (III) bis (VI) definiert sind, umfasst:
(III) ein Polypeptid, das die in SEQ ID No: 32 dargelegte Aminosäuresequenz auf der N-terminalen Seite der in Sequenz (1) dargestellten Aminosäuresequenz aufweist;
(IV) ein Polypeptid, das auf der N-terminalen Seite der in Sequenz (1) dargestellten Aminosäuresequenz eine Aminosäuresequenz aufweist, bei der 1 bis 10 Aminosäuren aus der in SEQ ID No: 32 dargelegten Aminosäuresequenz substituiert, deletiert, insertiert oder dazu hinzugefügt sind, und das eine Katalysator-Aktivität für die Glycerin-Phosphorylierungsreaktion aufweist, die mit jener des in (III) definierten entsprechenden Polypeptids vergleichbar ist oder diese übertrifft;
(V) ein Polypeptid, das die in einer beliebigen der SEQ ID No: 33 bis 39 und 111 bis 115 dargelegte Aminosäuresequenz auf der C-terminalen Seite der in Sequenz (1) dargestellten Aminosäuresequenz aufweist; und
(VI) ein Polypeptid, das auf der C-terminalen Seite der in Sequenz (1) dargestellten Aminosäuresequenz eine Aminosäuresequenz aufweist, bei der 1 bis 10 Aminosäuren aus der in einer beliebigen der SEQ ID No: 33 bis 39 und 111 bis 115 dargelegten Aminosäuresequenz substituiert, deletiert, insertiert oder dazu hinzugefügt sind, und das eine Katalysator-Aktivität für die Glycerin-Phosphorylierungsreaktion aufweist, die mit jener des in (V) definierten entsprechenden Polypeptids vergleichbar ist oder diese übertrifft.

10. Verfahren zur Herstellung nach einem der Ansprüche 1 bis 6 und 9, wobei das phosphorylierende Enzym ein Polypeptid, wie in den nachstehenden Punkten (VII) oder (VIII) definiert, umfasst:
(VII) ein Polypeptid mit der in SEQ ID No: 9 dargelegten Aminosäuresequenz; oder
(VIII) ein Polypeptid, umfassend eine Aminosäuresequenz, die in der in SEQ ID No: 9 dargelegten Aminosäuresequenz 1 bis 10 Aminosäuren in einer Region außerhalb des aktiven Zentrums, umfassend die Aminosäuresequenz, die durch Sequenz (1) dargestellt ist, aufweist, die substituiert, deletiert, insertiert oder hinzugefügt sind, und das eine Katalysator-Aktivität für die Glycerin-Phosphorylierungsreaktion aufweist, die mit jener des in (VII) definierten entsprechenden Polypeptids vergleichbar ist oder diese übertrifft.

11. Verfahren zur Herstellung nach einem der Ansprüche 1 bis 10, wobei der Phosphatgruppen-Donor eine Polyphosphorsäure ist.

12. Verfahren zur Herstellung nach einem der Ansprüche 1 bis 11, wobei das phosphorylierte Glycerin ein α-Glycerophosphat ist.

13. Verfahren zur Herstellung nach einem der Ansprüche 1 bis 12, wobei der pH der Reaktionslösung in dem Schritt, bei dem einem phosphorylierenden Enzym ermöglicht wird, bei Vorhandensein eines Phosphatgruppen-Donors mit Glycerin zu reagieren, 4 bis 5 beträgt.

14. Verfahren zur Herstellung nach einem der Ansprüche 1 bis 13, wobei das Glycerin in dem Schritt, bei dem einem phosphorylierenden Enzym ermöglicht wird, bei Vorhandensein eines Phosphatgruppen-Donors mit Glycerin zu reagieren, in einer Menge von 1.000 bis 50.000 Gewichtsteilen pro Gewichtsteil phosphorylierendem Enzym hinzugefügt wird.

15. Verfahren zur Herstellung nach einem der Ansprüche 1 bis 14, wobei die Konzentration des Phosphatgruppen-Donors in der Reaktionslösung in dem Schritt, bei dem einem phosphorylierenden Enzym ermöglicht wird, bei Vorhandensein eines Phosphatgruppen-Donors mit Glycerin zu reagieren, 2 Gew.-% bis 10 Gew.-% beträgt.

## Revendications

1. Procédé de production d'un glycérol phosphorylé (= glycérol phosphate), comprenant une étape consistant à permettre à une enzyme de phosphorylation d'agir sur du glycérol en présence d'un donneur de groupe phosphate,
dans lequel l'enzyme de phosphorylation est une phosphatase acide de classe A et comprend :
- un polypeptide tel que défini en (i) ou (ii) ci-dessous :
(i) un polypeptide qui possède la séquence d'acides aminés décrite dans SEQ ID NO: 2 et possède une activité pour catalyser la réaction de phosphorylation du glycérol, ou
(ii) un polypeptide qui possède une séquence d'acides aminés comportant 1 à 3 acides aminés substitués, supprimés, insérés, ou ajoutés dans la séquence d'acides aminés décrite dans SEQ ID NO: 2 et possède une activité pour catalyser la réaction de phosphorylation du glycérol qui est comparable ou supérieure à celle du polypeptide tel que défini en (i) ; et, en tant que centre actif,
- un polypeptide possédant, du côté C-terminal de la séquence d'acides aminés décrite dans SEQ ID NO: 2, une séquence d'acides aminés représentée par la séquence (1) :
-X¹-X²-X³-Pro-Ser-Gly-His-X⁴- (1)
dans laquelle,
X¹ désigne la glycine, l'alanine, ou la phénylalanine ;
X² désigne un acide aminé quelconque ;
X³ désigne la tyrosine ou le tryptophane ; et
X⁴ désigne la thréonine, la sérine, ou l'alanine ;
à condition que lorsque X¹ est la phénylalanine, X⁴ soit la thréonine ou la sérine.

2. Procédé de production selon la revendication 1, dans lequel le résidu d'acide aminé à la première position dans la séquence d'acides aminés décrite dans SEQ ID NO: 2 est situé entre les positions 5 et 40 à partir de l'extrémité N-terminale de l'enzyme de phosphorylation.

3. Procédé de production selon la revendication 1 ou 2, dans lequel X¹ et X⁴ dans la séquence (1) sont l'un quelconque de (A1) à (A8) ci-dessous :
(A1) X¹ est la glycine et X⁴ est la thréonine ;
(A2) X¹ est la glycine et X⁴ est la sérine ;
(A3) X¹ est l'alanine et X⁴ est la sérine ;
(A4) X¹ est l'alanine et X⁴ est la thréonine ;
(A5) X¹ est l'alanine et X⁴ est l'alanine ;
(A6) X¹ est la glycine et X⁴ est l'alanine ;
(A7) X¹ est la phénylalanine et X⁴ est la thréonine ; et
(A8) X¹ est la phénylalanine et X⁴ est la sérine.

4. Procédé de production selon l'une quelconque des revendications 1 à 3, dans lequel, dans la séquence (1), X² est la sérine, l'alanine, ou l'acide aspartique.

5. Procédé de production selon l'une quelconque des revendications 1 à 4, dans lequel X¹, X², X³, et X⁴ dans la séquence (1) sont l'un quelconque de (B1) à (B11) ci-dessous :
(B1) X¹ est la glycine, X² est la sérine, X³ est la tyrosine, et X⁴ est la thréonine ;
(B2) X¹ est la glycine, X² est l'alanine, X³ est la tyrosine, et X⁴ est la thréonine ;
(B3) X¹ est la glycine, X² est la sérine, X³ est la tyrosine, et X⁴ est la sérine ;
(B4) X¹ est la glycine, X² est l'acide aspartique, X³ est la tyrosine, et X⁴ est la thréonine ;
(B5) X¹ est l'alanine, X² est la sérine, X³ est la tyrosine, et X⁴ est la sérine ;
(B6) X¹ est l'alanine, X² est la sérine, X³ est la tyrosine, et X⁴ est la thréonine ;
(B7) X¹ est l'alanine, X² est la sérine, X³ est la tyrosine, et X⁴ est l'alanine ;
(B8) X¹ est la glycine, X² est la sérine, X³ est la tyrosine, et X⁴ est l'alanine ;
(B9) X¹ est la phénylalanine, X² est la sérine, X³ est la tyrosine, et X⁴ est la thréonine ;
(B10) X¹ est la phénylalanine, X² est la sérine, X³ est la tyrosine, et X⁴ est la sérine ; et
(B11) X¹ est la glycine, X² est la sérine, X³ est le tryptophane, et X⁴ est la sérine.

6. Procédé de production selon l'une quelconque des revendications 1 à 5, dans lequel le résidu histidine (His) à la 7^{e} position dans la séquence d'acides aminés représentée par la séquence (1) est situé entre les positions 120 et 160 à partir de l'extrémité N-terminale de l'enzyme de phosphorylation.

7. Procédé de production selon l'une quelconque des revendications 1 à 6, dans lequel l'enzyme de phosphorylation comprend en outre au moins une des séquences d'acides aminés telles que définies en (iii) à (vi) ci-dessous :
(iii) un polypeptide qui possède, du côté N-terminal de la séquence d'acides aminés décrite dans SEQ ID NO: 2, la séquence d'acides aminés décrite dans SEQ ID NO: 40 et possède une activité pour catalyser la réaction de phosphorylation du glycérol ;
(iv) un polypeptide qui possède, du côté N-terminal de la séquence d'acides aminés décrite dans SEQ ID NO: 2, une séquence d'acides aminés comportant 1 à 3 acides aminés substitués, supprimés, insérés, ou ajoutés dans la séquence d'acides aminés décrite dans SEQ ID NO: 40 et possède une activité pour catalyser la réaction de phosphorylation du glycérol qui est comparable ou supérieure à celle du polypeptide tel que défini en (iii) ;
(v) un polypeptide qui possède, du côté C-terminal de la séquence d'acides aminés décrite dans SEQ ID NO: 2, la séquence d'acides aminés décrite dans SEQ ID NO: 41 et possède une activité pour catalyser la réaction de phosphorylation du glycérol ; et
(vi) un polypeptide qui possède, du côté C-terminal de la séquence d'acides aminés décrite dans SEQ ID NO: 2, une séquence d'acides aminés comportant 1 à 10 acides aminés substitués, supprimés, insérés, ou ajoutés dans la séquence d'acides aminés décrite dans SEQ ID NO: 41 et possède une activité pour catalyser la réaction de phosphorylation du glycérol qui est comparable ou supérieure à celle du polypeptide tel que défini en (v).

8. Procédé de production selon l'une quelconque des revendications 1 à 7, dans lequel l'enzyme de phosphorylation comprend un polypeptide tel que défini en (vii) ou (viii) ci-dessous :
(vii) un polypeptide qui possède la séquence d'acides aminés décrite dans l'une quelconque de SEQ ID NO: 9, 15 à 17, 19, 22, et 25 ; ou
(viii) un polypeptide qui comprend une séquence d'acides aminés comportant, dans la séquence d'acides aminés décrite dans SEQ ID NO: 9, 15 à 17, 19, 22, et 25, 1 à 10 acides aminés substitués, supprimés, insérés, ou ajoutés dans une région en dehors de la séquence d'acides aminés décrite dans SEQ ID NO: 2 et possède une activité pour catalyser la réaction de phosphorylation du glycérol qui est comparable ou supérieure à celle du polypeptide tel que défini en (vii).

9. Procédé de production selon l'une quelconque des revendications 1 à 6, dans lequel l'enzyme de phosphorylation comprend en outre l'un quelconque des polypeptides tels que définis en (III) à (VI) ci-dessous :
(III) un polypeptide qui possède la séquence d'acides aminés décrite dans SEQ ID NO: 32, du côté N-terminal de la séquence d'acides aminés représentée par la séquence (1) ;
(IV) un polypeptide qui possède, du côté N-terminal de la séquence d'acides aminés représentée par la séquence (1), une séquence d'acides aminés comportant 1 à 10 acides aminés substitués, supprimés, insérés, ou ajoutés dans la séquence d'acides aminés décrite dans SEQ ID NO: 32 et possède une activité pour catalyser la réaction de phosphorylation du glycérol qui est comparable ou supérieure à celle de son polypeptide correspondant tel que défini en (III) ;
(V) un polypeptide qui possède la séquence d'acides aminés décrite dans l'une quelconque de SEQ ID NO: 33 à 39 et 111 à 115, du côté C-terminal de la séquence d'acides aminés représentée par la séquence (1) ; et
(VI) un polypeptide qui possède, du côté C-terminal de la séquence d'acides aminés représentée par la séquence (1), une séquence d'acides aminés comportant 1 à 10 acides aminés substitués, supprimés, insérés, ou ajoutés dans la séquence d'acides aminés décrite dans l'une quelconque de SEQ ID NO: 33 à 39 et 111 à 115 et possède une activité pour catalyser la réaction de phosphorylation du glycérol qui est comparable ou supérieure à celle de son polypeptide correspondant tel que défini en (V).

10. Procédé de production selon l'une quelconque des revendications 1 à 6 et 9, dans lequel l'enzyme de phosphorylation comprend un polypeptide tel que défini en (VII) ou (VIII) ci-dessous :
(VII) un polypeptide qui possède la séquence d'acides aminés décrite dans SEQ ID NO: 9, ou
(VIII) un polypeptide qui comprend une séquence d'acides aminés comportant, dans la séquence d'acides aminés décrite dans SEQ ID NO: 9, 1 à 10 acides aminés substitués, supprimés, insérés, ou ajoutés dans une région en dehors du centre actif comprenant la séquence d'acides aminés représentée par la séquence (1) et possède une activité pour catalyser la réaction de phosphorylation du glycérol qui est comparable ou supérieure à celle de son polypeptide correspondant tel que défini en (VII).

11. Procédé de production selon l'une quelconque des revendications 1 à 10, dans lequel le donneur de groupe phosphate est un acide polyphosphorique.

12. Procédé de production selon l'une quelconque des revendications 1 à 11, dans lequel le glycérol phosphorylé est l'α-glycérophosphate.

13. Procédé de production selon l'une quelconque des revendications 1 à 12, dans lequel le pH de la solution de réaction est compris entre 4 et 5 lors de l'étape consistant à permettre à l'enzyme de phosphorylation d'agir sur le glycérol en présence du donneur de groupe phosphate.

14. Procédé de production selon l'une quelconque des revendications 1 à 13, dans lequel le glycérol est ajouté en une quantité comprise entre 1 000 et 50 000 parties en poids par partie en poids de l'enzyme de phosphorylation, lors de l'étape consistant à permettre à l'enzyme de phosphorylation d'agir sur le glycérol en présence du donneur de groupe phosphate.

15. Procédé de production selon l'une quelconque des revendications 1 à 14, dans lequel la concentration du donneur de groupe phosphate dans la solution de réaction est comprise entre 2 % et 10 % en poids lors de l'étape consistant à permettre à l'enzyme de phosphorylation d'agir sur le glycérol en présence du donneur de groupe phosphate.


### SEQUENCE LISTING

<110> Thermostable Enzyme Laboratory Co.,Ltd
<120> Method for Phosphorylating Glycerol
<130> 14009WO
<150> JP 2013-29701
   <151> 2013-02-19
<160> 139
<170> PatentIn version 3.5
<210> 1
   <211> 8
   <212> PRT
   <213> artificial sequence
<220>
   <223> amino acid sequence for active site.
<220>
   <221> MISC_FEATURE
   <222> (1)..(1)
   <223> Gly Ala or Phe
<220>
   <221> MISC_FEATURE
   <222> (2)..(2)
   <223> Asp, Ser, or Ala.
<220>
   <221> MISC_FEATURE
   <222> (3)..(3)
   <223> Tyr or Trp
<220>
   <221> MISC_FEATURE
   <222> (8)..(8)
   <223> Thr or Ser
<220>
   <221> MISC_FEATURE
   <222> (8)..(8)
   <223> Thr, Ser, or Ala
<400> 1
   Xaa Xaa Xaa Pro Ser Gly His Xaa
   1 5
<210> 2
   <211> 18
   <212> PRT
   <213> Gluconobacter oxydans
<400> 2
   Pro Asp Glu Arg Leu Val Leu Ala Pro Pro Pro Ala Pro Gly Ser Ala
   1 5 10 15
   Ala Gln
<210> 3
   <211> 238
   <212> PRT
   <213> Gluconacetobacter hansenii
<400> 3
   Met Asp Ala Gly Tyr Leu Thr Pro Ala Thr Gln Pro Asp Ala Thr Gln
   1               5                   10                  15
   Tyr Leu Pro Pro Pro Pro Gln Ala Gly Ser Ala Arg Gln Ala Ala Asp
               20                  25                  30
   Asp His Ala Phe Glu Ser Thr Arg Gly Leu Lys Gly Gly Ala Arg Trp
           35                  40                  45
   Ala Leu Ala Thr Ser Asp Ala Asp Leu Arg Ile Glu Ala Leu Leu Arg
       50                  55                  60
   Ser Phe Ser Cys Ala Ala Gly Phe Thr Ile Asp Ala Ser Lys Ala Pro
   65                  70                  75                  80
   Arg Leu Ala Ala Leu Ile His Arg Met Asp Val Ser Glu Ile Pro Asp
                   85                  90                  95
   Met Arg Asn Ala Lys Ala Ser Trp His Arg Ala Arg Pro Phe Val Gly
               100                 105                 110
   Asn Thr Gln Ser Ile Cys Thr Glu Asp Asp Arg Ser His Leu Ala Thr
           115                 120                 125
   Ser Gly Ala Tyr Pro Ser Gly His Thr Leu Leu Gly Trp Ser Thr Ala
       130                 135                 140
   Leu Val Leu Ala Glu Leu Leu Pro Asp Arg Ser Thr Glu Ile Leu Gln
   145                 150                 155                 160
   Arg Gly Arg Val Phe Gly Glu Ser Arg Ile Val Cys Gly Val His Trp
                   165                 170                 175
   Ala Ser Asp Val Leu Glu Gly Tyr Met Thr Gly Ala Gly Asp Ile Ala
               180                 185                 190
   Ala Met His Gly Asn Pro Ala Phe Arg Ala Asp Leu Asp Ala Ala Arg
           195                 200                 205
   Thr Glu Leu Glu Gly Leu Arg His Glu Ala Pro Lys Pro Asn Pro Gln
       210                 215                 220
   Ala Cys Thr Ile Glu His Asp Ala Ala Ala His Ser Pro Leu
   225                 230                 235
<210> 4
   <211> 243
   <212> PRT
   <213> Xanthomonas oryzae
<400> 4
   Met Asp Ala His Gly Tyr Leu Glu Lys Ser Glu Leu Pro Asp Ser Leu
   1               5                   10                  15
   Gln Leu Val Pro Pro Pro Pro Gln Asp Asp Ser Ala Ala Leu Ala Asn
               20                  25                  30
   Asp Glu Thr Val Ser Lys Ala Met Leu Ala Leu Arg Gly Thr Pro Arg
           35                  40                  45
   Trp Glu Leu Ala Ala Gln Asp Ala Val Leu Arg Phe Pro Ala Ala Ala
       50                  55                  60
   Thr His Phe Ser Cys Ala Leu Gly Ile Gln Ile Asp Gln Thr Ser Thr
   65                  70                  75                  80
   Pro His Leu Val Arg Val Leu Glu Arg Ser Met Arg Asp Ala Ser Thr
                   85                  90                  95
   Ala Thr Ser Ala Ala Lys Ala Arg Tyr Gln Arg Pro Arg Pro Phe Met
               100                 105                 110
   Arg Asn Ala Gln Pro Met Cys Thr Pro Asp Asp Asp Ala Ala Leu Arg
           115                 120                 125
   Lys Asn Gly Ser Tyr Pro Ser Gly His Thr Ala Ile Gly Trp Thr Trp
       130                 135                 140
   Gly Leu Ile Leu Ser Glu Ile Ala Pro Ala His Arg Asp Ala Leu Leu
   145                 150                 155                 160
   Ala Arg Gly Arg Ala Phe Gly Asp Ser Arg Leu Val Cys Asn Val His
                   165                 170                 175
   Trp Gln Ser Asp Val Ile Gln Gly Arg Met Val Gly Ala Ala Ala Val
               180                 185                 190
   Ala Ala Leu His Gly Asn Pro Ala Phe Glu Lys Asp Leu Ala Ala Ala
           195                 200                 205
   Arg Arg Glu Ile Glu Lys Ala Gln Ala Lys Gln Pro Thr Ala Ala Ala
       210                 215                 220
   Ala Ala Ala Cys Asn Ala Glu Arg Glu Ala Leu Lys Thr Val Leu Pro
   225                 230                 235                 240
   Gly Val Met
<210> 5
   <211> 233
   <212> PRT
   <213> Brevundimonas diminuta
<400> 5
   Met Asp His Pro His Gly Tyr Leu Thr Ala Glu Asn Thr Pro Asn Ala
   1               5                   10                  15
   Ala Asn Phe Leu Pro Pro Pro Pro Ala Glu Gly Ser Leu Arg Glu Gln
               20                  25                  30
   Ala Asp Ile Ala Ala Tyr Arg Ala Met Arg Ser Leu Glu Gly Ser Glu
           35                  40                  45
   Arg Trp Ala Ile Ala Arg Ala Asp Asn Glu Ile Glu Thr Pro Gly Ala
       50                  55                  60
   Pro Arg Ala Phe Asp Cys Ala Leu Gly Phe Lys Phe Glu Pro Glu Gln
   65                  70                  75                  80
   Met Pro Thr Leu Thr Leu Leu Met Gly Lys Met Leu Gly Asp Leu Glu
                   85                  90                  95
   Met Ile Gln Thr Pro Ala Lys Lys Gly Tyr Phe Arg Lys Arg Pro Phe
               100                 105                 110
   Val Val Glu Pro Leu Pro Thr Cys Ile Ala Pro Glu Thr Trp Leu Ala
           115                 120                 125
   Ala Ser Gly Ser Tyr Pro Ser Gly His Ser Ala Leu Gly Trp Ala Trp
       130                 135                 140
   Gly Leu Val Leu Ala Glu Leu Ala Pro Asp Arg Ala Asp Ala Ile Leu
   145                 150                 155                 160
   Arg Arg Gly Leu Ala Tyr Gly Glu Ser Arg Ala Val Cys Gly Val His
                   165                 170                 175
   Tyr Pro Ser Asp Val Glu Ala Gly Arg Ile Val Gly Ala Thr Ile Val
               180                 185                 190
   Thr Arg Leu Lys Ala Asp Pro Ala Phe Gln Ala Asp Phe Ala Lys Ala
           195                 200                 205
   Lys Glu Glu Phe Asp Ala Ala Arg Ala Ala Ala Thr Glu Ala Thr Ala
       210                 215                 220
   Ala Cys Pro Ala Ser Leu Ala Arg Gln
   225                 230
<210> 6
   <211> 232
   <212> PRT
   <213> Sphingomonas trueperi
<400> 6
   Met Asp Thr Ala Pro Tyr Leu Ala Ala Gly Gln Tyr Pro Asp Gly Met
   1               5                   10                  15
   Ala Ile Leu Pro Pro Pro Pro Ala Leu Asp Ser Pro Gly Ala Ala Leu
               20                  25                  30
   Asp Met Ala Val Phe Arg Ala Thr Arg Lys Leu Glu Gly Thr Pro Arg
           35                  40                  45
   Trp Arg Ile Ala Thr Asp Asp Val Thr Asn Asp Pro Leu Arg Arg Asn
       50                  55                  60
   Ala Cys Ala Met Gly Met Val Leu Asp Val Lys Thr Ala Pro Ala Leu
   65                  70                  75                  80
   Ala Arg Leu Leu Asp Arg Ala Gly Thr Gly Pro Val Val Gly Arg Val
                   85                  90                  95
   Lys Ala Ala Tyr Gln Val Pro Arg Pro Tyr Leu Arg Glu Asp Gly Pro
               100                 105                 110
   Ile Cys Glu Ala Lys Thr Ala His Leu Ala Ser Asn Gly Asp Tyr Pro
           115                 120                 125
   Ser Gly His Thr Ala Asn Gly Trp Leu Glu Ala Gln Ile Leu Ala Glu
   130 135 140
   Val Met Pro Asp Lys Ala Thr Ala Ile Leu Ala Arg Gly Arg Ala Tyr
   145 150 155 160
   Gly Glu Ser Arg Ala Ile Cys Gly Ser His Ser Lys Ser Ala Val Glu
   165 170 175
   Ala Gly Tyr Met Ala Gly Ala Ser Val Phe Ala Val Leu Gln Thr Ser
   180 185 190
   Pro Ala Tyr Gln Arg Asp Leu Ala Ala Ala Arg Gln Glu Ala Ala Arg
   195 200 205
   Leu Arg Thr Thr Ala Pro Arg Pro Asp Ala Gln Ser Cys Val Ala Glu
   210 215 220
   Ala Glu Ala Leu Arg Val Arg Pro
   225 230
<210> 7
   <211> 245
   <212> PRT
   <213> Zymomonas mobilis
<400> 7
   Met Asp Leu Ser Gln Ser Val Ser Ala His Thr Glu Lys Ser Glu Pro
   1               5                   10                  15
   Ser Ser Thr Tyr His Phe His Ser Asp Pro Leu Leu Tyr Leu Ala Pro
               20                  25                  30
   Pro Pro Thr Ser Gly Ser Pro Leu Gln Ala His Asp Asp Gln Thr Phe
           35                  40                  45
   Asn Ser Thr Arg Gln Leu Lys Gly Ser Thr Arg Trp Ala Leu Ala Thr
       50                  55                  60
   Gln Asp Ala Asp Leu His Leu Ala Ser Val Leu Lys Asp Tyr Ala Cys
   65                  70                  75                  80
   Ala Ala Gly Met Asn Leu Asp Ile Ala Gln Leu Pro His Leu Ala Asn
                   85                  90                  95
   Leu Ile Lys Arg Ala Leu Arg Thr Glu Tyr Asp Asp Ile Gly Arg Ala
               100                 105                 110
   Lys Asn Asn Trp Asn Arg Lys Arg Pro Phe Val Asp Thr Asp Gln Pro
               115                 120                 125
   Ile Cys Thr Glu Lys Asp Arg Glu Gly Leu Gly Lys Gln Gly Ser Tyr
       130                 135                 140
   Pro Ser Gly His Thr Thr Ile Gly Trp Ser Val Ala Leu Ile Leu Ala
   145                 150                 155                 160
   Glu Leu Ile Pro Asp His Ala Ala Asn Ile Leu Gln Arg Gly Gln Ile
                   165                 170                 175
   Phe Gly Thr Ser Arg Ile Val Cys Gly Ala His Trp Phe Ser Asp Val
               180                 185                 190
   Gln Ala Gly Tyr Ile Met Ala Ser Gly Glu Ile Ala Ala Leu His Gly
           195                 200                 205
   Asp Ala Asp Phe Arg Arg Asp Met Glu Leu Ala Arg Lys Glu Leu Glu
       210                 215                 220
   Lys Ala Arg Thr Ser Ala His Thr Pro Asp Asp Leu Leu Cys Lys Ile
   225                 230                 235                 240
   Glu Gln Ser Ala Arg
                   245
<210> 8
   <211> 223
   <212> PRT
   <213> Desulfovibrio magneticus
<400> 8
   Met Asp Ile Gly Ile Asn Ser Asp Pro Gln Leu Ala Trp Ser Glu Thr
   1               5                   10                  15
   Gln Phe Val Ser Pro Gln Gln Val Asp Leu Ala Arg Leu Leu Pro Pro
               20                  25                  30
   Pro Pro Ala Met Asp Ser Ala Glu Gln Arg Asp Glu Ile Ala Leu Leu
           35                  40                  45
   Leu Gln Leu Gln Lys Asp Arg Thr Pro Asp Met Val Ala Phe Ala Gln
       50                  55                  60
   Ala Asp Ala Ala Arg Glu Val Phe Arg Phe Thr Asp Val Val Gly Pro
   65                  70                  75                  80
   Gln Phe Thr Ala Glu Lys Leu Pro Val Ala Ala Ala Phe Phe Lys Ala
                   85                  90                  95
   Val Lys Glu Asn Gly Asp Ala Ile Leu Gly Asn Ala Lys Lys His Trp
               100                 105                 110
   Asp Arg Pro Arg Pro Tyr Ala Ala Ser Ser Gln Ile Asp Pro Cys Val
           115                 120                 125
   Pro Lys Pro Gly Asn Ala Ser Tyr Pro Ser Gly His Ser Thr Tyr Gly
       130                 135                 140
   Thr Leu Met Gly Ile Ile Leu Ala Asn Met Val Pro Glu Lys Ala Gln
   145                 150                 155                 160
   Ala Leu Ala Ala Arg Ala Glu Gln Tyr Arg Phe Asn Arg Glu Ile Gly
                   165                 170                 175
   Gly Val His Tyr Pro Ser Asp Val Ala Ala Gly Arg Ile Thr Gly Thr
               180                 185                 190
   Val Ile Ala Ala Phe Leu Phe Asn Ser Pro Glu Phe Gln Gln Gln Tyr
           195                 200                 205
   Ala Ala Ala Arg Ala Glu Val Arg Ser Ala Leu Gly Leu Ala Gln
       210                 215                 220
<210> 9
   <211> 244
   <212> PRT
   <213> Gluconobacter oxydans
<400> 9
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Ala Pro Gly Ser
               20                  25                  30
   Ala Ala Gln Ser Asp Asp Asp Arg Val Phe His Val Thr Arg Ala Leu
           35                  40                  45
   Lys Asp Thr Pro Arg Trp Lys Leu Ala Gln Ser Asp Ala Asp Leu Asp
       50                  55                  60
   Pro Ala His Val Val Arg Asp Phe Ser Cys Ala Ala Gly Phe Glu Ile
   65                  70                  75                  80
   Asp Leu Ala Arg Ala Pro His Leu Ala Arg Val Leu Glu Arg Ile Arg
                   85                  90                  95
   His Ala Val Gly His Arg Thr Ser Asp Val Lys Lys Tyr Trp His Arg
               100                 105                 110
   Thr Arg Pro Phe Val Gly Thr Asn Leu Pro Ile Cys Thr Ser Pro Glu
           115                 120                 125
   Gly Leu Gly Leu Asn Ala Ser Tyr Pro Ser Gly His Thr Thr Ala Gly
       130                 135                 140
   Tyr Gly Met Ala Leu Leu Leu Ala His Leu Met Pro Glu His Ala Ser
   145                 150                 155                 160
   Ala Ile Leu Gln Arg Gly Arg Val Phe Gly Glu Ser Arg Ile Val Cys
                   165                 170                 175
   Gly Ala His Trp Lys Ser Asp Val Gln Ala Gly Tyr Leu Asn Ala Ser
               180                 185                 190
   Ser Leu Met Asp Val Leu Leu Ala Arg Pro Glu Leu Gln Asp Asp Leu
           195                 200                 205
   Ala Ala Ala Arg Gln Glu Leu Leu Ala Met Gln Gly Thr Ala Pro Val
       210                 215                 220
   Pro Asp Ala Gly Thr Cys Ala Val Glu His Asp Ala Ala Ile His Ser
   225                 230                 235                 240
   Leu Leu Ser Glu
<210> 10
   <211> 217
   <212> PRT
   <213> Flavobacterium johnsoniae
<400> 10
   Met Asp Ala Leu Leu Asp Gly Tyr Leu Ser Glu Ala Glu Met Pro Asp
   1               5                   10                  15
   Ser Leu Leu Leu Leu Ser Pro Pro Pro Glu His Asn Ser Ser Leu Phe
               20                  25                  30
   Asp Leu Asp Leu Glu His Ala Lys Lys Ala Val Glu Ser Lys Asp Lys
           35                  40                  45
   Glu Arg Phe Leu Gln Ala Ala Arg Asp Ala Asp Leu Ser Phe Pro Phe
       50                  55                  60
   Ala Val Lys Ser Phe Glu Pro Ile Leu Gly Ile Glu Ile Ser Glu Thr
   65                  70                  75                  80
   Lys Thr Pro Lys Phe Tyr Val Leu Met Arg Arg Val Met Thr Asp Ala
                   85                  90                  95
   Gly Leu Ser Thr Tyr Ala Ala Lys Asn His Tyr Lys Arg Glu Arg Pro
               100                 105                 110
   Phe Met Val Asn Asn Gln Lys Thr Cys Thr Pro Asp Gln Glu Asn Ile
           115                 120                 125
   Leu Tyr Lys Val Gly Ser Phe Pro Ser Gly His Ala Ala Val Gly Trp
       130                 135                 140
   Ala Trp Ser Leu Val Leu Ile Lys Leu Phe Pro Asp Lys Gln Glu Glu
   145                 150                 155                 160
   Ile Leu Lys Arg Gly His Asp Phe Gly Glu Ser Arg Val Ile Cys Asn
                   165                 170                 175
   Ala His Trp Tyr Ser Asp Val Glu Met Gly Arg Val Met Gly Arg Ala
               180                 185                 190
   Ala Val Glu Cys Leu Cys Val Asn Ser Ala Phe Leu Ser Asp Leu Glu
           195                 200                 205
   Glu Val Lys Lys Glu Met Ala Gly Ala
       210                 215
<210> 11
   <211> 253
   <212> PRT
   <213> Methylobacterium ectorquens
<400> 11
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
           50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Phe
       130                 135                 140
   Ser Tyr Pro Ser Gly His Ala Ser Gln Gly Trp Ala Tyr Gly Leu Ile
   145                 150                 155                 160
   Met Ala Asn Leu Met Pro Glu Lys Ala Thr Gln Phe Leu Val Arg Ser
                   165                 170                 175
   Arg Leu Tyr Gly Glu Ser Arg Val Val Cys Gly Val His Trp Leu Ser
               180                 185                 190
   Asp Ile Glu Ala Ala Arg Thr Gly Ala Ser Ala Leu Val Ala Val Leu
           195                 200                 205
   Leu Ala Asp Pro Gly Phe Arg Thr Asp Leu Glu Arg Ala Arg Thr Asp
       210                 215                 220
   Leu Lys Arg Ala Leu Ser Gly Glu Gly Ala Lys Pro Asp Pro Ala Leu
   225                 230                 235                 240
   Cys Ala Arg Glu Asp Ala Ala Ala Arg Gln Pro Leu Leu
                   245                 250
<210> 12
   <211> 242
   <212> PRT
   <213> Acetobacter pasteurianus
<400> 12
   Met Asp Ser Ser Leu Phe Gly Tyr Thr Ala Gln Ala Gln Gln Phe Thr
   1               5                   10                  15
   Leu Pro Asp Gly Arg Ala Phe Leu Pro Pro Pro Pro Gln Ala Glu Glu
                   20                  25                  30
   Pro Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 13
   <211> 253
   <212> PRT
   <213> artificial sequence
<220>
   <223> variant a
<400> 13
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Gly
       130                 135                 140
   Ser Tyr Pro Ser Gly His Thr Ser Gln Gly Trp Ala Tyr Gly Leu Ile
   145                 150                 155                 160
   Met Ala Asn Leu Met Pro Glu Lys Ala Thr Gln Phe Leu Val Arg Ser
                   165                 170                 175
   Arg Leu Tyr Gly Glu Ser Arg Val Val Cys Gly Val His Trp Leu Ser
               180                 185                 190
   Asp Ile Glu Ala Ala Arg Thr Gly Ala Ser Ala Leu Val Ala Val Leu
           195                 200                 205
   Leu Ala Asp Pro Gly Phe Arg Thr Asp Leu Glu Arg Ala Arg Thr Asp
       210                 215                 220
   Leu Lys Arg Ala Leu Ser Gly Glu Gly Ala Lys Pro Asp Pro Ala Leu
   225 230 235 240
   Cys Ala Arg Glu Asp Ala Ala Ala Arg Gln Pro Leu Leu
   245 250
<210> 14
   <211> 245
   <212> PRT
   <213> artificial sequence
<220>
   <223> variant A
<400> 14
   Met Asp Ser Ser Leu Phe Gly Tyr Thr Ala Gln Ala Gln Gln Phe Thr
   1               5                   10                  15
   Leu Pro Asp Gly Arg Ala Phe Leu Pro Pro Pro Pro Gln Ala Glu Glu
               20                  25                  30
   Pro Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Ala
       130                 135                 140
   Gly Tyr Gly Met Ala Leu Leu Leu Ala His Leu Met Pro Glu His Ala
   145                 150                 155                 160
   Ser Ala Ile Leu Gln Arg Gly Arg Val Phe Gly Glu Ser Arg Ile Val
                   165                 170                 175
   Cys Gly Ala His Trp Lys Ser Asp Val Gln Ala Gly Tyr Leu Asn Ala
               180                 185                 190
   Ser Ser Leu Met Asp Val Leu Leu Ala Arg Pro Glu Leu Gln Asp Asp
           195                 200                 205
   Leu Ala Ala Ala Arg Gln Glu Leu Leu Ala Met Gln Gly Thr Ala Pro
       210                 215                 220
   Val Pro Asp Ala Gly Thr Cys Ala Val Glu His Asp Ala Ala Ile His
   225                 230                 235                 240
   Ser Leu Leu Ser Glu
                   245
<210> 15
   <211> 241
   <212> PRT
   <213> artificial sequence
<220>
   <223> variant B
<400> 15
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Ala Pro Gly Ser
               20                  25                  30
   Ala Ala Gln Ser Asp Asp Asp Arg Val Phe His Val Thr Arg Ala Leu
           35                  40                  45
   Lys Asp Thr Pro Arg Trp Lys Leu Ala Gln Ser Asp Ala Asp Leu Asp
       50                  55                  60
   Pro Ala His Val Val Arg Asp Phe Ser Cys Ala Ala Gly Phe Glu Ile
   65                  70                  75                  80
   Asp Leu Ala Arg Ala Pro His Leu Ala Arg Val Leu Glu Arg Ile Arg
                   85                  90                  95
   His Ala Val Gly His Arg Thr Ser Asp Val Lys Lys Tyr Trp His Arg
               100                 105                 110
   Thr Arg Pro Phe Val Gly Thr Asn Leu Pro Ile Cys Thr Ser Pro Glu
           115                 120                 125
   Gly Leu Gly Leu Asn Ala Ser Tyr Pro Ser Gly His Thr Thr Ala Gly
       130                 135                 140
   Trp Leu Thr Ala Ser Ile Leu Ala Ser Ala Leu Pro Asp Arg Ala Thr
   145 150 155 160
   Gln Ile Met Gln Arg Gly Arg Ile Phe Gly Glu Ser Arg Ile Val Cys
   165 170 175
   Gly Val His Trp Lys Ser Asp Val Gln Ala Gly Tyr Met Asn Gly Ser
   180 185 190
   Ala Ile Phe Ala Ala Leu Gln Glu Gln Pro Thr Phe Thr Glu Gln Met
   195 200 205
   Ala Lys Val Arg Gln Glu Leu Leu Ala Leu Arg Asp Ala Lys Thr Ala
   210 215 220
   Pro Asp Ala Lys Thr Cys Ala Val Glu Gln Gln Ala Ala Gln Asp Ala
   225 230 235 240
   Phe
<210> 16
   <211> 242
   <212> PRT
   <213> artificial sequence
<220>
   <223> variant C
<400> 16
   Met Asp Thr Ser Ala Thr Ala Gln Gly Gly Ile Leu Pro Asp Ser Ala
   1 5 10 15
   Ala Pro Asp Glu Arg Leu Val Leu Ala Pro Pro Pro Ala Pro Gly Ser
   20 25 30
   Ala Ala Gln Ser Asp Asp Asp Arg Val Phe His Val Thr Arg Ala Leu
   35 40 45
   Lys Asp Thr Pro Arg Trp Lys Leu Ala Gln Ser Asp Ala Asp Leu Asp
   50 55 60
   Pro Ala His Val Val Arg Asp Phe Ser Cys Ala Ala Gly Phe Glu Ile
   65 70 75 80
   Asp Leu Ala Arg Ala Pro His Leu Ala Arg Val Leu Glu Arg Ile Arg
   85 90 95
   His Ala Val Gly His Arg Thr Ser Asp Val Lys Lys Tyr Trp His Arg
               100                 105                 110
   Thr Arg Pro Phe Val Gly Thr Asn Leu Pro Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 17
   <211> 242
   <212> PRT
   <213> artificial sequence
<220>
   <223> variant D
<400> 17
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Ala Pro Gly Ser
               20                  25                  30
   Ala Ala Gln Ser Asp Asp Asp Arg Val Phe His Val Thr Arg Ala Leu
           35                  40                  45
   Lys Asp Thr Pro Arg Trp Lys Leu Ala Gln Ser Asp Ala Asp Leu Asp
           50                  55                  60
   Pro Ala His Val Val Arg Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 18
   <211> 241
   <212> PRT
   <213> artificial sequence
<220>
   <223> variant E
<400> 18
   Met Asp Ser Ser Leu Phe Gly Tyr Thr Ala Gln Ala Gln Gln Phe Thr
   1               5                   10                  15
   Leu Pro Asp Gly Arg Ala Phe Leu Pro Pro Pro Pro Gln Ala Glu Glu
               20                  25                  30
   Pro Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ser Pro Glu
           115                 120                 125
   Gly Leu Gly Leu Asn Ala Ser Tyr Pro Ser Gly His Thr Thr Ala Gly
       130                 135                 140
   Trp Leu Thr Ala Ser Ile Leu Ala Ser Ala Leu Pro Asp Arg Ala Thr
   145                 150                 155                 160
   Gln Ile Met Gln Arg Gly Arg Ile Phe Gly Glu Ser Arg Ile Val Cys
                   165                 170                 175
   Gly Val His Trp Lys Ser Asp Val Gln Ala Gly Tyr Met Asn Gly Ser
               180                 185                 190
   Ala Ile Phe Ala Ala Leu Gln Glu Gln Pro Thr Phe Thr Glu Gln Met
           195                 200                 205
   Ala Lys Val Arg Gln Glu Leu Leu Ala Leu Arg Asp Ala Lys Thr Ala
       210                 215                 220
   Pro Asp Ala Lys Thr Cys Ala Val Glu Gln Gln Ala Ala Gln Asp Ala
   225                 230                 235                 240
   Phe
<210> 19
   <211> 242
   <212> PRT
   <213> artificial sequence
<220>
   <223> variant F
<400> 19
   Met Asp Ser Ser Leu Phe Gly Tyr Thr Ala Gln Ala Gln Gln Phe Thr
   1               5                   10                  15
   Leu Pro Asp Glu Arg Leu Val Leu Ala Pro Pro Pro Ala Pro Gly Ser
               20                  25                  30
   Ala Ala Gln Ser Asp Asp Asp Arg Val Phe His Val Thr Arg Ala Leu
           35                  40                  45
   Lys Asp Thr Pro Arg Trp Lys Leu Ala Gln Ser Asp Ala Asp Leu Asp
       50                  55                  60
   Pro Ala His Val Val Arg Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225 230 235 240
   Ala Phe
<210> 20
   <211> 242
   <212> PRT
   <213> artificial sequence
<220>
   <223> variant G
<400> 20
   Met Asp Ser Ser Leu Phe Gly Tyr Thr Ala Gln Ala Gln Gln Phe Thr
   1 5 10 15
   Leu Pro Asp Gly Arg Ala Phe Leu Pro Pro Pro Pro Ala Pro Gly Ser
   20 25 30
   Ala Ala Gln Ser Asp Asp Asp Arg Val Phe His Val Thr Arg Ala Leu
   35 40 45
   Lys Asp Thr Pro Arg Trp Lys Leu Ala Gln Ser Asp Ala Asp Leu Asp
   50 55 60
   Pro Ala His Val Val Arg Asp Phe Ser Cys Ala Ala Gly Phe Asn Leu
   65 70 75 80
   Asp Pro Glu Lys Leu Pro Ala Met Val Asn Leu Leu Thr Ser Leu Ala
   85 90 95
   Gln Pro Val Glu Gln Asp Val Ser Asn Glu Lys Asp Phe Trp Lys Arg
   100 105 110
   Arg Arg Pro Phe Val Gly Thr Asn Lys Asp Ile Cys Thr Ala His Ser
   115 120 125
   Asp Gly Leu Asp Asn Ser Tyr Ala Tyr Pro Ser Gly His Thr Thr Trp
   130 135 140
   Gly Trp Leu Thr Ala Ser Ile Leu Ala Ser Ala Leu Pro Asp Arg Ala
   145 150 155 160
   Thr Gln Ile Met Gln Arg Gly Arg Ile Phe Gly Glu Ser Arg Ile Val
   165 170 175
   Cys Gly Val His Trp Lys Ser Asp Val Gln Ala Gly Tyr Met Asn Gly
   180 185 190
   Ser Ala Ile Phe Ala Ala Leu Gln Glu Gln Pro Thr Phe Thr Glu Gln
   195 200 205
   Met Ala Lys Val Arg Gln Glu Leu Leu Ala Leu Arg Asp Ala Lys Thr
   210 215 220
   Ala Pro Asp Ala Lys Thr Cys Ala Val Glu Gln Gln Ala Ala Gln Asp
   225 230 235 240
   Ala Phe
<210> 21
   <211> 242
   <212> PRT
   <213> artificial sequence
<220>
   <223> variant H
<400> 21
   Met Asp Ser Ser Leu Phe Gly Tyr Thr Ala Gln Ala Gln Gln Phe Thr
   1 5 10 15
   Leu Pro Asp Gly Arg Ala Phe Leu Pro Pro Pro Pro Gln Ala Glu Glu
   20 25 30
   Pro Ala Gln Gln Ala Asp Leu Arg Ala Phe Glu Lys Thr Arg Gly Leu
   35 40 45
   Lys Asp Lys Ala Arg Trp Lys Leu Ala Gln Ser Asp Ala Asp Leu Asp
   50 55 60
   Pro Ala His Val Val Arg Asp Phe Ser Cys Ala Ala Gly Phe Asn Leu
   65 70 75 80
   Asp Pro Glu Lys Leu Pro Ala Met Val Asn Leu Leu Thr Ser Leu Ala
   85 90 95
   Gln Pro Val Glu Gln Asp Val Ser Asn Glu Lys Asp Phe Trp Lys Arg
   100 105 110
   Arg Arg Pro Phe Val Gly Thr Asn Lys Asp Ile Cys Thr Ala His Ser
   115 120 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 22
   <211> 242
   <212> PRT
   <213> artificial sequence
<220>
   <223> variant I
<400> 22
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Ala Pro Gly Ser
               20                  25                  30
   Ala Ala Gln Ser Asp Asp Asp Arg Val Phe His Val Thr Arg Ala Leu
           35                  40                  45
   Lys Asp Thr Pro Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 23
   <211> 242
   <212> PRT
   <213> artificial sequence
<220>
   <223> variant J
<400> 23
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Gln Ala Glu Glu
               20                  25                  30
   Pro Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 24
   <211> 242
   <212> PRT
   <213> artificial sequence
<220>
   <223> variant K
<400> 24
   Met Asp Thr Ser Ala Thr Ala Gln Gly Gly Ile Leu Pro Asp Ser Ala
   1               5                   10                  15
   Ala Pro Asp Gly Arg Ala Phe Leu Pro Pro Pro Pro Gln Ala Glu Glu
               20                  25                  30
   Pro Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 25
   <211> 242
   <212> PRT
   <213> artificial sequence
<220>
   <223> variant L
<400> 25
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Ala Pro Gly Ser
               20                  25                  30
   Ala Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
                   85                  90                  95
   Gln Pro Val Glu Gln Asp Val Ser Asn Glu Lys Asp Phe Trp Lys Arg
               100                 105                 110
   Arg Arg Pro Phe Val Gly Thr Asn Lys Asp Ile Cys Thr Ala His Ser
           115                 120                 125
   Asp Gly Leu Asp Asn Ser Tyr Ala Tyr Pro Ser Gly His Thr Thr Trp
       130                 135                 140
   Gly Trp Leu Thr Ala Ser Ile Leu Ala Ser Ala Leu Pro Asp Arg Ala
   145                 150                 155                 160
   Thr Gln Ile Met Gln Arg Gly Arg Ile Phe Gly Glu Ser Arg Ile Val
                   165                 170                 175
   Cys Gly Val His Trp Lys Ser Asp Val Gln Ala Gly Tyr Met Asn Gly
               180                 185                 190
   Ser Ala Ile Phe Ala Ala Leu Gln Glu Gln Pro Thr Phe Thr Glu Gln
           195                 200                 205
   Met Ala Lys Val Arg Gln Glu Leu Leu Ala Leu Arg Asp Ala Lys Thr
       210                 215                 220
   Ala Pro Asp Ala Lys Thr Cys Ala Val Glu Gln Gln Ala Ala Gln Asp
   225                 230                 235                 240
   Ala Phe
<210> 26
   <211> 129
   <212> PRT
   <213> Gluconacetobacter hansenii
<400> 26
   Met Asp Ala Gly Tyr Leu Thr Pro Ala Thr Gln Pro Asp Ala Thr Gln
   1               5                   10                  15
   Tyr Leu Pro Pro Pro Pro Gln Ala Gly Ser Ala Arg Gln Ala Ala Asp
               20                  25                  30
   Asp His Ala Phe Glu Ser Thr Arg Gly Leu Lys Gly Gly Ala Arg Trp
           35                  40                  45
   Ala Leu Ala Thr Ser Asp Ala Asp Leu Arg Ile Glu Ala Leu Leu Arg
       50                  55                  60
   Ser Phe Ser Cys Ala Ala Gly Phe Thr Ile Asp Ala Ser Lys Ala Pro
   65                  70                  75                  80
   Arg Leu Ala Ala Leu Ile His Arg Met Asp Val Ser Glu Ile Pro Asp
                   85                  90                  95
   Met Arg Asn Ala Lys Ala Ser Trp His Arg Ala Arg Pro Phe Val Gly
               100                 105                 110
   Asn Thr Gln Ser Ile Cys Thr Glu Asp Asp Arg Ser His Leu Ala Thr
           115                 120                 125
   Ser
<210> 27
   <211> 130
   <212> PRT
   <213> Xanthomonas oryzae
<400> 27
   Met Asp Ala His Gly Tyr Leu Glu Lys Ser Glu Leu Pro Asp Ser Leu
   1               5                   10                  15
   Gln Leu Val Pro Pro Pro Pro Gln Asp Asp Ser Ala Ala Leu Ala Asn
               20                  25                  30
   Asp Glu Thr Val Ser Lys Ala Met Leu Ala Leu Arg Gly Thr Pro Arg
           35                  40                  45
   Trp Glu Leu Ala Ala Gln Asp Ala Val Leu Arg Phe Pro Ala Ala Ala
       50                  55                  60
   Thr His Phe Ser Cys Ala Leu Gly Ile Gln Ile Asp Gln Thr Ser Thr
   65                  70                  75                  80
   Pro His Leu Val Arg Val Leu Glu Arg Ser Met Arg Asp Ala Ser Thr
                   85                  90                  95
   Ala Thr Ser Ala Ala Lys Ala Arg Tyr Gln Arg Pro Arg Pro Phe Met
               100                 105                 110
   Arg Asn Ala Gln Pro Met Cys Thr Pro Asp Asp Asp Ala Ala Leu Arg
           115                 120                 125
   Lys Asn
       130
<210> 28
   <211> 130
   <212> PRT
   <213> Brevundimonas diminuta
<400> 28
   Met Asp His Pro His Gly Tyr Leu Thr Ala Glu Asn Thr Pro Asn Ala
   1               5                   10                  15
   Ala Asn Phe Leu Pro Pro Pro Pro Ala Glu Gly Ser Leu Arg Glu Gln
               20                  25                  30
   Ala Asp Ile Ala Ala Tyr Arg Ala Met Arg Ser Leu Glu Gly Ser Glu
           35                  40                  45
   Arg Trp Ala Ile Ala Arg Ala Asp Asn Glu Ile Glu Thr Pro Gly Ala
       50                  55                  60
   Pro Arg Ala Phe Asp Cys Ala Leu Gly Phe Lys Phe Glu Pro Glu Gln
   65                  70                  75                  80
   Met Pro Thr Leu Thr Leu Leu Met Gly Lys Met Leu Gly Asp Leu Glu
                   85                  90                  95
   Met Ile Gln Thr Pro Ala Lys Lys Gly Tyr Phe Arg Lys Arg Pro Phe
               100                 105                 110
   Val Val Glu Pro Leu Pro Thr Cys Ile Ala Pro Glu Thr Trp Leu Ala
           115                 120                 125
   Ala Ser
       130
<210> 29
   <211> 124
   <212> PRT
   <213> Sphingomonas trueperi
<400> 29
   Met Asp Thr Ala Pro Tyr Leu Ala Ala Gly Gln Tyr Pro Asp Gly Met
   1               5                   10                  15
   Ala Ile Leu Pro Pro Pro Pro Ala Leu Asp Ser Pro Gly Ala Ala Leu
               20                  25                  30
   Asp Met Ala Val Phe Arg Ala Thr Arg Lys Leu Glu Gly Thr Pro Arg
           35                  40                  45
   Trp Arg Ile Ala Thr Asp Asp Val Thr Asn Asp Pro Leu Arg Arg Asn
       50                  55                  60
   Ala Cys Ala Met Gly Met Val Leu Asp Val Lys Thr Ala Pro Ala Leu
   65                  70                  75                  80
   Ala Arg Leu Leu Asp Arg Ala Gly Thr Gly Pro Val Val Gly Arg Val
                   85                  90                  95
   Lys Ala Ala Tyr Gln Val Pro Arg Pro Tyr Leu Arg Glu Asp Gly Pro
               100                 105                 110
   Ile Cys Glu Ala Lys Thr Ala His Leu Ala Ser Asn
           115                 120
<210> 30
   <211> 141
   <212> PRT
   <213> Zymomonas mobilis
<400> 30
   Met Asp Leu Ser Gln Ser Val Ser Ala His Thr Glu Lys Ser Glu Pro
   1 5 10 15
   Ser Ser Thr Tyr His Phe His Ser Asp Pro Leu Leu Tyr Leu Ala Pro
   20 25 30
   Pro Pro Thr Ser Gly Ser Pro Leu Gln Ala His Asp Asp Gln Thr Phe
   35 40 45
   Asn Ser Thr Arg Gln Leu Lys Gly Ser Thr Arg Trp Ala Leu Ala Thr
   50 55 60
   Gln Asp Ala Asp Leu His Leu Ala Ser Val Leu Lys Asp Tyr Ala Cys
   65 70 75 80
   Ala Ala Gly Met Asn Leu Asp Ile Ala Gln Leu Pro His Leu Ala Asn
   85 90 95
   Leu Ile Lys Arg Ala Leu Arg Thr Glu Tyr Asp Asp Ile Gly Arg Ala
   100 105 110
   Lys Asn Asn Trp Asn Arg Lys Arg Pro Phe Val Asp Thr Asp Gln Pro
   115 120 125
   Ile Cys Thr Glu Lys Asp Arg Glu Gly Leu Gly Lys Gln
   130 135 140
<210> 31
   <211> 133
   <212> PRT
   <213> Desulfovibrio magneticus
<400> 31
   Met Asp Ile Gly Ile Asn Ser Asp Pro Gln Leu Ala Trp Ser Glu Thr
   1               5                   10                  15
   Gln Phe Val Ser Pro Gln Gln Val Asp Leu Ala Arg Leu Leu Pro Pro
               20                  25                  30
   Pro Pro Ala Met Asp Ser Ala Glu Gln Arg Asp Glu Ile Ala Leu Leu
           35                  40                  45
   Leu Gln Leu Gln Lys Asp Arg Thr Pro Asp Met Val Ala Phe Ala Gln
       50                  55                  60
   Ala Asp Ala Ala Arg Glu Val Phe Arg Phe Thr Asp Val Val Gly Pro
   65                  70                  75                  80
   Gln Phe Thr Ala Glu Lys Leu Pro Val Ala Ala Ala Phe Phe Lys Ala
                   85                  90                  95
   Val Lys Glu Asn Gly Asp Ala Ile Leu Gly Asn Ala Lys Lys His Trp
               100                 105                 110
   Asp Arg Pro Arg Pro Tyr Ala Ala Ser Ser Gln Ile Asp Pro Cys Val
           115                 120                 125
   Pro Lys Pro Gly Asn
       130
<210> 32
   <211> 133
   <212> PRT
   <213> Gluconobacter oxydans
<400> 32
   Met Asp Thr Ser Ala Thr Ala Gln Gly Gly Ile Leu Pro Asp Ser Ala
   1               5                   10                  15
   Ala Pro Asp Glu Arg Leu Val Leu Ala Pro Pro Pro Ala Pro Gly Ser
               20                  25                  30
   Ala Ala Gln Ser Asp Asp Asp Arg Val Phe His Val Thr Arg Ala Leu
           35                  40                  45
   Lys Asp Thr Pro Arg Trp Lys Leu Ala Gln Ser Asp Ala Asp Leu Asp
       50                  55                  60
   Pro Ala His Val Val Arg Asp Phe Ser Cys Ala Ala Gly Phe Glu Ile
   65                  70                  75                  80
   Asp Leu Ala Arg Ala Pro His Leu Ala Arg Val Leu Glu Arg Ile Arg
                   85                  90                  95
   His Ala Val Gly His Arg Thr Ser Asp Val Lys Lys Tyr Trp His Arg
               100                 105                 110
   Thr Arg Pro Phe Val Gly Thr Asn Leu Pro Ile Cys Thr Ser Pro Glu
           115                 120                 125
   Gly Leu Gly Leu Asn
       130
<210> 33
   <211> 101
   <212> PRT
   <213> Gluconacetobacter hansenii
<400> 33
   Leu Leu Gly Trp Ser Thr Ala Leu Val Leu Ala Glu Leu Leu Pro Asp
   1               5                   10                  15
   Arg Ser Thr Glu Ile Leu Gln Arg Gly Arg Val Phe Gly Glu Ser Arg
               20                  25                  30
   Ile Val Cys Gly Val His Trp Ala Ser Asp Val Leu Glu Gly Tyr Met
           35                  40                  45
   Thr Gly Ala Gly Asp Ile Ala Ala Met His Gly Asn Pro Ala Phe Arg
       50                  55                  60
   Ala Asp Leu Asp Ala Ala Arg Thr Glu Leu Glu Gly Leu Arg His Glu
   65                  70                  75                  80
   Ala Pro Lys Pro Asn Pro Gln Ala Cys Thr Ile Glu His Asp Ala Ala
                   85                  90                  95
   Ala His Ser Pro Leu
               100
<210> 34
   <211> 105
   <212> PRT
   <213> Xanthomonas oryzae
<400> 34
   Ala Ile Gly Trp Thr Trp Gly Leu Ile Leu Ser Glu Ile Ala Pro Ala
   1               5                   10                  15
   His Arg Asp Ala Leu Leu Ala Arg Gly Arg Ala Phe Gly Asp Ser Arg
               20                  25                  30
   Leu Val Cys Asn Val His Trp Gln Ser Asp Val Ile Gln Gly Arg Met
           35                  40                  45
   Val Gly Ala Ala Ala Val Ala Ala Leu His Gly Asn Pro Ala Phe Glu
       50                  55                  60
   Lys Asp Leu Ala Ala Ala Arg Arg Glu Ile Glu Lys Ala Gln Ala Lys
   65                  70                  75                  80
   Gln Pro Thr Ala Ala Ala Ala Ala Ala Cys Asn Ala Glu Arg Glu Ala
                   85                  90                  95
   Leu Lys Thr Val Leu Pro Gly Val Met
               100                 105
<210> 35
   <211> 95
   <212> PRT
   <213> Brevundimonas diminuta
<400> 35
   Ala Leu Gly Trp Ala Trp Gly Leu Val Leu Ala Glu Leu Ala Pro Asp
   1               5                   10                  15
   Arg Ala Asp Ala Ile Leu Arg Arg Gly Leu Ala Tyr Gly Glu Ser Arg
               20                  25                  30
   Ala Val Cys Gly Val His Tyr Pro Ser Asp Val Glu Ala Gly Arg Ile
           35                  40                  45
   Val Gly Ala Thr Ile Val Thr Arg Leu Lys Ala Asp Pro Ala Phe Gln
       50                  55                  60
   Ala Asp Phe Ala Lys Ala Lys Glu Glu Phe Asp Ala Ala Arg Ala Ala
   65                  70                  75                  80
   Ala Thr Glu Ala Thr Ala Ala Cys Pro Ala Ser Leu Ala Arg Gln
                   85                  90                  95
<210> 36
   <211> 100
   <212> PRT
   <213> Sphingomonas trueperi
<400> 36
   Ala Asn Gly Trp Leu Glu Ala Gln Ile Leu Ala Glu Val Met Pro Asp
   1               5                   10                  15
   Lys Ala Thr Ala Ile Leu Ala Arg Gly Arg Ala Tyr Gly Glu Ser Arg
               20                  25                  30
   Ala Ile Cys Gly Ser His Ser Lys Ser Ala Val Glu Ala Gly Tyr Met
           35                  40                  45
   Ala Gly Ala Ser Val Phe Ala Val Leu Gln Thr Ser Pro Ala Tyr Gln
       50                  55                  60
   Arg Asp Leu Ala Ala Ala Arg Gln Glu Ala Ala Arg Leu Arg Thr Thr
   65                  70                  75                  80
   Ala Pro Arg Pro Asp Ala Gln Ser Cys Val Ala Glu Ala Glu Ala Leu
                   85                  90                  95
   Arg Val Arg Pro
   100
<210> 37
   <211> 96
   <212> PRT
   <213> Zymomonas mobilis
<400> 37
   Thr Ile Gly Trp Ser Val Ala Leu Ile Leu Ala Glu Leu Ile Pro Asp
   1 5 10 15
   His Ala Ala Asn Ile Leu Gln Arg Gly Gln Ile Phe Gly Thr Ser Arg
   20 25 30
   Ile Val Cys Gly Ala His Trp Phe Ser Asp Val Gln Ala Gly Tyr Ile
   35 40 45
   Met Ala Ser Gly Glu Ile Ala Ala Leu His Gly Asp Ala Asp Phe Arg
   50 55 60
   Arg Asp Met Glu Leu Ala Arg Lys Glu Leu Glu Lys Ala Arg Thr Ser
   65 70 75 80
   Ala His Thr Pro Asp Asp Leu Leu Cys Lys Ile Glu Gln Ser Ala Arg
   85 90 95
<210> 38
   <211> 82
   <212> PRT
   <213> Desulfovibrio magneticus
<400> 38
   Thr Tyr Gly Thr Leu Met Gly Ile Ile Leu Ala Asn Met Val Pro Glu
   1 5 10 15
   Lys Ala Gln Ala Leu Ala Ala Arg Ala Glu Gln Tyr Arg Phe Asn Arg
   20 25 30
   Glu Ile Gly Gly Val His Tyr Pro Ser Asp Val Ala Ala Gly Arg Ile
   35 40 45
   Thr Gly Thr Val Ile Ala Ala Phe Leu Phe Asn Ser Pro Glu Phe Gln
   50 55 60
   Gln Gln Tyr Ala Ala Ala Arg Ala Glu Val Arg Ser Ala Leu Gly Leu
   65 70 75 80
   Ala Gln
<210> 39
   <211> 103
   <212> PRT
   <213> Gluconobacter oxydans
<400> 39
   Thr Ala Gly Tyr Gly Met Ala Leu Leu Leu Ala His Leu Met Pro Glu
   1 5 10 15
   His Ala Ser Ala Ile Leu Gln Arg Gly Arg Val Phe Gly Glu Ser Arg
   20 25 30
   Ile Val Cys Gly Ala His Trp Lys Ser Asp Val Gln Ala Gly Tyr Leu
   35 40 45
   Asn Ala Ser Ser Leu Met Asp Val Leu Leu Ala Arg Pro Glu Leu Gln
   50 55 60
   Asp Asp Leu Ala Ala Ala Arg Gln Glu Leu Leu Ala Met Gln Gly Thr
   65 70 75 80
   Ala Pro Val Pro Asp Ala Gly Thr Cys Ala Val Glu His Asp Ala Ala
   85 90 95
   Ile His Ser Leu Leu Ser Glu
   100
<210> 40
   <211> 17
   <212> PRT
   <213> Gluconacetobacter hansenii
<400> 40
   Met Asp Thr Ser Ala Thr Ala Gln Gly Gly Ile Leu Pro Asp Ser Ala
   1 5 10 15
   Ala
<210> 41
   <211> 209
   <212> PRT
   <213> Gluconacetobacter hansenii
<400> 41
   Ser Asp Asp Asp Arg Val Phe His Val Thr Arg Ala Leu Lys Asp Thr
   1 5 10 15
   Pro Arg Trp Lys Leu Ala Gln Ser Asp Ala Asp Leu Asp Pro Ala His
               20                  25                  30
   Val Val Arg Asp Phe Ser Cys Ala Ala Gly Phe Glu Ile Asp Leu Ala
           35                  40                  45
   Arg Ala Pro His Leu Ala Arg Val Leu Glu Arg Ile Arg His Ala Val
       50                  55                  60
   Gly His Arg Thr Ser Asp Val Lys Lys Tyr Trp His Arg Thr Arg Pro
   65                  70                  75                  80
   Phe Val Gly Thr Asn Leu Pro Ile Cys Thr Ser Pro Glu Gly Leu Gly
                   85                  90                  95
   Leu Asn Ala Ser Tyr Pro Ser Gly His Thr Thr Ala Gly Tyr Gly Met
               100                 105                 110
   Ala Leu Leu Leu Ala His Leu Met Pro Glu His Ala Ser Ala Ile Leu
           115                 120                 125
   Gln Arg Gly Arg Val Phe Gly Glu Ser Arg Ile Val Cys Gly Ala His
       130                 135                 140
   Trp Lys Ser Asp Val Gln Ala Gly Tyr Leu Asn Ala Ser Ser Leu Met
   145                 150                 155                 160
   Asp Val Leu Leu Ala Arg Pro Glu Leu Gln Asp Asp Leu Ala Ala Ala
                   165                 170                 175
   Arg Gln Glu Leu Leu Ala Met Gln Gly Thr Ala Pro Val Pro Asp Ala
               180                 185                 190
   Gly Thr Cys Ala Val Glu His Asp Ala Ala Ile His Ser Leu Leu Ser
           195                 200                 205
   Glu
<210> 42
   <211> 36
   <212> DNA
   <213> artificial sequence
<220>
   <223> Primer 45
<400> 42
   tgcacccatg gacgcagggt atctaacacc tgcgac 36
<210> 43
   <211> 37
   <212> DNA
   <213> artificial sequence
<220>
   <223> primer 46
<400> 43
   caataagctt gagcggagaa tgggccgcag catcatg 37
<210> 44
   <211> 34
   <212> DNA
   <213> artificial sequence
<220>
   <223> primer 47
<400> 44
   gatcgccatg gacgcgcatg gatacctgga aaag 34
<210> 45
   <211> 35
   <212> DNA
   <213> artificial sequence
<220>
   <223> primer 48
<400> 45
   ttccaagctt catcacacca ggcaacaccg tcttc 35
<210> 46
   <211> 37
   <212> DNA
   <213> artificial sequence
<220>
   <223> primer 49
<400> 46
   agcttccatg gatcatccgc acggctatct gaccgcc 37
<210> 47
   <211> 36
   <212> DNA
   <213> artificial sequence
<220>
   <223> primer 50
<400> 47
   atcaaagctt ctgacgcgcc agcgaggcgg ggcagg 36
<210> 48
   <211> 32
   <212> DNA
   <213> artificial sequence
<220>
   <223> primer 51
<400> 48
   tcaggccatg gacacggcgc cgtacctcgc cg 32
<210> 49
   <211> 34
   <212> DNA
   <213> artificial sequence
<220>
   <223> primer 52
<400> 49
   aagaaagctt ggggcggacg cgaagggcct ccgc 34
<210> 50
   <211> 34
   <212> DNA
   <213> artificial sequence
<220>
   <223> primer 53
<400> 50
   tctggccatg gatctttctc aaagcgtttc agct 34
<210> 51
   <211> 33
   <212> DNA
   <213> artificial sequence
<220>
   <223> primer 54
<400> 51
   tatgaagctt gcgagcgctt tgttcaatct tgc 33
<210> 52
   <211> 37
   <212> DNA
   <213> artificial sequence
<220>
   <223> primer 55
<400> 52
   tatggatccg cagctggcct ggtccgaaac gcagttc 37
<210> 53
   <211> 39
   <212> DNA
   <213> artificial sequence
<220>
   <223> primer 56
<400> 53
   cctcaagctt ctgggccaga cccagggcgc tgcggactt 39
<210> 54
   <211> 38
   <212> DNA
   <213> artificial sequence
<220>
   <223> primer 57
<400> 54
   taacgcccat ggatacgtcc gctaccgccc aaggcggc 38
<210> 55
   <211> 37
   <212> DNA
   <213> artificial sequence
<220>
   <223> primner 58
<400> 55
   aacaaagctt ttcgctgagc agggaatgga tcgcggc 37
<210> 56
   <211> 35
   <212> DNA
   <213> artificial sequence
<220>
   <223> primer 59
<400> 56
   cttagaccat ggatgcattg ctggatggat atttg 35
<210> 57
   <211> 36
   <212> DNA
   <213> artificial sequence
<220>
   <223> primer 60
<400> 57
   ccataagctt agctcctgcc atttcttttt taacct 36
<210> 58
   <211> 37
   <212> DNA
   <213> artificial sequence
<220>
   <223> primer 61
<400> 58
   tacgttccat ggattccgcc ccctcgctcg agaagga 37
<210> 59
   <211> 36
   <212> DNA
   <213> artificial seuqence
<220>
   <223> primer 62
<400> 59
   atccaagctt gagcaggggc tggcgcgcgg ccgcat 36
<210> 60
   <211> 35
   <212> DNA
   <213> artificial sequence
<220>
   <223> primer 63
<400> 60
   cttagaccat ggatagcagc ctttttggat atacc 35
<210> 61
   <211> 37
   <212> DNA
   <213> artificial sequence
<220>
   <223> primer 64
<400> 61
   aagtaagctt aaaagcgtct tgcgcagcct gctgttc 37
<210> 62
   <211> 37
   <212> DNA
   <213> artificial sequence
<220>
   <223> primer (3)
<400> 62
   tacgttccat ggattccgcc ccctcgctcg agaagga 37
<210> 63
   <211> 36
   <212> DNA
   <213> artificial sequence
<220>
   <223> primer (4)
<400> 63
   atccaagctt gagcaggggc tggcgcgcgg ccgcat 36
<210> 64
   <211> 36
   <212> DNA
   <213> artificial sequence
<220>
   <223> primer (11)
<400> 64
   agcggatcct atccctccgg tcatactagc cagggg 36
<210> 65
   <211> 33
   <212> DNA
   <213> artificial sequence
<220>
   <223> primer (12)
<400> 65
   ataggatccg ctgtccgcga gttcggccgt gcg 33
<210> 66
   <211> 35
   <212> DNA
   <213> artificial sequence
<220>
   <223> primer 1
<400> 66
   cttagaccat ggatagcagc ctttttggat atacc 35
<210> 67
   <211> 37
   <212> DNA
   <213> artificial sequence
<220>
   <223> primer 2
<400> 67
   aagtaagctt aaaagcgtct tgcgcagcct gctgttc 37
<210> 68
   <211> 38
   <212> DNA
   <213> artificial sequence
<220>
   <223> primer 3
<400> 68
   taacgcccat ggatacgtcc gctaccgccc aaggcggc 38
<210> 69
   <211> 37
   <212> DNA
   <213> artificial sequence
<220>
   <223> primer 4
<400> 69
   aacaaagctt ttcgctgagc agggaatgga tcgcggc 37
<210> 70
   <211> 31
   <212> DNA
   <213> artificial sequence
<220>
   <223> primer 5
<400> 70
   gactgcaggc tacgggatgg cgctgctcct g 31
<210> 71
   <211> 28
   <212> DNA
   <213> artificial sequence
<220>
   <223> primer 6
<400> 71
   atcctgcagt tgtatggcca gagggata 28
<210> 72
   <211> 29
   <212> DNA
   <213> artificial sequence
<220>
   <223> primer 7
<400> 72
   caactgcagg atggctcacg gcatccatt 29
<210> 73
   <211> 33
   <212> DNA
   <213> artificial sequence
<220>
   <223> primer 8
<400> 73
   tcctgcagtc gtatgacccg acggatagga cgc 33
<210> 74
   <211> 36
   <212> DNA
   <213> artificial sequence
<220>
   <223> primer 9
<400> 74
   aataaacaca tatgcacagc acattcagat gggctt 36
<210> 75
   <211> 36
   <212> DNA
   <213> artificial sequence
<220>
   <223> primer 10
<400> 75
   gagagctgca tatgggcagg ttcgtaccca cgaacg 36
<210> 76
   <211> 33
   <212> DNA
   <213> artificial sequence
<220>
   <223> primer 11
<400> 76
   tttaatgtcg acccagaaaa acttcccgca atg 33
<210> 77
   <211> 35
   <212> DNA
   <213> artificial sequence
<220>
   <223> primer 12
<400> 77
   gccagctcga gattgaaacc cgccgcacag gagaa 35
<210> 78
   <211> 44
   <212> DNA
   <213> artificial sequence
<220>
   <223> primer 13
<400> 78
   acactagtcc ggaaggtctt ggtctcaacg cttcctatcc ctct 44
<210> 79
   <211> 32
   <212> DNA
   <213> artificial sequence
<220>
   <223> primer 14
<400> 79
   ggactagtgc agatgtcttt atttgtgccg ac 32
<210> 80
   <211> 27
   <212> DNA
   <213> artificial sequence
<220>
   <223> primer 15
<400> 80
   cgactagttc tggctccgcc gccggcg 27
<210> 81
   <211> 31
   <212> DNA
   <213> artificial sequence
<220>
   <223> primer 16
<400> 81
   aaactagtcg ttcatcaggt agggtaaact g 31
<210> 82
   <211> 33
   <212> DNA
   <213> artificial sequence
<220>
   <223> primer 17
<400> 82
   ggaactgcag cgcagagtga tgacgaccgg gtg 33
<210> 83
   <211> 37
   <212> DNA
   <213> artificial sequence
<220>
   <223> primer 18
<400> 83
   agtgctgcag atccgggcgc tggtggcggg ggcaaaa 37
<210> 84
   <211> 33
   <212> DNA
   <213> artificial sequence
<220>
   <223> primer 19
<400> 84
   cgctggaagc tagcgcagag cgatgcggat ctg 33
<210> 85
   <211> 37
   <212> DNA
   <213> artificial sequence
<220>
   <223> primer 20
<400> 85
   attctgtagc tagcttccag cgcgccttat cttttaa 37
<210> 86
   <211> 33
   <212> DNA
   <213> artificial sequence
<220>
   <223> primer 21
<400> 86
   cgctggaagc tagcccagaa tgacgccaac ctt 33
<210> 87
   <211> 36
   <212> DNA
   <213> artificial sequence
<220>
   <223> primer 22
<400> 87
   gctctgagct agcttccagc gcggcgtgtc cttcag 36
<210> 88
   <211> 38
   <212> DNA
   <213> artificial sequence
<220>
   <223> primer 23
<400> 88
   gacttcagct agcaccgcca ccacaggcag aagaaccc 38
<210> 89
   <211> 36
   <212> DNA
   <213> artificial sequence
<220>
   <223> primer 24
<400> 89
   cggcggagct agcacgagac gctcgtccgg agccgc 36
<210> 90
   <211> 33
   <212> DNA
   <213> artificial sequence
<220>
   <223> primer 25
<400> 90
   cagtctgcag cacctgatgg acgtgcattt ttg 33
<210> 91
   <211> 32
   <212> DNA
   <213> artificial sequence
<220>
   <223> primer 26
<400> 91
   ggagctgcag agtccggcag gatgccgcct tg 32
<210> 92
   <211> 32
   <212> DNA
   <213> artificial sequence
<220>
   <223> primer 27
<400> 92
   aacctgcagc acaacaggca gacttgcggg ct 32
<210> 93
   <211> 31
   <212> DNA
   <213> artificial sequence
<220>
   <223> primer 28
<400> 93
   ctgctgcaga tccgggcgcc ggcggcggag c 31
<210> 94
   <211> 714
   <212> DNA
   <213> Gluconacetobacter hansenii
<400> 94
   atggacgcag ggtatctaac acctgcgacg cagcctgatg ccacacagta ccttcccccg 60
   ccgccgcagg ctggctcggc gcgtcaggcc gcggatgacc atgcctttga atccacgcgt 120
   gggctgaagg gcggcgcgcg ctgggcactg gcgacatccg atgcggacct gcgtatcgag 180
   gcgctgctgc gcagcttttc ctgtgcggca ggctttacga tcgacgccag caaggcaccg 240
   cgccttgccg cgctgatcca tcggatggat gtcagcgaaa ttcccgatat gcgcaacgcc 300
   aaggcatcat ggcatcgcgc acgtcctttc gtgggcaata cgcagtcgat ctgcacagag 360
   gacgatcggt cccacctggc gacatcgggg gcttatcctt ccgggcacac ccttctgggc 420
   tggagtacgg cgctggtgct ggcggaactt ctgccggacc gttcgaccga aatcctgcaa 480
   cgtgggcgcg tattcggtga aagccgtatc gtctgtggcg tgcattgggc cagtgacgtg 540
   ctggagggat atatgaccgg ggcaggcgat attgccgcca tgcacggcaa ccctgccttc 600
   cgggcggatc tggatgccgc acggaccgag ttggagggcc tgcgtcacga agcccccaaa 660
   cccaatccgc aggcttgtac gatcgagcat gatgctgcgg cccattctcc gctc 714
<210> 95
   <211> 729
   <212> DNA
   <213> Xanthomonas oryzae
<400> 95
   atggacgcgc atggatacct ggaaaagagc gaactgcccg acagcctgca gctggtgccg 60
   ccgccaccac aggatgacag cgccgcgctc gccaacgacg agacggtttc caaggccatg 120
   ctcgccctgc gcggcacgcc gcgctgggag ctggccgcgc aggacgcagt gctgcgcttt 180
   ccggccgcag ccacgcattt cagctgcgcg ctcggcatac agatcgacca gaccagcacc 240
   ccgcatctgg tgcgcgtgct cgaacgcagc atgcgcgatg ccagtaccgc caccagtgcc 300
   gccaaggcgc gctatcagcg tccacgcccg ttcatgcgca atgcgcagcc gatgtgcacc 360
   ccggacgatg acgccgccct gcgcaagaac ggctcttacc cttccggtca caccgccatc 420
   ggctggacct ggggcctgat cctgtcggaa atcgcacccg cccaccgcga tgccctgctc 480
   gcccgcggcc gtgccttcgg cgatagccgc ctggtctgca acgtgcactg gcagagcgat 540
   gtcatccaag gccgcatggt gggcgccgcc gccgtcgccg ccttgcacgg caaccccgca 600
   ttcgaaaaag acctggccgc cgcacgccgc gagatcgaga aagcgcaggc gaagcagccg 660
   acggcagcgg cagcggcggc gtgtaatgcg gaacgcgaag cgctgaagac ggtgttgcct 720
   ggtgtgatg 729
<210> 96
   <211> 699
   <212> DNA
   <213> Brevundimonas diminuta
<400> 96
   atggatcatc cgcacggcta tctgaccgcc gagaacacgc ccaacgccgc caacttcctg 60
   ccgccgccgc ccgccgaagg ctcgttgcgc gaacaggccg acatcgccgc ctatcgcgcc 120
   atgcggtcgc tggaaggctc cgagcgctgg gccatcgccc gcgccgacaa tgagatcgaa 180
   acgcccggcg ccccgcgcgc cttcgactgc gccctcggct tcaagttcga gccggaacag 240
   atgccgaccc tgacgctgtt gatgggcaag atgctgggcg atctggagat gatccaaacg 300
   cccgccaaga agggctattt ccgcaaacgc cccttcgtgg tcgagcccct gccgacctgc 360
   atcgcgcccg agacctggct ggcggccagc ggctcctatc cgtcgggcca ctcggcgctg 420
   ggctgggcct ggggcctggt gctggccgaa ctggcgcccg accgcgccga cgccatcctg 480
   cgccgcggcc tggcctatgg cgaaagccgc gcggtgtgcg gcgtccacta tcccagcgac 540
   gtcgaggcgg gccgcatcgt cggcgcgacc atcgtcaccc ggctgaaggc cgatccggcg 600
   ttccaggccg atttcgccaa ggccaaggaa gagttcgacg ccgcccgcgc cgccgcgacc 660
   gaggctacgg ccgcctgccc cgcctcgctg gcgcgtcag 699
<210> 97
   <211> 696
   <212> DNA
   <213> Sphingomonas trueperi
<400> 97
   atggacacgg cgccgtacct cgccgcgggc caatatcccg acggcatggc gatcctgccg 60
   ccgccgccgg cgctcgacag ccccggcgct gccctggaca tggcggtgtt ccgggcaacg 120
   cggaagctgg agggcacccc gcgctggcgg atcgccaccg acgacgtcac caacgatccg 180
   ctgcgccgca acgcctgcgc gatggggatg gtgctcgacg tcaagactgc gccggcactt 240
   gcccggctgc tcgatcgcgc aggcacgggc ccggtcgtcg gcagggtgaa ggctgcctac 300
   caagtgccgc gtccctatct gcgcgaggac ggcccgatct gcgaggccaa gaccgcacat 360
   ctcgccagca atggcgacta cccgtcgggg cacaccgcca atggttggct ggaagcgcag 420
   atcctcgccg aggtgatgcc cgacaaggcg actgcgatcc tcgcgcgcgg ccgtgcctat 480
   ggcgagagcc gggcgatttg cgggtcgcac agcaagagcg cggtcgaagc cggctacatg 540
   gccggcgcct cggtgttcgc ggtgctgcag acctcgcccg cctaccagcg ggatctcgcg 600
   gcggcgcggc aggaagcggc gcggctgcgc actaccgcgc cgcgccccga tgcacaaagc 660
   tgtgtcgcgg aagcggaggc ccttcgcgtc cgcccc 696
<210> 98
   <211> 735
   <212> DNA
   <213> Zymomonas mobilis
<400> 98
   atggatcttt ctcaaagcgt ttcagctcat acagaaaaaa gtgaaccctc ctcgacttat 60
   catttccaca gcgatcccct tctttacctt gcgcccccac ccacttccgg cagtccatta 120
   caggcgcatg atgatcaaac ctttaacagc accagacaat taaaaggtag cacgcgctgg 180
   gcattggcaa ctcaagatgc cgatcttcat ctcgcttcag ttctcaaaga ctatgcctgc 240
   gccgcaggaa tgaatctcga tattgcgcaa ttaccgcatc ttgccaattt gattaaacgc 300
   gcacttcgca ccgaatatga cgatattggc agagccaaaa ataactggaa tcgcaaacgg 360
   ccttttgtgg ataccgatca acccatctgc acggaaaaag atcgcgaagg tctgggaaaa 420
   caaggctcct atccttcagg tcatacgact atcggttgga gcgttgcact cattctggct 480
   gaattgatcc ccgatcatgc ggcgaatatt ttgcagcgtg gccaaatttt tggaaccagc 540
   cggattgtct gcggcgccca ttggttcagc gatgtgcagg caggctatat catggcatcg 600
   ggcgaaattg cagctttaca tggggatgcc gatttccgcc gagatatgga attagctcgg 660
   aaagaattag aaaaggcacg cacatcagcg cacacgccag acgatcttct atgcaagatt 720
   gaacaaagcg ctcgc 735
<210> 99
   <211> 645
   <212> DNA
   <213> Desulfovibrio magneticus
<400> 99
   ccgcagctgg cctggtccga aacgcagttc gtcagcccgc agcaagtcga tctggcccgg 60
   ctgctgccgc cgccgccggc catggactcg gccgagcagc gcgacgagat cgccctgctc 120
   ctgcaactgc aaaaggaccg caccccggac atggtggcct ttgcccaggc cgacgccgcc 180
   cgcgaggttt tccgcttcac cgatgtggtc ggcccccagt tcacggctga aaagctaccc 240
   gtggcggcgg cctttttcaa ggcggtcaag gaaaacggcg acgccattct cggcaacgcc 300
   aagaagcact gggaccgccc ccgaccctat gccgctagtt cgcaaatcga tccctgcgtg 360
   cccaagcccg gcaacgcctc ctaccccagc ggccattcca cctacggcac cttgatgggc 420
   atcatcctgg ccaacatggt cccggaaaag gcccaagccc tggcggcccg ggccgagcag 480
   taccgcttca accgggagat cggcggcgtc cactatccca gcgacgtggc cgccggccgc 540
   atcaccggca cggtcatcgc cgccttcctg ttcaacagcc cggaattcca gcagcagtac 600
   gccgccgccc gggccgaagt ccgcagcgcc ctggggctgg cccag 645
<210> 100
   <211> 732
   <212> DNA
   <213> Gluconobacter oxydans
<400> 100
   atggatacgt ccgctaccgc ccaaggcggc atcctgccgg acagtgcggc tccggacgag     60
   cgtctcgttc tggctccgcc gccggcgccc ggaagtgccg cgcagagtga tgacgaccgg    120
   gtgtttcatg tcacgcgcgc gctgaaggac acgccgcgct ggaaactggc gcagagcgat    180
   gcggatctgg atccggcgca tgtggtccgg gatttctcct gtgcggcggg tttcgagatc    240
   gatctggcca gggcgccgca tctggcccgt gttctggagc gcatccgcca tgccgtcggg    300
   caccggactt cggacgtgaa gaaatactgg caccgtacgc ggccgttcgt gggtacgaac    360
   ctgccgatct gcacgtctcc cgaagggctg gggctgaacg cgtcctatcc gtcgggtcat     420
   acgacggcgg gatacgggat ggcgctgctc ctggcgcatc tgatgccgga acatgcttcg    480
   gccattctcc agcgcgggcg tgtgttcggg gaaagccgga tcgtctgcgg agcgcactgg    540
   aagtcggatg tccaggcggg atacctgaat gcgtcttcgc tgatggacgt gctgctggcg    600
   cggccggagc ttcaggacga tctggcggcg gcgcggcagg agctgctggc catgcaggga    660
   acggccccgg ttcctgatgc ggggacgtgt gccgtagagc acgacgccgc gatccattcc    720
   ctgctcagcg aa                                                         732
<210> 101
   <211> 651
   <212> DNA
   <213> Flavobacterium johnsoniae
<400> 101
   atggatgcat tgctggatgg atatttgtct gaggcagaaa tgcctgatag tcttctttta     60
   ttgtcgccgc ctccggaaca taactcgagc ctttttgatc ttgatttaga gcatgcaaaa    120
   aaggctgttg agtctaagga taaagagcgt tttttacagg ctgcaagaga tgcagatctg    180
   tcctttcctt ttgctgttaa atcatttgaa ccaattttag gtatagaaat tagtgaaaca    240
   aaaacaccta agttttatgt tcttatgcga agagtaatga ccgatgcggg attatctact    300
   tatgcggcta aaaatcatta taaacgagaa cggccgttta tggttaataa tcaaaaaacc    360
   tgcactccgg atcaggaaaa tatattatat aaagttgggt cttttccttc aggtcatgcg    420
   gctgtaggct gggcatggtc tttggttttg attaaacttt ttccggataa acaggaagag    480
   attttaaaac gcggacatga ctttggtgaa agccgtgtta tttgtaatgc acattggtat    540
   agtgatgttg aaatgggaag agttatgggg cgggctgctg ttgagtgtct ttgtgttaat    600
   tctgcttttc tatccgattt ggaggaggtt aaaaaagaaa tggcaggagc t             651
<210> 102
   <211> 759
   <212> DNA
   <213> Methylobacterium ectorquens
<400> 102
   atggattccg ccccctcgct cgagaaggac aagctcgcag ccgcggctcc gaagggctat 60
   ctcagcgagg aggcgacgcc gaacctcgtg gcgatcctgc cgccgccccc cgcgggccac 120
   tcggcggcgg aggcggcgga ccgggcggtc tacaacgcga cccgcgcctt ccagggcagc 180
   ccgcgctggg ccctcgccac cgacgacgtc gccgatggcg gtgccgcgct tctcgaggat 240
   tatgcctgcg tcctcggcca gcgcatcgat caggcgaacg tgccggacct gatgcggctc 300
   ctcgaccggg cccggatcga catcgcccgc gccacccgcg tggccaagcg gcgctaccgt 360
   cgcctgcgcc ccttcgtcgg caacgacttg ccgatctgcg tcgcccgcac ggccgaactc 420
   gcggacagct tcagctatcc ctccggtcat gccagccagg ggtgggccta cgggctgatc 480
   atggcgaacc tgatgccgga gaaggcgacg cagttgctgg tgcgaagccg gctttatggc 540
   gagagccggg tcgtgtgtgg ggtccactgg ctcagcgata tcgaggccgc ccgcaccggc 600
   gcctcggcgc tggtcgccgt actgctcgcc gatcccagct tccgcaccga tctggagcgc 660
   gcccgggccg acctcaaacg cgcgctgagc ggggaggggg cgaaacccga tcccgcactc 720
   tgtgcccgcg aggatgcggc cgcgcgccag cccctgctc 759
<210> 103
   <211> 726
   <212> DNA
   <213> Acetobacter pasteurianus
<400> 103
   atggatagca gcctttttgg atataccgca caggcacaac agtttaccct acctgatgga       60
   cgtgcatttt tgcccccgcc accacaggca gaagaacccg cacaacaggc agacttgcgg      120
   gcttttgaaa aaacacgcgg attaaaagat aaggcgcgct ggaaattggc ccagaatgac      180
   gccaacctta acccatccca tgtcatcaag gatttttcat gtgcggccgg gtttaatcta      240
   gacccagaaa aacttcccgc aatggtcaat cttctcacat cgctcgcgca gccagtagag      300
   caagatgttt caaacgaaaa agacttctgg aagcggcgca gaccttttgt cggcacaaat      360
   aaagacatct gcacagcaca ttcagatggg cttgataaca gctatgccta tccctctggc      420
   catacaacat ggggatggct cacggcatcc attcttgcca gcgctctgcc agatcgtgca      480
   acacagatta tgcagcgcgg ccgtatattt ggtgaaagcc gtattgtctg cggcgtgcac      540
   tggaaaagcg acgtacaggc tggttacatg aacggaagcg ccatatttgc ggcacttcag      600
   gaacaaccca catttacaga gcaaatggca aaagttcgtc aggaactact tgccctgcgt      660
   gatgcaaaaa cagcaccaga cgcaaaaact tgcgcggtag aacagcaggc tgcgcaagac      720
   gctttt                                                                726
<210> 104
   <211> 250
   <212> PRT
   <213> Serratia plymutica\201@
<400> 104
   Met Asp Thr Gly Pro Thr Val Thr Asp Pro His Phe Lys Leu Ala Pro
   1               5                   10                  15
   Gly Tyr Leu Glu Pro Ala Ser Leu Pro Val Arg Leu Ala Leu Leu Gly
               20                  25                  30
   Gly Pro Pro Lys Pro Asp Ser Ala Ala Phe Ala Arg Asp Glu Glu Ala
   35 40 45
   Arg Arg Ala Ala Leu Ala Leu Arg Gly Ser Ala Arg Glu Lys Leu Ala
   50 55 60
   Ala Thr Asp Ala Glu Leu Thr Phe Pro Ala Pro Ala Lys Ser Phe Ser
   65 70 75 80
   Cys Ala Leu Gly Thr Asp Ile Asn Glu Lys Lys Thr Pro His Leu Tyr
   85 90 95
   Ala Met Met Gln His Val Leu Thr Asp Ala Gly Gly Ser Thr Tyr Ala
   100 105 110
   Gly Lys Asn Ala Tyr Asn Arg Thr Arg Pro Phe Val Gln His Asp Glu
   115 120 125
   Gly Thr Cys Arg Lys Asp Met Glu Pro Val Leu Arg Thr Asp Gly Ser
   130 135 140
   Trp Pro Ser Gly His Ser Ala Ala Gly Trp Ala Trp Gly Leu Val Leu
   145 150 155 160
   Ala Glu Val Gln Pro Ala Arg Ala Thr Glu Leu Leu Ala Arg Gly Leu
   165 170 175
   Ala Phe Gly Gln Ser Arg Val Val Cys Asn Ala His Trp Gln Ser Asp
   180 185 190
   Val Asp Ala Gly Arg Ile Met Gly Ala Ala Thr Val Ala Val Leu His
   195 200 205
   Asp Asn Pro Ala Phe Leu Ala Asp Leu Ala Ala Ala Lys Arg Glu Val
   210 215 220
   Gln Asp Ala Thr Asn Ala Asn Leu Lys Pro Thr Glu Asp Cys Ala Ala
   225 230 235 240
   Glu Arg Val Ala Leu Ser Leu Ser Met His
   245 250
<210> 105
   <211> 211
   <212> PRT
   <213> Rahnella aquatilis
<400> 105
   Met Glu Glu Ala Lys Pro Phe Ile Thr Ser Gln Glu Leu Asp Leu Thr
   1               5                   10                  15
   Gln Tyr Leu Pro Ala Pro Pro Ala Asp Asp Ser Ala Gln Thr Gln Ala
               20                  25                  30
   Glu Leu Lys Glu Leu Leu Gln Ile Gln Ala Thr Arg Thr Pro Glu Gln
           35                  40                  45
   Glu Lys Ala Ala Ile Ala Asp Ala Gln Glu Asn Val Trp Arg Phe Ala
       50                  55                  60
   Asp Val Met Gly Pro Gly Phe Asp Ala Glu Lys Leu Pro Lys Thr Ala
   65                  70                  75                  80
   Ala Leu Phe Glu Arg Ile Val Ala Thr Glu Asp Val Val Asp Asp His
                   85                  90                  95
   Ala Lys Lys Ala Phe Asn Arg Pro Arg Pro Tyr Met Leu Asp Glu Gln
               100                 105                 110
   Ile His Pro Leu Leu Lys Lys Ser Lys Ser Gly Ser Trp Pro Ser Gly
           115                 120                 125
   His Ser Thr Ile Gly Tyr Leu Met Ala Thr Val Leu Gly Glu Met Val
       130                 135                 140
   Pro Glu Lys Arg Asn Ala Leu Phe Ala Arg Ala Ser Gly Tyr Ala Glu
   145                 150                 155                 160
   Asn Arg Leu Val Ala Gly Phe His Tyr Arg Ser Asp Thr Val Met Ser
                   165                 170                 175
   Arg Thr Gly Ala Ala Leu Ile Ala Gln Lys Met Glu Glu Gln Pro Asp
               180                 185                 190
   Phe Lys Thr Glu Phe Asp Ala Ala Lys Ala Glu Leu Arg Ala Gln Ser
           195                 200                 205
   Gly Leu Lys
       210
<210> 106
   <211> 142
   <212> PRT
   <213> Serratia plymutica
<400> 106
   Met Asp Thr Gly Pro Thr Val Thr Asp Pro His Phe Lys Leu Ala Pro
   1 5 10 15
   Gly Tyr Leu Glu Pro Ala Ser Leu Pro Val Arg Leu Ala Leu Leu Gly
   20 25 30
   Gly Pro Pro Lys Pro Asp Ser Ala Ala Phe Ala Arg Asp Glu Glu Ala
   35 40 45
   Arg Arg Ala Ala Leu Ala Leu Arg Gly Ser Ala Arg Glu Lys Leu Ala
   50 55 60
   Ala Thr Asp Ala Glu Leu Thr Phe Pro Ala Pro Ala Lys Ser Phe Ser
   65 70 75 80
   Cys Ala Leu Gly Thr Asp Ile Asn Glu Lys Lys Thr Pro His Leu Tyr
   85 90 95
   Ala Met Met Gln His Val Leu Thr Asp Ala Gly Gly Ser Thr Tyr Ala
   100 105 110
   Gly Lys Asn Ala Tyr Asn Arg Thr Arg Pro Phe Val Gln His Asp Glu
   115 120 125
   Gly Thr Cys Arg Lys Asp Met Glu Pro Val Leu Arg Thr Asp
   130 135 140
<210> 107
   <211> 122
   <212> PRT
   <213> Rahnella aquatilis
<400> 107
   Met Glu Glu Ala Lys Pro Phe Ile Thr Ser Gln Glu Leu Asp Leu Thr
   1 5 10 15
   Gln Tyr Leu Pro Ala Pro Pro Ala Asp Asp Ser Ala Gln Thr Gln Ala
   20 25 30
   Glu Leu Lys Glu Leu Leu Gln Ile Gln Ala Thr Arg Thr Pro Glu Gln
   35 40 45
   Glu Lys Ala Ala Ile Ala Asp Ala Gln Glu Asn Val Trp Arg Phe Ala
   50 55 60
   Asp Val Met Gly Pro Gly Phe Asp Ala Glu Lys Leu Pro Lys Thr Ala
   65 70 75 80
   Ala Leu Phe Glu Arg Ile Val Ala Thr Glu Asp Val Val Asp Asp His
   85 90 95
   Ala Lys Lys Ala Phe Asn Arg Pro Arg Pro Tyr Met Leu Asp Glu Gln
   100 105 110
   Ile His Pro Leu Leu Lys Lys Ser Lys Ser
   115 120
<210> 108
   <211> 132
   <212> PRT
   <213> Flavobacterium johnsoniae
<400> 108
   Met Asp Ala Leu Leu Asp Gly Tyr Leu Ser Glu Ala Glu Met Pro Asp
   1 5 10 15
   Ser Leu Leu Leu Leu Ser Pro Pro Pro Glu His Asn Ser Ser Leu Phe
   20 25 30
   Asp Leu Asp Leu Glu His Ala Lys Lys Ala Val Glu Ser Lys Asp Lys
   35 40 45
   Glu Arg Phe Leu Gln Ala Ala Arg Asp Ala Asp Leu Ser Phe Pro Phe
   50 55 60
   Ala Val Lys Ser Phe Glu Pro Ile Leu Gly Ile Glu Ile Ser Glu Thr
   65 70 75 80
   Lys Thr Pro Lys Phe Tyr Val Leu Met Arg Arg Val Met Thr Asp Ala
   85 90 95
   Gly Leu Ser Thr Tyr Ala Ala Lys Asn His Tyr Lys Arg Glu Arg Pro
   100 105 110
   Phe Met Val Asn Asn Gln Lys Thr Cys Thr Pro Asp Gln Glu Asn Ile
   115 120 125
   Leu Tyr Lys Val
   130
<210> 109
   <211> 143
   <212> PRT
   <213> Methylobacterium ectorquens
<400> 109
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser
       130                 135                 140
<210> 110
   <211> 134
   <212> PRT
   <213> Acetobacter pasteurianus
<400> 110
   Met Asp Ser Ser Leu Phe Gly Tyr Thr Ala Gln Ala Gln Gln Phe Thr
   1               5                   10                  15
   Leu Pro Asp Gly Arg Ala Phe Leu Pro Pro Pro Pro Gln Ala Glu Glu
               20                  25                  30
   Pro Ala Gln Gln Ala Asp Leu Arg Ala Phe Glu Lys Thr Arg Gly Leu
           35                  40                  45
   Lys Asp Lys Ala Arg Trp Lys Leu Ala Gln Asn Asp Ala Asn Leu Asn
       50                  55                  60
   Pro Ser His Val Ile Lys Asp Phe Ser Cys Ala Ala Gly Phe Asn Leu
   65                  70                  75                  80
   Asp Pro Glu Lys Leu Pro Ala Met Val Asn Leu Leu Thr Ser Leu Ala
   85 90 95
   Gln Pro Val Glu Gln Asp Val Ser Asn Glu Lys Asp Phe Trp Lys Arg
   100 105 110
   Arg Arg Pro Phe Val Gly Thr Asn Lys Asp Ile Cys Thr Ala His Ser
   115 120 125
   Asp Gly Leu Asp Asn Ser
   130
<210> 111
   <211> 101
   <212> PRT
   <213> Serratia plymutica
<400> 111
   Ser Ala Ala Gly Trp Ala Trp Gly Leu Val Leu Ala Glu Val Gln Pro
   1 5 10 15
   Ala Arg Ala Thr Glu Leu Leu Ala Arg Gly Leu Ala Phe Gly Gln Ser
   20 25 30
   Arg Val Val Cys Asn Ala His Trp Gln Ser Asp Val Asp Ala Gly Arg
   35 40 45
   Ile Met Gly Ala Ala Thr Val Ala Val Leu His Asp Asn Pro Ala Phe
   50 55 60
   Leu Ala Asp Leu Ala Ala Ala Lys Arg Glu Val Gln Asp Ala Thr Asn
   65 70 75 80
   Ala Asn Leu Lys Pro Thr Glu Asp Cys Ala Ala Glu Arg Val Ala Leu
   85 90 95
   Ser Leu Ser Met His
   100
<210> 112
   <211> 82
   <212> PRT
   <213> Rahnella aquatilis
<400> 112
   Ser Thr Ile Gly Tyr Leu Met Ala Thr Val Leu Gly Glu Met Val Pro
   1               5                   10                  15
   Glu Lys Arg Asn Ala Leu Phe Ala Arg Ala Ser Gly Tyr Ala Glu Asn
               20                  25                  30
   Arg Leu Val Ala Gly Phe His Tyr Arg Ser Asp Thr Val Met Ser Arg
           35                  40                  45
   Thr Gly Ala Ala Leu Ile Ala Gln Lys Met Glu Glu Gln Pro Asp Phe
       50                  55                  60
   Lys Thr Glu Phe Asp Ala Ala Lys Ala Glu Leu Arg Ala Gln Ser Gly
   65                  70                  75                  80
   Leu Lys
<210> 113
   <211> 77
   <212> PRT
   <213> Flavobacterium johnsoniae
<400> 113
   Ala Val Gly Trp Ala Trp Ser Leu Val Leu Ile Lys Leu Phe Pro Asp
   1               5                   10                  15
   Lys Gln Glu Glu Ile Leu Lys Arg Gly His Asp Phe Gly Glu Ser Arg
               20                  25                  30
   Val Ile Cys Asn Ala His Trp Tyr Ser Asp Val Glu Met Gly Arg Val
           35                  40                  45
   Met Gly Arg Ala Ala Val Glu Cys Leu Cys Val Asn Ser Ala Phe Leu
       50                  55                  60
   Ser Asp Leu Glu Glu Val Lys Lys Glu Met Ala Gly Ala
   65                  70                  75
<210> 114
   <211> 102
   <212> PRT
   <213> Methylobacterium ectorquens
<400> 114
   Ser Gln Gly Trp Ala Tyr Gly Leu Ile Met Ala Asn Leu Met Pro Glu
   1               5                   10                  15
   Lys Ala Thr Gln Phe Leu Val Arg Ser Arg Leu Tyr Gly Glu Ser Arg
               20                  25                  30
   Val Val Cys Gly Val His Trp Leu Ser Asp Ile Glu Ala Ala Arg Thr
           35                  40                  45
   Gly Ala Ser Ala Leu Val Ala Val Leu Leu Ala Asp Pro Gly Phe Arg
       50                  55                  60
   Thr Asp Leu Glu Arg Ala Arg Thr Asp Leu Lys Arg Ala Leu Ser Gly
   65                  70                  75                  80
   Glu Gly Ala Lys Pro Asp Pro Ala Leu Cys Ala Arg Glu Asp Ala Ala
                   85                  90                  95
   Ala Arg Gln Pro Leu Leu
               100
<210> 115
   <211> 98
   <212> PRT
   <213> Acetobacter pasteurianus
<400> 115
   Thr Trp Gly Trp Leu Thr Ala Ser Ile Leu Ala Ser Ala Leu Pro Asp
   1               5                   10                  15
   Arg Ala Thr Gln Ile Met Gln Arg Gly Arg Ile Phe Gly Glu Ser Arg
               20                  25                  30
   Ile Val Cys Gly Val His Trp Lys Ser Asp Val Gln Ala Gly Tyr Met
           35                  40                  45
   Asn Gly Ser Ala Ile Phe Ala Ala Leu Gln Glu Gln Pro Thr Phe Thr
       50                  55                  60
   Glu Gln Met Ala Lys Val Arg Gln Glu Leu Leu Ala Leu Arg Asp Ala
   65                  70                  75                  80
   Lys Thr Ala Pro Asp Ala Lys Thr Cys Ala Val Glu Gln Gln Ala Ala
                   85                  90                  95
   Gln Asp
<210> 116
   <211> 253
   <212> PRT
   <213> Artificial Sequence
<220>
   <223> variant b
<400> 116
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Ala
       130                 135                 140
   Ser Tyr Pro Ser Gly His Ala Ser Gln Gly Trp Ala Tyr Gly Leu Ile
   145                 150                 155                 160
   Met Ala Asn Leu Met Pro Glu Lys Ala Thr Gln Phe Leu Val Arg Ser
                   165                 170                 175
   Arg Leu Tyr Gly Glu Ser Arg Val Val Cys Gly Val His Trp Leu Ser
               180                 185                 190
   Asp Ile Glu Ala Ala Arg Thr Gly Ala Ser Ala Leu Val Ala Val Leu
           195                 200                 205
   Leu Ala Asp Pro Gly Phe Arg Thr Asp Leu Glu Arg Ala Arg Thr Asp
       210                 215                 220
   Leu Lys Arg Ala Leu Ser Gly Glu Gly Ala Lys Pro Asp Pro Ala Leu
   225                 230                 235                 240
   Cys Ala Arg Glu Asp Ala Ala Ala Arg Gln Pro Leu Leu
                   245                 250
<210> 117
   <211> 253
   <212> PRT
   <213> Artificial Sequence
<220>
   <223> variant c
<400> 117
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Gly
       130                 135                 140
   Ser Tyr Pro Ser Gly His Ala Ser Gln Gly Trp Ala Tyr Gly Leu Ile
   145                 150                 155                 160
   Met Ala Asn Leu Met Pro Glu Lys Ala Thr Gln Phe Leu Val Arg Ser
                   165                 170                 175
   Arg Leu Tyr Gly Glu Ser Arg Val Val Cys Gly Val His Trp Leu Ser
               180                 185                 190
   Asp Ile Glu Ala Ala Arg Thr Gly Ala Ser Ala Leu Val Ala Val Leu
           195                 200                 205
   Leu Ala Asp Pro Gly Phe Arg Thr Asp Leu Glu Arg Ala Arg Thr Asp
       210                 215                 220
   Leu Lys Arg Ala Leu Ser Gly Glu Gly Ala Lys Pro Asp Pro Ala Leu
   225                 230                 235                 240
   Cys Ala Arg Glu Asp Ala Ala Ala Arg Gln Pro Leu Leu
                   245                 250
<210> 118
   <211> 253
   <212> PRT
   <213> Artificial Sequence
<220>
   <223> variant d
<400> 118
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Phe
       130                 135                 140
   Ser Tyr Pro Ser Gly His Asp Ser Gln Gly Trp Ala Tyr Gly Leu Ile
   145                 150                 155                 160
   Met Ala Asn Leu Met Pro Glu Lys Ala Thr Gln Phe Leu Val Arg Ser
   165 170 175
   Arg Leu Tyr Gly Glu Ser Arg Val Val Cys Gly Val His Trp Leu Ser
   180 185 190
   Asp Ile Glu Ala Ala Arg Thr Gly Ala Ser Ala Leu Val Ala Val Leu
   195 200 205
   Leu Ala Asp Pro Gly Phe Arg Thr Asp Leu Glu Arg Ala Arg Thr Asp
   210 215 220
   Leu Lys Arg Ala Leu Ser Gly Glu Gly Ala Lys Pro Asp Pro Ala Leu
   225 230 235 240
   Cys Ala Arg Glu Asp Ala Ala Ala Arg Gln Pro Leu Leu
   245 250
<210> 119
   <211> 253
   <212> PRT
   <213> Artificial Sequence
<220>
   <223> variant e
<400> 119
   Met Asp Ser Ala Pro Ser Leu Glu Lys Asp Lys Leu Ala Ala Ala Ala
   1               5                   10                  15
   Pro Lys Gly Tyr Leu Ser Glu Glu Ala Thr Pro Asn Leu Val Ala Ile
               20                  25                  30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Phe
   130 135 140
   Ser Tyr Pro Ser Gly His Thr Ser Gln Gly Trp Ala Tyr Gly Leu Ile
   145 150 155 160
   Met Ala Asn Leu Met Pro Glu Lys Ala Thr Gln Phe Leu Val Arg Ser
   165 170 175
   Arg Leu Tyr Gly Glu Ser Arg Val Val Cys Gly Val His Trp Leu Ser
   180 185 190
   Asp Ile Glu Ala Ala Arg Thr Gly Ala Ser Ala Leu Val Ala Val Leu
   195 200 205
   Leu Ala Asp Pro Gly Phe Arg Thr Asp Leu Glu Arg Ala Arg Thr Asp
   210 215 220
   Leu Lys Arg Ala Leu Ser Gly Glu Gly Ala Lys Pro Asp Pro Ala Leu
   225 230 235 240
   Cys Ala Arg Glu Asp Ala Ala Ala Arg Gln Pro Leu Leu
   245 250
<210> 120
   <211> 253
   <212> PRT
   <213> Artificial Sequence
<220>
   <223> variant f
<400> 120
   Met Asp Ser Ala Pro Ser Leu Glu Lys Asp Lys Leu Ala Ala Ala Ala
   1 5 10 15
   Pro Lys Gly Tyr Leu Ser Glu Glu Ala Thr Pro Asn Leu Val Ala Ile
   20 25 30
   Leu Pro Pro Pro Pro Ala Gly His Ser Ala Ala Glu Ala Ala Asp Arg
   35 40 45
   Ala Val Tyr Asn Ala Ala Arg Ala Phe Gln Gly Ser Pro Arg Trp Ala
   50 55 60
   Leu Ala Thr Asp Asp Val Ala Asp Gly Gly Ala Ala Leu Leu Gln Asp
   65 70 75 80
   Tyr Ala Cys Val Leu Gly Gln Arg Ile Asp Gln Ala Ser Val Pro Asp
   85 90 95
   Leu Met Arg Leu Leu Asp Arg Ala Arg Ile Asp Ile Ala Arg Ala Thr
   100 105 110
   Arg Val Ala Lys Arg Arg Tyr Arg Arg Leu Arg Pro Phe Val Gly Asn
   115 120 125
   Asp Leu Pro Ile Cys Val Ala Arg Thr Ala Glu Leu Ala Asp Ser Phe
   130 135 140
   Ser Tyr Pro Ser Gly His Ser Ser Gln Gly Trp Ala Tyr Gly Leu Ile
   145 150 155 160
   Met Ala Asn Leu Met Pro Glu Lys Ala Thr Gln Phe Leu Val Arg Ser
   165 170 175
   Arg Leu Tyr Gly Glu Ser Arg Val Val Cys Gly Val His Trp Leu Ser
   180 185 190
   Asp Ile Glu Ala Ala Arg Thr Gly Ala Ser Ala Leu Val Ala Val Leu
   195 200 205
   Leu Ala Asp Pro Gly Phe Arg Thr Asp Leu Glu Arg Ala Arg Thr Asp
   210 215 220
   Leu Lys Arg Ala Leu Ser Gly Glu Gly Ala Lys Pro Asp Pro Ala Leu
   225 230 235 240
   Cys Ala Arg Glu Asp Ala Ala Ala Arg Gln Pro Leu Leu
   245 250
<210> 121
   <211> 253
   <212> PRT
   <213> Artificial Sequence
<220>
   <223> variant g
<400> 121
   Met Asp Ser Ala Pro Ser Leu Glu Lys Asp Lys Leu Ala Ala Ala Ala
   1 5 10 15
   Pro Lys Gly Tyr Leu Ser Glu Glu Ala Thr Pro Asn Leu Val Ala Ile
   20 25 30
   Leu Pro Pro Pro Pro Ala Gly His Ser Ala Ala Glu Ala Ala Asp Arg
           35                  40                  45
   Ala Val Tyr Asn Ala Ala Arg Ala Phe Gln Gly Ser Pro Arg Trp Ala
       50                  55                  60
   Leu Ala Thr Asp Asp Val Ala Asp Gly Gly Ala Ala Leu Leu Gln Asp
   65                  70                  75                  80
   Tyr Ala Cys Val Leu Gly Gln Arg Ile Asp Gln Ala Ser Val Pro Asp
                   85                  90                  95
   Leu Met Arg Leu Leu Asp Arg Ala Arg Ile Asp Ile Ala Arg Ala Thr
               100                 105                 110
   Arg Val Ala Lys Arg Arg Tyr Arg Arg Leu Arg Pro Phe Val Gly Asn
           115                 120                 125
   Asp Leu Pro Ile Cys Val Ala Arg Thr Ala Glu Leu Ala Asp Ser Gly
       130                 135                 140
   Ser Tyr Pro Ser Gly His Ser Ser Gln Gly Trp Ala Tyr Gly Leu Ile
   145                 150                 155                 160
   Met Ala Asn Leu Met Pro Glu Lys Ala Thr Gln Phe Leu Val Arg Ser
                   165                 170                 175
   Arg Leu Tyr Gly Glu Ser Arg Val Val Cys Gly Val His Trp Leu Ser
               180                 185                 190
   Asp Ile Glu Ala Ala Arg Thr Gly Ala Ser Ala Leu Val Ala Val Leu
           195                 200                 205
   Leu Ala Asp Pro Gly Phe Arg Thr Asp Leu Glu Arg Ala Arg Thr Asp
       210                 215                 220
   Leu Lys Arg Ala Leu Ser Gly Glu Gly Ala Lys Pro Asp Pro Ala Leu
   225                 230                 235                 240
   Cys Ala Arg Glu Asp Ala Ala Ala Arg Gln Pro Leu Leu
                   245                 250
<210> 122
   <211> 750
   <212> DNA
   <213> Serratia plymutica
<400> 122
   atggataccg ggccgacggt aacggacccg cattttaagc tggcgccggg ttaccttgaa 60
   ccggccagcc tgccggtgcg cttggccctg ctcggggggc cgccaaaacc ggactctgcg 120
   gcattcgcac gcgatgaaga ggcgcggcgg gcggcactgg cgttacgcgg gtctgcccgt 180
   gaaaaactgg ccgcaacgga tgccgagttg accttccctg caccggcaaa atcgttctcg 240
   tgcgcgctgg gcaccgatat caacgagaaa aagacgccgc acctttacgc catgatgcag 300
   cacgtactga cggatgcggg tggttcaacc tatgcaggaa agaacgccta taaccgtact 360
   cgcccgtttg tgcagcatga cgagggaacc tgtcgcaaag acatggagcc ggtgctacgt 420
   accgacggct cctggccgtc gggccactcg gcggcaggct gggcctgggg attggtgctg 480
   gccgaggtgc agccggcacg ggcgaccgag ctgctggcac gcggcttggc ctttggtcag 540
   agccgtgtgg tctgcaatgc gcattggcag agcgacgtgg atgcagggcg catcatgggg 600
   gctgccacgg tcgccgtgct gcatgataac ccggctttcc tggcagattt ggccgccgcc 660
   aagcgtgaag tgcaggacgc taccaacgcg aacctgaaac ccaccgagga ctgcgcggct 720
   gaacgtgttg cactctcttt gagtatgcac 750
<210> 123
   <211> 633
   <212> DNA
   <213> Rahnella aquatilis
<400> 123
   atggaagaag ccaaaccctt tatcaccagt caggaactgg atctgaccca atatctgcca 60
   gcgccaccgg cggatgattc ggcgcagacc caagcggagc tgaaagaatt gctccaaatt 120
   caggccaccc gcacgccgga gcaggaaaaa gcggcgattg ctgatgcgca agaaaacgtc 180
   tggcgttttg ccgatgtgat ggggccgggc tttgatgccg agaaactgcc gaaaaccgcc 240
   gcgctgtttg agcgtattgt ggcgacagaa gacgtggtgg acgatcacgc caagaaagcg 300
   tttaaccgtc cgcgtcctta tatgctggat gaacaaattc atccgctgct gaaaaagtct 360
   aaatccggtt catggccttc cggtcattcc accatcggtt atctgatggc gacggtgctg 420
   ggcgaaatgg tgccggaaaa acgcaatgcg ctgtttgccc gtgcatccgg ttatgccgaa 480
   aaccgtctgg tggctggttt ccattaccgt tctgataccg tcatgagccg caccggtgcc 540
   gcgctgattg ctcagaaaat ggaagaacag ccagatttca aaaccgaatt cgacgcggcg 600
   aaagcggaac ttcgcgccca atctggcctg aaa 633
<210> 124
   <211> 36
   <212> DNA
   <213> Artificial Sequence
<220>
   <223> primer124
<400> 124
   gctgccacca tggataccgg gccgacggta acggac 36
<210> 125
   <211> 34
   <212> DNA
   <213> Artificial Sequence
<220>
   <223> primer125
<400> 125
   gatcaagctt gtgcatactc aaagagagtg caac 34
<210> 126
   <211> 36
   <212> DNA
   <213> Artificial Sequence
<220>
   <223> primer126
<400> 126
   gttcaggcca tggaagaagc caaacccttt atcacc 36
<210> 127
   <211> 34
   <212> DNA
   <213> Artificial Sequence
<220>
   <223> primer 127
<400> 127
   acgaaagctt tttcaggcca gattgggcgc gaag 34
<210> 128
   <211> 759
   <212> DNA
   <213> Artificial Sequence
<220>
   <223> DNA coding phosphatase
<400> 128
   atggacagtg ccccgagtct ggaaaaagat aaactggcgg ccgcagctcc gaaaggctat 60
   ctgagcgaag aagctacccc gaacctggtt gcaattctgc cgccgccgcc ggcaggtcat 120
   tctgcagccg aagcagctga tcgtgccgtc tataatgcga cccgcgcctt tcagggtagc 180
   ccgcgttggg cactggcaac ggatgacgtg gcagatggtg gtgcagcact gctggaagac 240
   tacgcttgcg tgctgggtca gcgcatcgat caagcaaacg ttccggacct gatgcgtctg 300
   ctggatcgtg cacgcattga catcgcacgt gctacccgcg ttgcgaaacg tcgctatcgt 360
   cgcctgcgcc cgtttgttgg taatgatctg ccgatttgtg tcgcacgtac cgctgaactg 420
   gcggacagct tctcttatcc gagtggtcat gcgtcccagg gttgggccta cggtctgatc 480
   atggcaaacc tgatgccgga aaaagccacg caactgctgg tccgttcacg cctgtacggc 540
   gaatcgcgcg tggtttgcgg tgtgcactgg ctgagcgata ttgaagcagc tcgtaccggt 600
   gcatctgccc tggtcgccgt gctgctggca gatccgagtt tccgcacgga cctggaacgt 660
   gcacgcgctg atctgaaacg tgcgctgtcc ggcgaaggtg caaaaccgga cccggctctg 720
   tgtgcccgtg aagatgctgc tgcccgtcaa ccgctgctg 759
<210> 129
   <211> 34
   <212> DNA
   <213> Artificial Sequence
<220>
   <223> primer(5)
<400> 129
   ggcgatggcc atggacagtg ccccgagtct ggaa 34
<210> 130
   <211> 32
   <212> DNA
   <213> Artificial Sequence
<220>
   <223> primer(6)
<400> 130
   taaagaagct tcagcagcgg ttgacgggca gc 32
<210> 131
   <211> 33
   <212> DNA
   <213> Artificial Sequence
<220>
   <223> primer(7)
<400> 131
   actcggatag ctagcgctgt ccgccagttc agc 33
<210> 132
   <211> 39
   <212> DNA
   <213> Artificial Sequence
<220>
   <223> primer(8)
<400> 132
   gcggacagcg ctagctatcc gagtggtcat gcgtcccag 39
<210> 133
   <211> 31
   <212> DNA
   <213> Artificial Sequence
<220>
   <223> primer(9)
<400> 133
   aataggatcc gctgtccgcc agttcagcgg t 31
<210> 134
   <211> 39
   <212> DNA
   <213> Artificial Sequence
<220>
   <223> primer(10)
<400> 134
   gacagaggat cctatccgag tggtcatgcg tcccagggt 39
<210> 135
   <211> 39
   <212> DNA
   <213> Artificial Sequence
<220>
   <223> primer(11)
<400> 135
   gatcagacca tatgcccaac cctgggagtc atgaccact 39
<210> 136
   <211> 33
   <212> DNA
   <213> Artificial Sequence
<220>
   <223> primer(12)
<400> 136
   cagggttggg catatggtct gatcatggca aac 33
<210> 137
   <211> 39
   <212> DNA
   <213> Artificial Sequence
<220>
   <223> primer(13)
<400> 137
   gatcagacca tatgcccaac cctgggacgt atgaccact 39
<210> 138
   <211> 39
   <212> DNA
   <213> Artificial Sequence
<220>
   <223> primer(14)
<400> 138
   gatcagacca tatgcccaac cctgggagct atgaccact 39
<210> 139
   <211> 39
   <212> DNA
   <213> Artificial Sequence
<220>
   <223> primer(15)
<400> 139
   gacagaggat cctatccgag tggtcattcg tcccagggt 39